# EUROPEAN PATENT APPLICATION

(11) **EP 4 790 066 A1**
(43) Date of publication of application: **12.08.2026**
(21) Application number: 24874041.7
(22) Date of filing: 02.09.2024
(51) Int. Cl.: C07K 14/605, C07K 14/62, A61K 38/28, A61K 38/26, A61P 3/00, A61P 9/00, A61P 19/00, A61P 25/28

(54) **GLP-1R/GIPR/GCGR TRIPLE AGONIST ANALOGUE AND USE THEREOF**

(30) Priority: 07.10.2023 CN 202311295490
(71) Applicant: Shenzhen Bay Laboratory, Shenzhen, Guangdong 518000 (CN)
(72) Inventor: SHEN, Weijun, Shenzhen, Guangdong 518000 (CN); ZHENG, Nan, Shenzhen, Guangdong 518000 (CN); WANG, Leiming, Shenzhen, Guangdong 518000 (CN)
(74) Representative: Cabinet Becker et Associés
(86) International application number: PCT/CN2024/116381
(87) International publication number: WO 2025/073222

(57) **Abstract**

Provided is a polypeptide or a derivative thereof or a pharmaceutically acceptable salt thereof. The polypeptide or the derivative thereof has a structure as represented by formula (I): X₁X₂X₃GTX₆X₇X₈DX₁₀X₁₁X₁₂X₁₃X₁₄X₁₅X₁₆X₁₇X₁₈X₁₉X₂₀X₂₁X₂₂X₂₃X₂₄X₂₅LX₂₇X₂₈X₂₀X₃₀PX ₃₂SX₃₄X₃₅PPPX₃₉ (I); the structure represented by formula (I) contains two amino acids with -SH on the side chain or two amino acids with -NH₂ on the side chain, wherein -SH or -NH₂ is used for linking a modification group. The polypeptide or the derivative thereof or the pharmaceutically acceptable salt thereof exhibits strong agonistic activity on three receptor targets of GLP-1R, GCGR and GIPR, can effectively control blood sugar and reduce body weight, and can be used for preventing or treating metabolic disorder related diseases and the like.

## Description

### FIELD

The present disclosure belongs to the technical field of biological pharmaceuticals. Specifically, the present disclosure relates to a GLP-1R/GIPR/GCGR triple agonist analogue and use thereof. More specifically, the present disclosure relates to a polypeptide or a derivative thereof or a pharmaceutically acceptable salt thereof, a pharmaceutical composition, and uses thereof.

### BACKGROUND

Diabetes (e.g., type 2 diabetes mellitus (T2DM)) belongs to a class of energy metabolism disorders and has become an increasingly serious health problem in many countries, leading to a wide range of related risk diseases, such as cardiovascular and cerebrovascular disease, dyslipidemia, kidney disease, liver disease, osteoporosis, and neurodegenerative disease. Type 2 diabetes mellitus is mainly characterized by hyperglycemia caused by insulin resistance, and obesity is one of the main causes of insulin resistance. Obesity is a condition characterized by excessive fat accumulation in the body due to excessive energy intake or metabolic abnormalities. This excessive fat accumulation may impair islet cell function and exacerbate diabetes. In addition, obesity and insulin resistance are also two major causative factors of non-alcoholic fatty liver disease/non-alcoholic steatohepatitis (NAFLD/NASH). Therefore, there is an urgent need to find safe and effective drugs for treating these metabolic disorder related diseases. Furthermore, for the growing population of obese individuals, there is an urgent need for a treatment that offers better efficacy, broader benefits, and higher safety to control body weight.

Incretin is a substance secreted by the intestine under normal physiological conditions and includes glucose-dependent insulinotropic polypeptide (GIP), glucagon-like peptide-1 (GLP-1), and glucagon (GCG). Its secretion is regulated by glucose levels and has the effects of maintaining pancreatic β-cell function, stimulating insulin secretion by pancreatic β-cells, regulating the stability of glucose levels, inhibiting gastric acid secretion, delaying gastric emptying, increasing satiety, and regulating fat metabolism. These effects are of great significance for the treatment and management of diabetes, obesity, and related metabolic diseases.

GLP-1 is a polypeptide consisting of 31 amino acids, expressed by the proglucagon gene in L cells in the intestinal mucosa. GLP-1 mainly binds to the GLP-1 receptor (GLP-1R) to stimulate insulin secretion, inhibit glucagon secretion, protect pancreatic β-cells, and regulate blood glucose homeostasis. In addition, GLP-1 can inhibit appetite and gastric emptying through central nervous system signaling pathways, increase satiety, and thus reduce body weight.

GIP is a single-chain polypeptide of 42 amino acids produced by K cells in the small intestinal mucosa. GIP primarily acts on the GIP receptor (GIPR) in pancreatic cells and adipocytes. GIP can promote insulin secretion, enhance pancreatic β-cell mass, inhibit gastric acid secretion, and slow gastric motility, thereby regulating glucose metabolism. In addition, GIP can stimulate the uptake and utilization of fatty acids by adipose tissue cells. Studies also indicate that GIP has physiological effects on bone protection, including promoting osteocyte differentiation, inhibiting osteocyte apoptosis, inhibiting bone resorption, and increasing bone mineral density.

GCG is a 29-amino acid polypeptide secreted by pancreatic α cells. It primarily acts on the glucagon receptor (GCGR), which is mainly distributed in the liver and kidneys. GCG stimulates hepatic glycogenolysis, raises blood glucose levels, activates lipases, promotes lipolysis, and enhances fatty acid oxidation, thereby increasing ketone body production. The study results show that GCG has certain effects on reducing food intake, increasing adipose tissue energy expenditure, and reducing body fat content.

Therefore, there is an urgent need to develop an incretin analogue that is simultaneously active against the GLP-1R, GIPR, and GCGR, in order to effectively treat metabolic disorder related diseases such as diabetes.

### SUMMARY

The present disclosure aims to solve, at least to a certain extent, at least one of the technical problems existing in the prior art. To this end, the present disclosure provides a GLP-1R/GIPR/GCGR triple agonist analogue. The GLP-1R/GIPR/GCGR triple agonist analogue of the present disclosure has agonistic activity for GLP-1R, GIPR, and GCGR.

The present disclosure is completed based on the following findings of the inventors:

Natural GLP-1 is highly susceptible to degradation by dipeptidyl peptidase-IV (DPP-IV) and neutral endopeptidase (NEP), which are ubiquitously present in plasma, resulting in a half-life of less than 2 min. To improve the half-life of GLP-1 and the therapeutic effect for diabetes, various GLP-1 analogues are currently available for treating T2DM, including Exenatide, Liraglutide, and Semaglutide. Currently, the weight loss achieved with GLP-1 drugs used for obesity is generally around 5-10%, with the overall average weight loss not exceeding 10% of the patient's body weight. Meanwhile, many GLP-1 analogues currently on the market have their doses limited due to gastrointestinal side effects such as nausea and vomiting.

In addition, some GLP-1R/GCGR dual agonists developed based on oxyntomodulin are also believed to have better effects than GLP-1R single agonists in reducing food intake, reducing body weight, and improving blood glucose and triglyceride levels. However, the development of polypeptide drugs on the market is often hindered by short half-life and low bioavailability. Patients often need to receive higher doses of therapeutic drugs more frequently, which may lead to reduced compliance, increased costs, and a higher risk of side effects. Thus, the present disclosure has developed a therapeutic agent with balanced GLP-1R/GIPR/GCGR triple receptor agonistic activity, good stability, and a sufficiently long duration of action to support low-frequency and high-efficiency administration, such as once a week, once every two weeks, or even once a month.

Based on this, in a first aspect of the present disclosure, a polypeptide or a derivative thereof or a pharmaceutically acceptable salt thereof is provided. According to an embodiment of the present disclosure, the polypeptide or the derivative thereof has a structure represented by Formula (I):

X₁X₂X₃GTX₆X₇X₈DX₁₀X₁₁X₁₂X₁₃X₁₄X₁₅X₁₆X₁₇X₁₈X₁₉X₂₀X₂₁X₂₂X₂₃X₂₄X₂₅LX₂₇X_{2 8}X₂₉X₃₀PX₃₂SX₃₄X₃₅PPPX₃₉ (I);

where X₁ is Y, H, 3-iodo-Y, 4-Pal, or 4-amino-F;
X₂ is Aib, s, or Ac4c;
X₃ is Q, H, or Dab(Ac);
X₆ is F, F(2-F), or αMeF;
X₈ is S, HoS, or αMeS;
X₁₀ is Z₂, Y, Z₁, L, or 4-Pal;
X₁₁ is S or αMeS;
X₁₂ is K, R, or I;
X₁₃ is Z₂, Z₁, Y, L, A, αMeL, Q, Aib, or Iva;
X₁₄ is Z₂, Z₁, L, K(Ac), HoL, Npg, or Tle;
X₁₅ is D, E, or Aad;
X₁₆ is K, E, K(Ac), or αMeK;
X₁₇ is R, Z₂, Z₁, K(Ac), Q, or I;
X₁₈ is Z₂, Z₁, A, R, or Y;
X₁₉ is A, Q, Aib, H, or K(Ac);
X₂₀ is Q, Z₂, Aib, H, R, a, or Z₁;
X₂₁ is Z₁, D, A, L, Z₂, or Aib;
X₂₂ is F or HoF;
X₂₃ is V or I;
X₂₄ is Z₂, Q, Z₁, or E;
X₂₅ is Z₂, Z₁, W, or Y;
X₂₇ is L, K, or I;
X₂₈ is A, D, E, Z₁, or Z₂;
X₂₉ is G or H;
X₃₀ is G or H;
X₃₂ is Z₂, Z₁, S, or P;
X₃₄ is G or Aib;
X₃₅ is A, Q, or K;
X₃₉ is S or K.

In the structure represented by Formula (I), two sites of any one of the following groups are each independently selected from amino acid Z₁ with a side chain containing -SH, or in the structure represented by Formula (I), two sites of any one of the following groups are each independently selected from amino acid Z₂ with a side chain containing -NH₂:
1) i and i+3;
2) i and i+4;
3) i and i+7; or
4) i and i+11;

where i is 10, 14, 17, or 21;
the two amino acids Z₁ with the side chain containing -SH are each independently selected from C, c, αMeC, HoC, Hoc, Pen, or N-Me-C;
the two amino acids Z₂ with the side chain containing -NH₂ are each independently selected from K, k, αMeK, HoK, Dap, Dab, Orn, or N-Me-K.

As an incretin analogue, the above-mentioned polypeptide or the derivative thereof or the pharmaceutically acceptable salt thereof of the present disclosure can exhibit strong agonistic activity on all three receptor targets of GLP-1R, GCGR and GIPR, can effectively control blood sugar and reduce body weight, and can be used for the prevention or treatment of metabolic disorder related diseases, bone-related diseases, cardiovascular diseases and neurodegenerative diseases.

In a second aspect of the present disclosure, a polypeptide derivative or a pharmaceutically acceptable salt thereof is provided. The polypeptide derivative has a structure represented by any one of Table A (see details in claims).

In a third aspect of the present disclosure, a pharmaceutical composition is provided. According to an embodiment of the present disclosure, the pharmaceutical composition includes the polypeptide or the derivative thereof or the pharmaceutically acceptable salt thereof according to the first aspect, or the polypeptide derivative or the pharmaceutically acceptable salt thereof according to the second aspect. It can be seen from the foregoing that the polypeptide or the derivative thereof or the pharmaceutically acceptable salt thereof of the present disclosure exhibits strong agonistic activity on three receptor targets of GLP-1R, GCGR, and GIPR, and is effective in controlling blood sugar and reducing body weight. Thus, the use of a pharmaceutical composition including the polypeptide or the derivative thereof or the pharmaceutically acceptable salt thereof is effective in preventing or treating metabolic disorder related diseases, bone-related diseases, cardiovascular diseases, neurodegenerative diseases, and the like.

In a fourth aspect, use of the polypeptide or the derivative thereof or the pharmaceutically acceptable salt thereof according to the first aspect, the polypeptide derivative or the pharmaceutically acceptable salt thereof according to the second aspect, or the pharmaceutical composition according to the third aspect in the manufacture of a medicament for treating or preventing at least one of a metabolic disorder related disease, a bone-related disease, a cardiovascular disease, or a neurodegenerative disease.

According to an embodiment of the present application, the present disclosure provides use of the polypeptide or the derivative thereof or the pharmaceutically acceptable salt thereof according to the first aspect, or a pharmaceutical composition according to the second aspect in the treatment or prevention of at least one of a metabolic disorder related disease, a bone-related disease, a cardiovascular disease, or a neurodegenerative disease.

According to an embodiment of the present application, the present disclosure provides the polypeptide or the derivative thereof or the pharmaceutically acceptable salt thereof according to the first aspect, or a pharmaceutical composition according to the second aspect for use in treating or preventing at least one of a metabolic disorder related disease, a bone-related disease, a cardiovascular disease, or a neurodegenerative disease.

In a fifth aspect of the present disclosure, the present disclosure provides a method for preventing and/or treating a metabolic disorder related disease, a bone-related disease, a cardiovascular disease, and/or a neurodegenerative disease. According to an embodiment of the present disclosure, the method includes: administering to a subject a pharmaceutically acceptable amount of the polypeptide or the derivative thereof or the pharmaceutically acceptable salt thereof according to the first aspect, or the pharmaceutical composition according to the second aspect. The method of the present disclosure is effective in preventing and/or treating a metabolic disorder related disease, a bone-related disease, a cardiovascular disease, and/or a neurodegenerative disease.

Additional aspects and advantages of the present disclosure will be set forth in part in the description which follows and, in part, will be obvious from the description, or may be learned by practice of the present disclosure.

### BRIEF DESCRIPTION OF DRAWINGS

FIG. 1 is a synthetic route of Staple-A1 in Example 1 of the present disclosure.
FIG. 2 is a schematic diagram showing the procedure for linking linear peptide TG30 and Staple-A1 in Example 1 of the present disclosure.
FIG. 3 is a schematic diagram showing the procedure for linking linear peptide TG85 and Stable B1 in Example 2 of the present disclosure.
FIG. 4 shows the body weight change curve of diet-induced obese (DIO) mice injected with different doses of TG52, the compound of the present disclosure, in Test Example 3 of the present disclosure.
FIG. 5 shows the change curve of total daily food intake of DIO mice in each group injected with different doses of TG52, the compound of the present disclosure, in Test Example 3 of the present disclosure.
FIG. 6 shows the change curve of blood glucose levels in DIO mice in each group injected with different doses of TG52, the compound of the present disclosure, in Test Example 3 of the present disclosure.
FIG. 7 shows the stability test results of compound TG52 and Retatrutide (positive control) in simulated gastric fluid in Test Example 4 of the present disclosure.
FIG. 8 shows the result of measuring the serum albumin binding capacity of the target peptide in Test Example 5 of the present disclosure.
FIG. 9 shows the change in blood insulin levels in DIO mice after long-term treatment with the target peptide in Test Example 6 of the present disclosure.
FIG. 10 shows the fat content results in liver tissue of NASH mouse model after long-term treatment with the target peptide in Test Example 6 of the present disclosure.
FIG. 11 shows the quantitative analysis results of each index in liver tissue of NASH mouse model after long-term treatment with the target peptide in Test Example 6 of the present disclosure.

### DETAILED DESCRIPTION

Hereinafter, embodiments of the present disclosure will be described in detail. The embodiments described below are illustrative and are intended only to explain the present disclosure, and should not be construed as limiting the present disclosure.

It should be illustrated that the terms "first" and "second" are used for descriptive purposes only and should not be construed as indicating or implying relative importance or implicitly indicating the number of technical features indicated. Thus, features defined as "first" or "second" may explicitly or implicitly include one or more of those features. Further, in the illustration of the present disclosure, unless otherwise specified, "more" or "a plurality of" means two or more.

### Detailed description of the present disclosure

### Definitions and general terms

As used herein, the terms "comprise" or "include" are open expressions, meaning they include the elements specified by the present disclosure but do not exclude other elements.

As used herein, the terms "optionally", "optional", or "option" generally indicate that the subsequently described event or circumstance may or may not occur, and the description includes both instances where the event or circumstance occurs and where it does not.

As used herein, the terms "optional substitution", "optionally substituted", and "substituted or unsubstituted" are used interchangeably. Generally, the term "optionally", whether or not it precedes the term "substituted", indicates that one or more hydrogen atoms in a given structure are replaced by the radical of a specified substituent. Unless otherwise indicated, an optional substituent group may substitute at each substitutable position on the group. When more than one site in a given structural formula can be substituted with one or more substituents selected from a specified group, the substituents may be the same or different at each position. The substituents may include, but are not limited to, F, Cl, Br, CN, OH, NH₂, NO₂, and the like.

As used herein, the term "one or more" (e.g., in the definition of substituents for compounds (modification groups) of the general formulae of the present disclosure) means "one, two, three, four, or five, particularly one, two, three, or four, more particularly one, two, or three, and even more particularly one or two".

In addition, it should be noted that, unless otherwise explicitly stated, the descriptive terms "each...independently is", "...each independently is", and "...independently is" used in the present disclosure are interchangeable and should be broadly construed to mean that the specific options expressed by the same symbols in different groups do not affect each other, or that the specific options expressed by the same symbols within the same group do not affect each other.

As used herein, the term "halogen" refers to a fluorine, chlorine, bromine, or iodine atom.

As used herein, the minimum value and maximum value of carbon atoms in a hydrocarbon group are indicated by a prefix. For example, the prefix C_{a-b} means "a" to "b" carbon atoms. Illustratively, "C₁₋ₙ" refers to a straight chain or branched chain saturated/unsaturated carbon chain including 1, 2, 3, 4, 5, ..., or n carbon atoms; it is further understood that "C₁₋ₙ" is to be interpreted as including any subrange therein, for example, in C₁₋₂₀, including C₁₋₂₀, C₁₋₁₈, C₁₀₋₈, C₁₂₋₁₈, C₁₄₋₁₈, C₁₋₁₀, C₁₋₆, C₁₋₃, C₁₋₂, C₂₋₁₀, C₂₋₉, C₂₋₈, C₂₋₇, C₂₋₆, C₂₋₅, C₂₋₄, C₂₋₃, C₃₋₁₀, C₃₋₉, C₃₋₈, C₃₋₇, C₃₋₆, C₃₋₅, C₃₋₄, C₄₋₁₀, C₄₋₉, C₄₋₈, C₄₋₇, C₄₋₆, and C₄₋₅.

As used herein, the term "alkyl" has the general structural formula 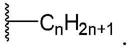 Alkylene can be a straight-chain alkyl or a branched-chain alkyl. Illustratively, the term "C₁₋₂₀ alkyl" refers to an alkyl group having 1 to 20 carbon atoms; the term "C₁₋₆ alkyl" refers to an alkyl group having 1 to 6 carbon atoms.

As used herein, the term "alkylene" is defined by the general structural formula 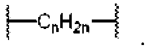 Alkylene can be straight-chain alkylene or branched-chain alkylene. Illustratively, the term "C₁₋₂₀ alkylene" refers to an alkylene group having 1 to 20 carbon atoms; the term "C₁₋₆ alkylene" refers to an alkylene group having 1 to 6 carbon atoms.

As used herein, the term "C₁₋₆ alkoxy" refers to a C₁₋₆ alkyl group containing the formula "-O-alkyl", where the term "alkyl" is as defined above. For example: methoxy, ethoxy, n-propoxy, isopropoxy, n-butoxy, isobutoxy, tert-butoxy, sec-butoxy, pentyloxy, isopentyloxy, and n-hexyloxy, or isomers of the above radicals. In particular, the "C₁₋₆ alkoxy" may contain 1, 2, 3, 4, or 5 carbon atoms ("C₁₋₅ alkoxy"), preferably 1, 2, 3, or 4 carbon atoms ("C₁₋₄ alkoxy").

As used herein, the term "oxyalkylene" refers to a group formed by removing one hydrogen atom from an "oxyalkyl".

As used herein, the term "aryl" refers to carbocyclic ring systems containing monocyclic, bicyclic, and tricyclic rings, in which at least one ring system is aromatic, and each ring system includes 6 to 10 atoms forming the ring. The aryl group is typically, but not necessarily, bound to the parent molecule through an aromatic ring of the aryl group. The term "aryl" is used interchangeably with the terms "aromatic ring" or "aryl ring". Aryl groups can include phenyl, indenyl, naphthyl, and anthracenyl. The aryl group is optionally substituted with one or more substituents described in the present disclosure.

As used herein, the term "arylene" refers to a group formed by removing one hydrogen atom from an "aryl" group.

As used herein, the term "heteroaryl" refers to carbocyclic ring systems that include monocyclic, bicyclic, and tricyclic rings with at least one heteroatom, where at least one ring system is aromatic, and the heteroatom refers to a nitrogen atom, an oxygen atom, or a sulfur atom. It generally denotes a monovalent saturated or partially unsaturated mono- or bicyclic ring system including multiple ring atoms, with 1, 2, or 3 ring heteroatoms selected from N, O, and S, and the remaining ring atoms being carbon.

As used herein, the term "heteroarylene" refers to a group formed by removing one hydrogen atom from a "heteroaryl" group.

The use of 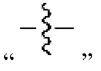 in the description of groups herein is intended to describe the position of substitution by the group.

As used herein, the term "-NH-CO-" or "-NH-C(=O)-" has the following structural formula

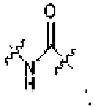

As used herein, the conventional one-letter and three-letter codes for natural amino acids are used, as well as the generally accepted three-letter codes for other α-amino acids. For example, α-aminoisobutyric acid (Aib) can be represented by the codes Aib or B. Unless otherwise specified, all amino acid residues in the present disclosure are preferably in the L-configuration.

The term "Aib" has the structural formula of

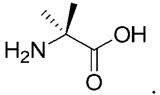

As used herein, the term "Ac4c" has the structural formula of

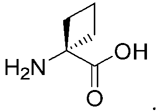

As used herein, the term "αMeC" has the structural formula of

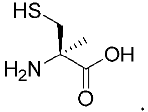

As used herein, the term "αMeL" has the structural formula of

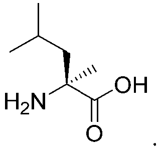

As used herein, the term "αMeS" has the structural formula of

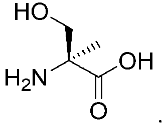

As used herein, the term "αMeF" has the structural formula of

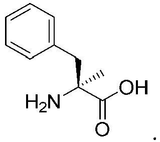

As used herein, the term "αMeK" has the structural formula of

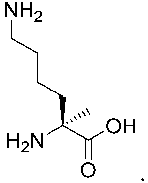

As used herein, the term "Iva" has the structural formula of

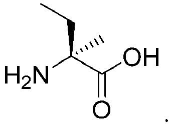

As used herein, the term "F(2-F)" has the structural formula of

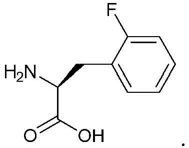

As used herein, the term "4-Pal" has the structural formula of

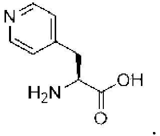

As used herein, the term "K(Ac)" has the structural formula of

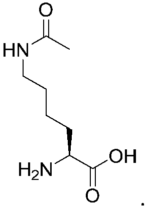

As used herein, the term "HoL" has the structural formula of

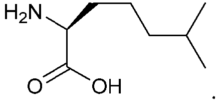

As used herein, the term "Tle" has the structural formula of

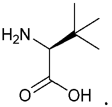

As used herein, the term "Npg" has the structural formula of

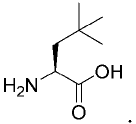

As used herein, the term "HoC" has the structural formula of

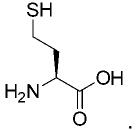

As used herein, the term "Hoc" has the structural formula of

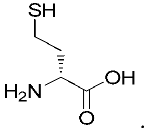

As used herein, the term "HoF" has the structural formula of

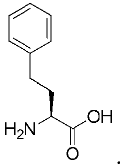

As used herein, the term "HoS" has the structural formula of

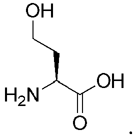

As used herein, the term "HoK" has the structural formula of

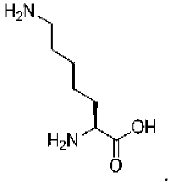

As used herein, the term "3-iodo-Y" has the structural formula of

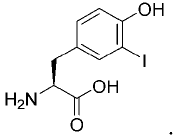

As used herein, the term "4-amino-F" has the structural formula

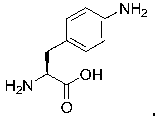

As used herein, the term "Dab(Ac)" has the structural formula

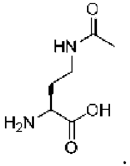

As used herein, the term "Aad" has the structural formula

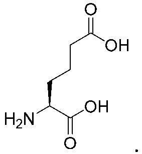

As used herein, the term "Pen" has the structural formula

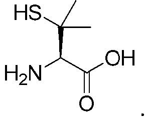

As used herein, the term "N-Me-C" has the structural formula

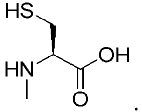

As used herein, the term "N-Me-K" has the structural formula

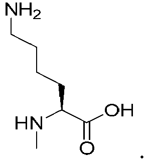

As used herein, the term "Orn" has the structural formula

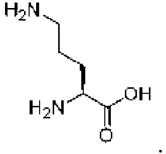

As used herein, the term "Dab" has the structural formula

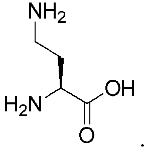

As used herein, the term "Dap" has the structural formula

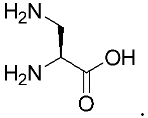

As used herein, a "pharmaceutical composition" can be used in the treatment of a disease or in an in vitro culture experiment of cells. The term "pharmaceutical composition", as used in the treatment of disease, refers generally to a unit dose form and may be prepared by any of the methods well known in the pharmaceutical art. All methods include the step of bringing the active ingredient into association with the excipient, which constitutes one or more accessory ingredients. In general, the compositions are prepared by uniformly and intimately bringing into association the active polypeptide or derivative thereof or the reactivating agent with liquid excipients, finely divided solid excipients, or both.

As used herein, the term "pharmaceutically acceptable" means that the substance or composition must be chemically and/or toxicologically compatible with the other ingredients including the polypeptide or the derivative thereof, and/or with the mammal being treated. Preferably, the term "pharmaceutically acceptable" as used herein means approved by a federal or state government regulatory agency or listed in the U.S. Pharmacopeia or other generally recognized pharmacopeia for use in animals, and more particularly in humans.

As used herein, the term "pharmaceutically acceptable salt" refers to organic and inorganic salts of the polypeptide or the derivative thereof of the present disclosure. Pharmaceutically acceptable salts are well known in the art, e.g., S. M. Berge et al., describe pharmaceutically acceptable salts in detail in J. Pharmaceutical Sciences, 1977, 66: 1-19. Salts formed from pharmaceutically acceptable non-toxic acids include, but are not limited to, salts of inorganic acids (e.g., hydrochloride, hydrobromide, phosphate, sulfate, perchlorate) and organic acids (e.g., acetate, oxalate, maleate, tartrate, citrate, succinate, malonate) formed by reaction with amino groups or obtained by other methods described in the literature, such as ion exchange.

In the chemical structures of the ligands or compounds described in this disclosure, the bond " " represents an unassigned configuration. If chiral isomerism exists in a chemical structure, the bond " 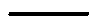 " may be " 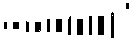 ", " 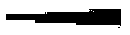 ", or include both " 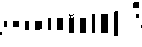 " and " 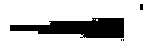 " configurations. While all of the above structural formulae are drawn as certain isomeric forms for convenience, the present disclosure includes all isomers, for example: tautomers, rotamers, geometric isomers, diastereomers, racemates, and enantiomers.

As used herein, the term "pharmaceutically acceptable excipient" can include any solvent, solid excipient, diluent, or other liquid excipient suitable for the particular dose form desired. Except insofar as any conventional excipient is incompatible with the polypeptide or the derivative thereof, the pharmaceutical composition thereof, or drugs containing them, such as by causing any adverse biological effects or interacting in a harmful manner with any other component(s) of the pharmaceutically acceptable composition, their use is within the scope of the present disclosure.

Except insofar as any conventional excipient is incompatible with the polypeptide or the derivative thereof, the pharmaceutical composition thereof, or drugs containing them, such as by causing any adverse biological effects or interacting in a harmful manner with any other component(s) of the pharmaceutically acceptable composition, their use is within the scope of the present disclosure.

The pharmaceutical compositions of the present disclosure includes formulations suitable for parenteral administration. The formulations may conveniently be presented in unit dose form and may be prepared by any of the methods well known in the art of pharmacy. The amount of active ingredient that can be combined with an excipient material to produce a single dose form will generally be the amount of the polypeptide or derivative thereof that produces a therapeutic effect.

As used herein, the term "agonist" refers to a substance (ligand) that activates the type of receptor in question.

As used herein, the term "treatment" or "treating" refers to obtaining a desired pharmacological and/or physiological effect. The effect may be prophylactic in terms of completely or partially preventing a disease or its symptoms, and/or therapeutic in terms of partially or completely curing a disease and/or its adverse effects. As used herein, "treating" or "treatment" encompasses a disease in a mammal, particularly in a human, and includes: (a) preventing the disease or condition from occurring in an individual who may be predisposed to the disease but has not yet been diagnosed as having it; (b) inhibiting the disease, for example, arresting the development of the disease; or (c) relieving the disease, e.g., alleviating symptoms associated with the disease. As used herein, "treatment" or "treating" encompasses any administration of a drug including the polypeptide or the derivative thereof to a subject to treat, cure, alleviate, ameliorate, mitigate, or suppress a disease in the subject, including but not limited to the administration of a drug including the polypeptide or the derivative thereof described herein to a subject in need thereof.

As used herein, the term "non-alcoholic fatty liver disease (NAFLD)" generally refers to a clinicopathological syndrome characterized primarily by excessive deposition of fat in hepatocytes, excluding that caused by alcohol and other well-defined liver-damaging factors. It is an acquired metabolic stress-induced liver injury closely related to insulin resistance and genetic predisposition, including but not limited to simple fatty liver (SFL), non-alcoholic steatohepatitis (NASH), and its associated cirrhosis.

### Detailed description of the polypeptide or the derivative thereof or the pharmaceutically acceptable salt thereof, the pharmaceutical composition, and uses thereof in the present disclosure

The present disclosure provides a polypeptide or a derivative thereof or a pharmaceutically acceptable salt thereof, a pharmaceutical composition, and uses thereof, each of which is described in detail below.

### A polypeptide or a derivative thereof or a pharmaceutically acceptable salt thereof

In a first aspect of the present disclosure, a polypeptide or a derivative thereof or a pharmaceutically acceptable salt thereof is provided. According to an embodiment of the present disclosure, the polypeptide or the derivative thereof has a structure represented by Formula (I):

X₁X₂X₃GTX₆X₇X₈DX₁₀X₁₁X₁₂X₁₃X₁₄X₁₅X₁₆X₁₇X₁₈X₁₉X₂₀X₂₁X₂₂X₂₃X₂₄X₂₅LX₂₇X_{2 8}X₂₉X₃₀PX₃₂SX₃₄X₃₅PPPX₃₉ (I);

where X₁ is Y, H, 3-iodo-Y, 4-Pal, or 4-amino-F;
X₂ is Aib, s, or Ac4c;
X₃ is Q, H, or Dab(Ac);
X₆ is F, F(2-F), or αMeF;
X₈ is S, HoS, or αMeS;
X₁₀ is Z₂, Y, Z₁, L, or 4-Pal;
X₁₁ is S or αMeS;
X₁₂ is K, R, or I;
X₁₃ is Z₂, Z₁, Y, L, A, αMeL, Q, Aib, or Iva;
X₁₄ is Z₂, Z₁, L, K(Ac), HoL, Npg, or Tle;
X₁₅ is D, E, or Aad;
X₁₆ is K, E, K(Ac), or αMeK;
X₁₇ is R, Z₂, Z₁, K(Ac), Q, or I;
X₁₈ is Z₂, Z₁, A, R, or Y;
X₁₉ is A, Q, Aib, H, or K(Ac);
X₂₀ is Q, Z₂, Aib, H, R, a, or Z₁;
X₂₁ is Z₁, D, A, L, Z₂, or Aib;
X₂₂ is F or HoF;
X₂₃ is V or I;
X₂₄ is Z₂, Q, Z₁, or E;
X₂₅ is Z₂, Z₁, W, or Y;
X₂₇ is L, K, or I;
X₂₈ is A, D, E, Z₁, or Z₂;
X₂₉ is G or H;
X₃₀ is G or H;
X₃₂ is Z₂, Z₁, S, or P;
X₃₄ is G or Aib;
X₃₅ is A, Q, or K;
X₃₉ is S or K.

In the structure represented by Formula (I), two sites of any one of the following groups are each independently selected from amino acid Z₁ with a side chain containing -SH, or in the structure represented by Formula (I), two sites of any one of the following groups are each independently selected from amino acid Z₂ with a side chain containing -NH₂:
1) i and i+3;
2) i and i+4;
3) i and i+7; or
4) i and i+11;
where i is 10, 14, 17, or 21.

The two amino acids Z₁ with the side chain containing -SH are each independently selected from C, c, αMeC, HoC, Hoc, Pen, or N-Me-C.

The two amino acids Z₂ with the side chain containing -NH₂ are each independently selected from K, k, αMeK, HoK, Dap, Dab, Orn, or N-Me-K.

The polypeptide or the derivative thereof or the pharmaceutically acceptable salt thereof of the present disclosure exhibits strong agonistic activity against the three receptor targets GLP-1R, GCGR, and GIPR, effectively controls blood sugar and reduces body weight, and can be used for the prevention or treatment of metabolic disorder related diseases, bone-related diseases, cardiovascular diseases, and neurodegenerative diseases.

Unless otherwise illustrated, as used herein, for amino acids abbreviated with a single letter, uppercase letters represent amino acids in the L configuration (e.g., S (i.e., L-configuration Ser), A (i.e., L-configuration Ala), etc.), and lowercase letters represent amino acids in the D configuration (e.g., s (i.e., D-configuration Ser), a (i.e., D-configuration Ala), etc.).

As used herein, Xᵢ in the structure represented by Formula (I) represents an amino acid, where i represents the site of the amino acid. For example, X₉ is the ninth amino acid in the structure represented by Formula (I).

It should be noted that, the two sites in group 1) are i and i+3; the two sites in group 2) are i and i+4; the two sites in group 3) are i and i+7; the two sites in group 4) are i and i+11. Also, the two sites in groups 1) to 4) are each amino acid Z₁, or the two sites in groups 1) to 4) are each amino acid Z₂. The two amino acids Z₁ in each group of sites may be the same or different, and the two amino acids Z₂ in each group of sites may be the same or different.

In some preferred embodiments of the present disclosure, the two amino acids Z₁ in the structure represented by Formula (I) are the same.

In some preferred embodiments of the present disclosure, the two amino acids Z₂ in the structure represented by Formula (I) are the same.

According to an embodiment of the present disclosure, the two sites i and i+3, i and i+4, i and i+7, or i and i+11 in the structure represented by Formula (I) are the amino acids Z₁ with the side chain containing -SH, with i being 10, 14, 17, or 21.

According to an embodiment of the present disclosure, the two sites i and i+3 in the structure represented by Formula (I) are the amino acids Z₁ with the side chain containing -SH, with i being 10, 14, 17, or 21, preferably i being 17.

According to an embodiment of the present disclosure, the two sites i and i+4 in the structure represented by Formula (I) are the amino acids Z₁ with the side chain containing -SH, with i being 10, 14, 17, or 21, preferably i being 10.

According to an embodiment of the present disclosure, the two sites i and i+7 in the structure represented by Formula (I) are the amino acids Z₁ with the side chain containing -SH, with i being 10, 14, 17, or 21, preferably i being 14, 17, or 21.

According to an embodiment of the present disclosure, the two sites i and i+11 in the structure represented by Formula (I) are the amino acids Z₁ with the side chain containing -SH, with i being 10, 14, 17, or 21, preferably i being 10 or 17.

According to an embodiment of the present disclosure, the two sites i and i+3, i and i+4, i and i+7, or i and i+11 in the structure represented by Formula (I) are the amino acids Z₂ with the side chain containing -NH₂, i being 10, 14, 17, or 21.

According to an embodiment of the present disclosure, the two sites i and i+3, or i and i+7 in the structure represented by Formula (I) are the amino acids Z₂ with the side chain containing -NH₂, with i being 17 or 21.

According to an embodiment of the present disclosure, the two sites 17 and 20 or 21 and 28 in the structure represented by Formula (I) are the amino acids Z₂ with the side chain containing -NH₂.

According to an embodiment of the present disclosure, X₁ is Y or H.

According to an embodiment of the present disclosure, X₂ is Aib or Ac4c.

According to an embodiment of the present disclosure, X₃ is Q or H.

According to an embodiment of the present disclosure, X₆ is F or F(2-F).

According to an embodiment of the present disclosure, X₆ is F(2-F).

According to an embodiment of the present disclosure, X₁₀ is Y, C, L, or 4-Pal.

According to an embodiment of the present disclosure, X₁₀ is Y, L, or 4-Pal.

According to an embodiment of the present disclosure, X₁₀ is Y or 4-Pal.

According to an embodiment of the present disclosure, X₁₁ is S or αMeS.

According to an embodiment of the present disclosure, X₁₂ is K or I.

According to an embodiment of the present disclosure, X₁₃ is Y, L, A, αMeL, Q, Aib, or Iva.

According to an embodiment of the present disclosure, X₁₃ is Y, L, αMeL, Q, Aib, or Iva.

According to an embodiment of the present disclosure, X₁₃ is Y, L, αMeL, Q, or Iva.

According to an embodiment of the present disclosure, X₁₃ is L or αMeL.

According to an embodiment of the present disclosure, X₁₃ is Y, L, or A.

According to an embodiment of the present disclosure, X₁₃ is αMeL, Y, or Iva.

According to an embodiment of the present disclosure, X₁₃ is αMeL, Q, or L.

According to an embodiment of the present disclosure, X₁₃ is Y or L.

According to an embodiment of the present disclosure, X₁₄ is Z₁, L, K(Ac), HoL, Npg, or Tle.

According to an embodiment of the present disclosure, X₁₄ is L, K(Ac), HoL, Npg, or Tle.

According to an embodiment of the present disclosure, X₁₄ is C, L, K(Ac), HoL, Npg, or Tle.

According to an embodiment of the present disclosure, X₁₅ is D or E.

According to an embodiment of the present disclosure, X₁₆ is K or E.

According to an embodiment of the present disclosure, X₁₆ is E, K, αMeK, or K(Ac).

According to an embodiment of the present disclosure, X₁₆ is K or αMeK.

According to an embodiment of the present disclosure, X₁₇ is Z₁, Q, K(Ac), R, or I.

According to an embodiment of the present disclosure, X₁₇ is C, Q, K(Ac), R, or I.

According to an embodiment of the present disclosure, X₁₇ is R, Z₂, K(Ac), Q, or I.

According to an embodiment of the present disclosure, X₁₇ is R, K, K(Ac), Q, or I.

According to an embodiment of the present disclosure, X₁₇ is K(Ac) or K.

According to an embodiment of the present disclosure, X₁₇ is C or K(Ac).

According to an embodiment of the present disclosure, X₁₇ is R, K, or Q.

According to an embodiment of the present disclosure, X₁₈ is A, R, or Y.

According to an embodiment of the present disclosure, X₁₈ is A or Y.

According to an embodiment of the present disclosure, X₁₉ is A, Q, or Aib.

According to an embodiment of the present disclosure, X₁₉ is Q or A.

According to an embodiment of the present disclosure, X₁₉ is Q or H.

According to an embodiment of the present disclosure, X₂₀ is Q, Z₂, Aib, H, a, or Z₁.

According to an embodiment of the present disclosure, X₂₀ is Q, K, Aib, H, a, or C.

According to an embodiment of the present disclosure, X₂₀ is Aib, Q, H, Z₁, or a.

According to an embodiment of the present disclosure, X₂₀ is Aib, Q, H, C, or a.

According to an embodiment of the present disclosure, X₂₀ is Q, Aib, H, or R.

According to an embodiment of the present disclosure, X₂₀ is Q, Z_{2,} or Aib.

According to an embodiment of the present disclosure, X₂₀ is Q, K, or Aib.

According to an embodiment of the present disclosure, X₂₀ is Aib or Q.

According to an embodiment of the present disclosure, X₂₀ is Aib or K.

According to an embodiment of the present disclosure, X₂₁ is Z₁, D, A, or Z₂.

According to an embodiment of the present disclosure, X₂₁ is Z₁, D, A, L, or Aib.

According to an embodiment of the present disclosure, X₂₁ is C, D, A, L, or Aib.

According to an embodiment of the present disclosure, X₂₁ is D, A, or C.

According to an embodiment of the present disclosure, X₂₁ is D, A, or L.

According to an embodiment of the present disclosure, X₂₁ is K or A.

According to an embodiment of the present disclosure, X₂₁ is D or C.

According to an embodiment of the present disclosure, X₂₂ is F or HoF.

According to an embodiment of the present disclosure, X₂₃ is I or V.

According to an embodiment of the present disclosure, X₂₄ is Q, Z_{1,} or E.

According to an embodiment of the present disclosure, X₂₄ is E, C, or Q.

According to an embodiment of the present disclosure, X₂₄ is E or C.

According to an embodiment of the present disclosure, X₂₄ is Q or E.

According to an embodiment of the present disclosure, X₂₅ is W or Y.

According to an embodiment of the present disclosure, X₂₈ is A, E, Z_{1,} or Z₂.

According to an embodiment of the present disclosure, X₂₈ is A, E, or Z₁.

According to an embodiment of the present disclosure, X₂₈ is A, D, E, or Z₁.

According to an embodiment of the present disclosure, X₂₈ is A, D, E, C, or HoC.

According to an embodiment of the present disclosure, X₂₈ is A, D, or E.

According to an embodiment of the present disclosure, X₂₈ is A or E.

According to an embodiment of the present disclosure, X₂₈ is E or C.

According to an embodiment of the present disclosure, X₂₈ is K or A.

According to an embodiment of the present disclosure, X₃₂ is S or P.

According to an embodiment of the present disclosure, X₃₅ is A or Q.

According to an embodiment of the present disclosure, X₃₅ is A or K.

According to an embodiment of the present disclosure, X₃₉ is S, the C-terminus of the S being amidated.

According to an embodiment of the present disclosure, the polypeptide or the derivative thereof represented by Formula (I) has a structure represented by Formula (Ia):

X₁X₂X₃GTX₆TSDX₁₀X₁₁X₁₂X₁₃X₁₄X₁₅X₁₆X₁₇X₁₈X₁₉X₂₀X₂₁FX₂₃X₂₄X₂₅LLX₂₈GGP SSGGPPPS (Ia).

In some alternative embodiments of the present disclosure, in the structure represented by Formula (Ia), X₁ is Y, or H;
X₂ is Aib, s, or Ac4c;
X₃ is Q, H, or Dab(Ac);
X₆ is F, F(2-F), or αMeF;
X₁₀ is Y or 4-Pal;
X₁₁ is S or αMeS;
X₁₂ is K or I;
X₁₃ is Y, L, αMeL, Q, or Iva;
X₁₄ is Z₁, L, K(Ac), HoL, Npg or Tle;
X₁₅ is D or E;
X₁₆ is K, E, K(Ac), or αMeK;
X₁₇ is R, Z₂, Z₁, K(Ac), Q, or I;
X₁₈ is A or Y;
X₁₉ is A or Q;
X₂₀ is Q, Z₂, Aib, H, a, or Z₁;
X₂₁ is Z₁, D, A, Z₁ or Z₂;
X₂₃ is V or I;
X₂₄ is Q, Z₁ or E;
X₂₅ is W or Y;
X₂₈ is A, E, Z_{1,} or Z₂.

In some alternative embodiments of the present disclosure, in the structure represented by Formula (Ia), X₁ is Y or H;
X₂ is Aib, s, or Ac4c;
X₃ is Q, H, or Dab(Ac);
X₆ is F, F(2-F), or αMeF;
X₁₀ is Y or 4-Pal;
X₁₁ is S or αMeS;
X₁₂ is K or I;
X₁₃ is Y, L, αMeL, Q, or Iva;
X₁₄ is Z₁, L, K(Ac), HoL, Npg or Tle;
X₁₅ is D or E;
X₁₆ is K, E, K(Ac), or αMeK;
X₁₇ is R, Z₂, Z₁, K(Ac), Q, or I;
X₁₈ is A or Y;
X₁₉ is A or Q;
X₂₀ is Q, Z₂, Aib, H, a, or Z₁;
X₂₁ is Z₁, D, A, or Z₂;
X₂₃ is V or I;
X₂₄ is Q, Z₁ or E;
X₂₅ is W or Y;
X₂₈ is E or Z₁.

In some alternative embodiments of the present disclosure, in the structure represented by Formula (Ia), X₁ is Y or H.

In some alternative embodiments of the present disclosure, in the structure represented by Formula (Ia), X₁₀ is Y or 4-Pal.

In some alternative embodiments of the present disclosure, in the structure represented by Formula (Ia), X₁₂ is K or I.

In some alternative embodiments of the present disclosure, in the structure represented by Formula (Ia), X₁₃ is Y, L, αMeL, Q, or Iva.

In some alternative embodiments of the present disclosure, in the structure represented by Formula (Ia), X₁₄ is C, L, K(Ac), HoL, Npg, or Tle.

In some alternative embodiments of the present disclosure, in the structure represented by Formula (Ia), X₁₅ is D or E.

In some alternative embodiments of the present disclosure, in the structure represented by Formula (Ia), X₁₆ is E, K, αMeK, or K(Ac).

In some alternative embodiments of the present disclosure, in the structure represented by Formula (Ia), X₁₇ is C, Q, K(Ac), R, or I.

In some alternative embodiments of the present disclosure, in the structure represented by Formula (Ia), X₁₈ is A or Y.

In some alternative embodiments of the present disclosure, in the structure represented by Formula (Ia), X₁₉ is Q or A.

In some alternative embodiments of the present disclosure, in the structure represented by Formula (Ia), X₂₀ is Aib, Q, H, C, or a.

In some alternative embodiments of the present disclosure, in the structure represented by Formula (Ia), X₂₁ is D, A, or C.

In some alternative embodiments of the present disclosure, in the structure represented by Formula (Ia), X₂₄ is E, C, or Q.

In some alternative embodiments of the present disclosure, in the structure represented by Formula (Ia), X₂₅ is W or Y.

In some alternative embodiments of the present disclosure, in the structure represented by Formula (Ia), X₂₈ is E or C.

In some alternative embodiments of the present disclosure, sites 14 and 21, 17 and 24, 21 and 28, or 17 and 20 in the structure represented by Formula (Ia) are the two amino acids Z₁ with the side chain containing -SH.

In some alternative embodiments of the present disclosure, sites 21 and 28, or 17 and 20 in the structure represented by Formula (Ia) are the two amino acids Z₂ with the side chain containing -NH₂.

In some alternative embodiments of the present disclosure, sites 14 and 21 in the structure represented by Formula (Ia) are the two amino acids Z₁ with the side chain containing -SH.

In some alternative embodiments of the present disclosure, sites 17 and 24 in the structure represented by Formula (Ia) are the two amino acids Z₁ with the side chain containing -SH.

In some alternative embodiments of the present disclosure, sites 21 and 28 in the structure represented by Formula (Ia) are the two amino acids Z₁ with the side chain containing -SH.

In some alternative embodiments of the present disclosure, sites 17 and 20 in the structure represented by Formula (Ia) are the two amino acids Z₁ with the side chain containing -SH.

In some alternative embodiments of the present disclosure, in the structure represented by Formula (Ia), the two amino acids Z₁ are each independently selected from C, c, αMeC, HoC, Hoc, Pen, or N-Me-C.

In some alternative embodiments of the present disclosure, in the structure represented by Formula (Ia), the two amino acids Z₁ are each independently selected from C, c, αMeC, or HoC.

In some alternative embodiments of the present disclosure, in the structure represented by Formula (Ia), the two amino acids Z₁ are each C.

In some alternative embodiments of the present disclosure, in the structure represented by Formula (Ia), the two amino acids Z₁ are each c.

In some alternative embodiments of the present disclosure, in the structure represented by Formula (Ia), the two amino acids Z₁ are each αMeC.

In some alternative embodiments of the present disclosure, in the structure represented by Formula (Ia), the two amino acids Z₁ are each HoC.

In some alternative embodiments of the present disclosure, sites 21 and 28 in the structure represented by Formula (Ia) are the two amino acids Z₂ with the side chain containing -NH₂.

In some alternative embodiments of the present disclosure, sites 17 and 20 in the structure represented by Formula (Ia) are the two amino acids Z₂ with the side chain containing -NH₂.

In some alternative embodiments of the present disclosure, in the structure represented by Formula (Ia), the two amino acids Z₂ are each independently selected from K, k, αMeK, HoK, Dap, Dab, Orn, or N-Me-K.

In some alternative embodiments of the present disclosure, in the structure represented by Formula (Ia), the two amino acids Z₂ are each independently selected from K or Orn.

In some alternative embodiments of the present disclosure, in the structure represented by Formula (Ia), the two amino acids Z₂ are each K.

In some alternative embodiments of the present disclosure, in the structure represented by Formula (Ia), the two amino acids Z₂ are each Orn.

According to an embodiment of the present disclosure, the polypeptide or the derivative thereof represented by Formula (I) has a structure represented by Formula (Ib):

X₁X₂X₃GTX₆TSDYX₁₁X₁₂X₁₃LDX₁₆X₁₇X₁₈X₁₉X₂₀X₂₁FX₂₃X₂₄X₂₅LLX₂₈GGPSSG GPPPS (Ib).

In some alternative embodiments of the present disclosure, in the structure represented by Formula (Ib), X₁ is Y or H.

In some alternative embodiments of the present disclosure, in the structure represented by Formula (Ib), X₂ is Aib or Ac4c.

In some alternative embodiments of the present disclosure, in the structure represented by Formula (Ib), X₃ is Q or H.

In some alternative embodiments of the present disclosure, in the structure represented by Formula (Ib), X₆ is F or F(2-F).

In some alternative embodiments of the present disclosure, in the structure represented by Formula (Ib), X₁₁ is S or αMeS.

In some alternative embodiments of the present disclosure, in the structure represented by Formula (Ib), X₁₂ is K or I.

In some alternative embodiments of the present disclosure, in the structure represented by Formula (Ib), X₁₃ is αMeL, Y, or Iva.

In some alternative embodiments of the present disclosure, in the structure represented by Formula (Ib), X₁₆ is K or αMeK.

In some alternative embodiments of the present disclosure, in the structure represented by Formula (Ib), X₁₇ is C or K(Ac).

In some alternative embodiments of the present disclosure, in the structure represented by Formula (Ib), X₁₈ is A or Y.

In some alternative embodiments of the present disclosure, in the structure represented by Formula (Ib), X₁₉ is Q or A.

In some alternative embodiments of the present disclosure, in the structure represented by Formula (Ib), X₂₀ is Aib or Q.

In some alternative embodiments of the present disclosure, in the structure represented by Formula (Ib), X₂₁ is D or C.

In some alternative embodiments of the present disclosure, in the structure represented by Formula (Ib), X₂₃ is I or V.

In some alternative embodiments of the present disclosure, in the structure represented by Formula (Ib), X₂₄ is E or C.

In some alternative embodiments of the present disclosure, in the structure represented by Formula (Ib), X₂₅ is W or Y.

In some alternative embodiments of the present disclosure, in the structure represented by Formula (Ib), X₂₈ is E or C.

In some alternative embodiments of the present disclosure, sites 17 and 24, or 21 and 28 in the structure represented by Formula (Ib) are the two amino acids Z₁ with the side chain containing -SH.

In some alternative embodiments of the present disclosure, sites 17 and 24 in the structure represented by Formula (Ib) are the two amino acids Z₁ with the side chain containing -SH.

In some alternative embodiments of the present disclosure, sites 21 and 28 in the structure represented by Formula (Ib) are the two amino acids Z₁ with the side chain containing -SH.

In some alternative embodiments of the present disclosure, in the structure represented by Formula (Ib), the two amino acids Z₁ are each independently selected from C, c, αMeC, HoC, Hoc, Pen, or N-Me-C.

In some alternative embodiments of the present disclosure, in the structure represented by Formula (Ib), the two amino acids Z₁ are each independently selected from C, c, αMeC, or HoC.

In some alternative embodiments of the present disclosure, in the structure represented by Formula (Ib), the two amino acids Z₁ are each C.

In some alternative embodiments of the present disclosure, in the structure represented by Formula (Ib), the two amino acids Z₁ are each c.

In some alternative embodiments of the present disclosure, in the structure represented by Formula (Ib), the two amino acids Z₁ are each αMeC.

In some alternative embodiments of the present disclosure, in the structure represented by Formula (Ib), the two amino acids Z₁ are each HoC.

According to an embodiment of the present disclosure, the polypeptide or the derivative thereof represented by Formula (I) has a structure represented by Formula (II):

The polypeptide or the derivative thereof represented by Formula (I) has a structure represented by Formula (II):

X₁X₂X₃GTX₆X₇X₈DX₁₀X₁₁X₁₂X₁₃X₁₄X₁₅X₁₆X₁₇X₁₈X₁₉X₂₀X₂₁FX₂₃X₂₄X₂₅LX₂₇X₂₈ X₂₉X₃₀PX₃₂SX₃₄X₃₅PPPX₃₉ (II).

In some alternative embodiments of the present disclosure, in the structure represented by Formula (II), X₁₀ is Y, C, L, or 4-Pal.

In some alternative embodiments of the present disclosure, in the structure represented by Formula (II), X₁₃ is Y, L, A, αMeL, Q, Aib, or Iva.

In some alternative embodiments of the present disclosure, in the structure represented by Formula (II), X₁₄ is C, L, K(Ac), HoL, Npg, or Tle.

In some alternative embodiments of the present disclosure, in the structure represented by Formula (II), X₁₈ is A, R, or Y.

In some alternative embodiments of the present disclosure, in the structure represented by Formula (II), X₂₁ is C, D, A, L, or Aib.

In some alternative embodiments of the present disclosure, in the structure represented by Formula (II), X₂₄ is Q, C, or E.

In some alternative embodiments of the present disclosure, in the structure represented by Formula (II), X₂₅ is W or Y.

In some alternative embodiments of the present disclosure, in the structure represented by Formula (II), X₂₈ is A, D, E, C, or HoC.

In some alternative embodiments of the present disclosure, in the structure represented by Formula (II), X₃₂ is S or P.

In some alternative embodiments of the present disclosure, sites of the following combinations in the structure represented by Formula (II) are the two amino acids Z₁ with the side chain containing -SH:
i and i+3, i being 17;
i and i+4, i being 10;
i and i+7, i being 10, 14, 17, or 21; or
i and i+11, i being 10 or 17.

In some alternative embodiments of the present disclosure, sites 14 and 21 in the structure represented by Formula (II) are the two amino acids Z₁ with the side chain containing -SH.

In some alternative embodiments of the present disclosure, sites 17 and 20 in the structure represented by Formula (II) are the two amino acids Z₁ with the side chain containing -SH.

In some alternative embodiments of the present disclosure, sites 10 and 14 in the structure represented by Formula (II) are the two amino acids Z₁ with the side chain containing -SH.

In some alternative embodiments of the present disclosure, sites 10 and 17 in the structure represented by Formula (II) are the two amino acids Z₁ with the side chain containing -SH.

In some alternative embodiments of the present disclosure, sites 17 and 24 in the structure represented by Formula (II) are the two amino acids Z₁ with the side chain containing -SH.

In some alternative embodiments of the present disclosure, sites 21 and 28 in the structure represented by Formula (II) are the two amino acids Z₁ with the side chain containing -SH.

In some alternative embodiments of the present disclosure, sites 10 and 21 in the structure represented by Formula (II) are the two amino acids Z₁ with the side chain containing -SH.

In some alternative embodiments of the present disclosure, sites 17 and 28 in the structure represented by Formula (II) are the two amino acids Z₁ with the side chain containing -SH.

In some alternative embodiments of the present disclosure, in the structure represented by Formula (II), the two amino acids Z₁ are each independently selected from C, c, αMeC, HoC, Hoc, Pen, or N-Me-C.

In some alternative embodiments of the present disclosure, in the structure represented by Formula (II), the two amino acids Z₁ are each independently selected from C, c, αMeC, or HoC.

In some alternative embodiments of the present disclosure, in the structure represented by Formula (II), the two amino acids Z₁ are each C.

In some alternative embodiments of the present disclosure, in the structure represented by Formula (II), the two amino acids Z₁ are each c.

In some alternative embodiments of the present disclosure, in the structure represented by Formula (II), the two amino acids Z₁ are each αMeC.

In some alternative embodiments of the present disclosure, in the structure represented by Formula (II), the two amino acids Z₁ are each HoC.

According to an embodiment of the present disclosure, the polypeptide or the derivative thereof represented by Formula (I) has a structure represented by Formula (III):

X₁-Aib-X₃GTX₆TSDYSX₁₂X₁₃CCX₁₅X₁₆X₁₇X₁₈X₁₉X₂₀CCFX₂₃X₂₄X₂₅LLX₂₈X₂₉X_{3 0}PSSGX₃₅PPPS (III).

In some alternative embodiments of the present disclosure, in the structure represented by Formula (III), X₁ is Y or H.

In some alternative embodiments of the present disclosure, in the structure represented by Formula (III), X₃ is Q or H, preferably H.

In some alternative embodiments of the present disclosure, in the structure represented by Formula (III), X₆ is F or F(2-F), preferably F.

In some alternative embodiments of the present disclosure, in the structure represented by Formula (III), X₁₂ is K or I, preferably I.

In some alternative embodiments of the present disclosure, in the structure represented by Formula (III), X₁₃ is Y or L, preferably Y.

In some alternative embodiments of the present disclosure, in the structure represented by Formula (III), X₁₅ is D or E, preferably E.

In some alternative embodiments of the present disclosure, in the structure represented by Formula (III), X₁₆ is K or E, preferably K.

In some alternative embodiments of the present disclosure, in the structure represented by Formula (III), X₁₇ is R, K, or Q, preferably R.

In some alternative embodiments of the present disclosure, in the structure represented by Formula (III), X₁₈ is A, R, or Y, preferably Y.

In some alternative embodiments of the present disclosure, in the structure represented by Formula (III), X₁₉ is A or Q, preferably A.

In some alternative embodiments of the present disclosure, in the structure represented by Formula (III), X₂₀ is Q, K, or Aib, preferably Aib.

In some alternative embodiments of the present disclosure, in the structure represented by Formula (III), X₂₄ is Q or E, preferably Q.

In some alternative embodiments of the present disclosure, in the structure represented by Formula (III), X₂₅ is W or Y, preferably W.

In some alternative embodiments of the present disclosure, in the structure represented by Formula (III), X₂₈ is A, D, or E, preferably E.

In some alternative embodiments of the present disclosure, in the structure represented by Formula (III), X₃₅ is A or Q, preferably A.

In some alternative embodiments of the present disclosure, X₁ is Y or H; X₃ is Q or H; X₆ is F or F(2-F); X₁₂ is K or I; X₁₃ is Y or L; X₁₅ is D or E; X₁₆ is K or E; X₁₇ is R, K, or Q; X₁₈ is A, R, or Y; X₁₉ is A or Q; X₂₀ is Q, K, or Aib; X₂₃ is V or I; X₂₄ is Q or E; X₂₅ is W or Y; X₂₈ is A, D, or E; X₂₉ is G or H; X₃₀ is G or H; and X₃₅ is A or Q.

In some alternative embodiments of the present disclosure, X₁ is Y or H; X₃ is H; X₆ is F or F(2-F), X₁₂ is I; X₁₃ is Y; X₁₅ is E; X₁₆ is K; X₁₇ is R; X₁₈ is Y; X₁₉ is A; X₂₀ is Aib; X₂₃ is V or I; X₂₄ is Q; X₂₅ is W; X₂₈ is E; X₂₉ is G or H; X₃₀ is G or H; and X₃₅ is A.

According to an embodiment of the present disclosure, the polypeptide or the derivative thereof represented by Formula (I) has a structure represented by Formula (IV):

X₁X₂X₃GT-F(2-F)-TSDYX₁₁IX₁₃LDKCAX₁₉CAFIEYLLX₂₈GGPSSGAPPPS (IV).

In some alternative embodiments of the present disclosure, in the structure represented by Formula (IV), X₁ is Y or H, preferably Y.

In some alternative embodiments of the present disclosure, in the structure represented by Formula (IV), X₂ is Aib.

In some alternative embodiments of the present disclosure, in the structure represented by Formula (IV), X₃ is Q or H, preferably Q.

In some alternative embodiments of the present disclosure, in the structure represented by Formula (IV), X₁₃ is L or αMeL, preferably αMeL.

In some alternative embodiments of the present disclosure, in the structure represented by Formula (IV), X₁₉ is Q or H, preferably Q.

In some alternative embodiments of the present disclosure, in the structure represented by Formula (IV), X₂₈ is A or E, preferably A.

In some alternative embodiments of the present disclosure, X₁ is Y or H, X₂ is Aib, s, Ac4c or V, X₃ is Q or H, X₁₁ is S or αMeS, X₁₃ is L or αMeL, X₁₉ is Q or H, and X₂₈ is A or E.

In some alternative embodiments of the present disclosure, X₁ is Y; X₂ is Aib; X₃ is Q; X₁₁ is S or αMeS; X₁₃ is αMeL; X₁₉ is Q; X₂₈ is A.

According to an embodiment of the present disclosure, the polypeptide or the derivative thereof represented by Formula (I) has a structure represented by Formula (V):

X₁-Aib-X₃GTX₆TSDYSX₁₂X₁₃LX₁₅KCX₁₈X₁₉X₂₀X₂₁FX₂₃CX₂₅LLX₂₈GGPSSGAP PPS (V).

In some alternative embodiments of the present disclosure, in the structure represented by Formula (V), X₁ is Y or H.

In some alternative embodiments of the present disclosure, in the structure represented by Formula (V), X₃ is Q or H.

In some alternative embodiments of the present disclosure, in the structure represented by Formula (V), X₆ is F or F(2-F), preferably F.

In some alternative embodiments of the present disclosure, in the structure represented by Formula (V), X₁₂ is K or I.

In some alternative embodiments of the present disclosure, in the structure represented by Formula (V), X₁₃ is Y, L, or A, preferably Y or L.

In some alternative embodiments of the present disclosure, in the structure represented by Formula (V), X₁₅ is D or E, preferably D.

In some alternative embodiments of the present disclosure, in the structure represented by Formula (V), X₁₈ is A or Y.

In some alternative embodiments of the present disclosure, in the structure represented by Formula (V), X₁₉ is A or Q.

In some alternative embodiments of the present disclosure, in the structure represented by Formula (V), X₂₀ is Q, Aib, H, or R, preferably Q or Aib.

In some alternative embodiments of the present disclosure, in the structure represented by Formula (V), X₂₁ is D, A, or L, preferably D or A.

In some alternative embodiments of the present disclosure, in the structure represented by Formula (V), X₂₅ is W or Y.

In some alternative embodiments of the present disclosure, in the structure represented by Formula (V), X₂₈ is A or E, preferably E.

In some alternative embodiments of the present disclosure, X₁ is Y or H, X₃ is Q or H, X₆ is F or F(2-F), X₁₂ is K or I, X₁₃ is Y, L, or A, X₁₅ is D or E, X₁₈ is A or Y, X₁₉ is A or Q, X₂₀ is Q, Aib, H, or R, X₂₁ is D, A, or L, X₂₅ is W or Y, and X₂₈ is A or E.

In some alternative embodiments of the present disclosure, X₁ is Y or H; X₃ is Q or H; X₆ is F; X₁₂ is K or I; X₁₃ is Y or L; X₁₅ is D; X₁₈ is A or Y; X₁₉ is A or Q; X₂₀ is Q or Aib; X₂₁ is D or A; X₂₅ is W or Y; and X₂₈ is E.

According to an embodiment of the present disclosure, the polypeptide or the derivative thereof represented by Formula (I) has a structure represented by Formula (VI):

Y-Aib-QGTFTSDYSILLDKZ₁AQH-Aib-FIEYLLZ₁GGPSSGAPPPS (VI).

In some alternative embodiments of the present disclosure, two Z₁ are each independently selected from C or HoC.

In some alternative embodiments of the present disclosure, two Z₁ are each C.

In some alternative embodiments of the present disclosure, two Z₁ are each HoC.

According to an embodiment of the present disclosure, the polypeptide or the derivative thereof represented by Formula (I) has a structure represented by Formula (VII):

X₁X₂X₃GTX₆TX₈DX₁₀X₁₁X₁₂X₁₃X₁₄X₁₅X₁₆X₁₇X₁₈X₁₉X₂₀Z₁X₂₂X₂₃X₂₄X₂₅LX₂₇Z₁G GPX₃₂SX₃₄X₃₅PPPS (VII).

In some alternative embodiments of the present disclosure, in the structure represented by Formula (VII), X₁ is Y or H.

In some alternative embodiments of the present disclosure, in the structure represented by Formula (VII), X₂ is Aib, s, or Ac4c.

In some alternative embodiments of the present disclosure, in the structure represented by Formula (VII), X₁₀ is Y, L, or 4-Pal, preferably Y or 4-Pal.

In some alternative embodiments of the present disclosure, in the structure represented by Formula (VII), X₁₁ is S.

In some alternative embodiments of the present disclosure, in the structure represented by Formula (VII), X₁₂ is K or I.

In some alternative embodiments of the present disclosure, in the structure represented by Formula (VII), X₁₃ is Y, L, αMeL, Q, Aib, or Iva, preferably Y, L, αMeL, Q, or Iva.

In some alternative embodiments of the present disclosure, in the structure represented by Formula (VII), X₁₄ is L, K(Ac), HoL, Npg, or Tle.

In some alternative embodiments of the present disclosure, in the structure represented by Formula (VII), X₁₅ is D or E.

In some alternative embodiments of the present disclosure, in the structure represented by Formula (VII), X₁₆ is K(Ac), K, αMeK, or E.

In some alternative embodiments of the present disclosure, in the structure represented by Formula (VII), X₁₇ is R, K, K(Ac), Q, or I.

In some alternative embodiments of the present disclosure, in the structure represented by Formula (VII), X₁₈ is A, R, or Y, preferably Y or A.

In some alternative embodiments of the present disclosure, in the structure represented by Formula (VII), X₁₉ is A, Q, or Aib.

In some alternative embodiments of the present disclosure, in the structure represented by Formula (VII), X₂₀ is Q, K, Aib, H, a, or C, preferably Aib, a, H, or Q.

In some alternative embodiments of the present disclosure, in the structure represented by Formula (VII), X₂₂ is F or HoF, preferably F.

In some alternative embodiments of the present disclosure, in the structure represented by Formula (VII), X₂₄ is Q or E.

In some alternative embodiments of the present disclosure, in the structure represented by Formula (VII), X₂₅ is W or Y.

In some alternative embodiments of the present disclosure, in the structure represented by Formula (VII), X₃₂ is S or P, preferably S.

In some alternative embodiments of the present disclosure, in the structure represented by Formula (VII), X₃₅ is A or K, preferably A.

In some alternative embodiments of the present disclosure, in the structure represented by Formula (VII), the two Z₁ are each independently selected from C, c, HoC, or αMeC.

In some alternative embodiments of the present disclosure, in the structure represented by Formula (VII), two Z₁ are each C.

In some alternative embodiments of the present disclosure, in the structure represented by Formula (VII), two Z₁ are each c.

In some alternative embodiments of the present disclosure, in the structure represented by Formula (VII), two Z₁ are each HoC.

In some alternative embodiments of the present disclosure, in the structure represented by Formula (VII), two Z₁ are each αMeC.

In some alternative embodiments of the present disclosure, X₁ is Y or H; X₂ is Aib, s, or Ac4c; X₃ is Q, H, or Dab(Ac); X₆ is F, F(2-F), or αMeF; X₈ is S, HoS, or αMeS; X₁₀ is Y, L, or 4-Pal; X₁₁ is S; X₁₂ is K or I; X₁₃ is Y, L, αMeL, Q, Aib, or Iva; X₁₄ is L, K(Ac), HoL, Npg, or Tle; X₁₅ is D or E; X₁₆ is K(Ac), K, αMeK, or E; X₁₇ is R, K, K(Ac), Q, or I; X₁₈ is A, R, or Y; X₁₉ is A, Q or Aib; X₂₀ is Q, K, Aib, H, a or C; X₂₁ is C, c, HoC, or αMeC; X₂₂ is F or HoF; X₂₃ is V or I; X₂₄ is Q or E; X₂₅ is W or Y; X₂₇ is L, K, or I; X₂₈ is C, c, HoC, or αMeC; X₃₂ is S or P; X₃₄ is G or Aib; and X₃₅ is A or K.

In some alternative embodiments of the present disclosure, X₁ is Y or H; X₂ is Aib, s, or Ac4c; X₃ is Q, H, or Dab(Ac); X₆ is F, F(2-F), or αMeF; X₈ is S, HoS, or αMeS; X₁₀ is Y or 4-Pal; X₁₁ is S; X₁₂ is K or I; X₁₃ is Y, L, αMeL, Q or Iva; X₁₄ is L, K(Ac), HoL, Npg, or Tle; X₁₅ is D or E; X₁₆ is K(Ac), K, αMeK, or E; X₁₇ is R, K, K(Ac), Q, or I; X₁₈ is Y or A; X₁₉ is A, Q or Aib; X₂₀ is Q, K, Aib, H, a or C; X₂₁ is C, c, HoC, or αMeC; X₂₂ is F; X₂₃ is V or I; X₂₄ is Q or E; X₂₅ is W or Y; X₂₇ is L, K, or I; X₂₈ is C, c, HoC, or αMeC; X₃₄ is G or Aib; X₃₂ is S; and X₃₅ is A.

According to an embodiment of the present disclosure, the polypeptide or the derivative thereof represented by Formula (I) has a structure represented by Formula (VIII):

YX₂QGTX₆TSDYSIX₁₃LDX₁₆X₁₇AQX₂₀X₂₁FIEYLLX₂₈GGPSSGAPPPS (VIII).

In some alternative embodiments of the present disclosure, in the structure represented by Formula (VIII), X₂ is Aib or Ac4c.

In some alternative embodiments of the present disclosure, in the structure represented by Formula (VIII), X₆ is F(2-F) or F.

In some alternative embodiments of the present disclosure, in the structure represented by Formula (VIII), X₁₃ is αMeL, Q, or L.

In some alternative embodiments of the present disclosure, in the structure represented by Formula (VIII), X₁₆ is αMeK or K.

In some alternative embodiments of the present disclosure, in the structure represented by Formula (VIII), X₁₇ is K(Ac) or K.

In some alternative embodiments of the present disclosure, in the structure represented by Formula (VIII), X₂₀ is Aib or K.

In some alternative embodiments of the present disclosure, in the structure represented by Formula (VIII), X₂₁ is K or A.

In some alternative embodiments of the present disclosure, in the structure represented by Formula (VIII), X₂₈ is K or A.

In some alternative embodiments of the present disclosure, sites 17 and 20, or 21 and 28 in the structure represented by Formula (VIII) are the two amino acids Z₂ with the side chain containing -NH₂.

In some alternative embodiments of the present disclosure, sites 17 and 20 in the structure represented by Formula (VIII) are the two amino acids Z₂ with the side chain containing -NH₂.

In some alternative embodiments of the present disclosure, sites 21 and 28 in the structure represented by Formula (VIII) are the two amino acids Z₂ with the side chain containing -NH₂.

In some alternative embodiments of the present disclosure, in the structure represented by Formula (VIII), the two amino acids Z₂ are each independently selected from K, k, αMeK, HoK, Dap, Dab, Orn, or N-Me-K, preferably K or Orn.

In some alternative embodiments of the present disclosure, in the structure represented by Formula (VIII), the two amino acids Z₂ are each K.

In some alternative embodiments of the present disclosure, in the structure represented by Formula (VIII), the two amino acids Z₂ are each Orn.

According to an embodiment of the present disclosure, the polypeptide or the derivative thereof represented by Formula (I) has a structure represented by Formula (IX):

Y-Aib-QGTX₆TSDYSI-αMeL-LDX₁₆-K(Ac)-AQ-Aib-Z₂FIEYLLZ₂GGPSSGAPP PS (IX).

In some alternative embodiments of the present disclosure, in the structure represented by Formula (IX), X₆ is F(2-F).

In some alternative embodiments of the present disclosure, in the structure represented by Formula (IX), X₁₆ is αMeK or K.

In some alternative embodiments of the present disclosure, in the structure represented by Formula (IX), two Z₂ are each independently selected from K or Orn.

In some alternative embodiments of the present disclosure, in the structure represented by Formula (IX), two Z₂ are each K.

In some alternative embodiments of the present disclosure, in the structure represented by Formula (IX), two Z₂ are each Orn.

According to an embodiment of the present disclosure, the polypeptide or the derivative thereof represented by Formula (I) has a structure represented by Formula (X):

YX₂QGTX₆TSDYSIX₁₃LDKKAQKAFIEYLLAGGPSSGAPPPS (X).

In some alternative embodiments of the present disclosure, in the structure represented by Formula (X), X₂ is Aib or Ac4c.

In some alternative embodiments of the present disclosure, in the structure represented by Formula (X), X₆ is F(2-F) or F.

In some alternative embodiments of the present disclosure, in the structure represented by Formula (X), X₁₃ is αMeL, Q, or L.

According to an embodiment of the present disclosure, the polypeptide or the derivative thereof represented by Formula (I) has at least one of the following structures:
Y-Aib-QGTFTSDYSKYCDKRAAQCFVQWLLAGGPSSGAPPPS;
Y-Aib-QGTFTSDYSKYCDEKRAKCFVQWLLDHHPSSGQPPPS;
Y-Aib-QGTFTSDCSKYCDERAAQDFVQWLLAGGPSSGAPPPS;
Y-Aib-QGTFTSDCSKYLDECAAQDFVQWLLAGGPSSGAPPPS;
Y-Aib-QGTFTSDCSKYLDERAAQCFVQWLLAGGPSSGAPPPS;
H-Aib-HGTFTSDYSIYCEKKYAQCFVQWLLAGGPSSGAPPPS;
H-Aib-HGTFTSDYSIYCEKRYAQCFVQWLLAGGPSSGAPPPS;
H-Aib-HGTFTSDYSIYCEKRYA-Aib-CFVQWLLEGGPSSGAPPPS;
Y-Aib-QGTFTSDYSKYLDKCAAQDFVCWLLAGGPSSGAPPPS;
H-Aib-HGTFTSDYSKYLDKCYAQDFVCWLLEGGPSSGAPPPS;
Y-Aib-QGTFTSDYSILLDKCAQHAFICYLLEGGPSSGAPPPS;
Y-Aib-QGTFTSDYSILLDKCAQ-Aib-AFICYLLEGGPSSGAPPPS;
H-Aib-HGTFTSDYSRALEKCAARLFICWLLEGGPSSGAPPPS;
H-Aib-HGTFTSDYSKYLE-K(Ac)-KYA-Aib-CFVQWLLCGGPSSGAPPPS;
Y-Aib-QGTFTSDYSI-αMeL-LDK-K(Ac)-AQ-Aib-CFIEYLLCGGPSSGAPPPS;
H-Aib-HGTFTSDLSKL-K(Ac)-EEQRQ-Aib-CFIEWLKCGGPPS-Aib-KPPPK;
H-Aib-QGTFTSDLSKQ-K(Ac)-DEQRAKCFIEWLICGGPSSGAPPPS;
H-Aib-QGTFTSDYSI-αMeL-LDK-K(Ac)-AQ-Aib-CFIEYLLCGGPSSGAPPPS;

(3-iodo-Y)-Aib-QGTFTSDYSI-αMeL-LDK-K(Ac)-AQ-Aib-CFIEYLLCGGPSSG
APPPS;

(4-Pal)-Aib-QGTFTSDYSI-αMeL-LDK-K(Ac)-AQ-Aib-CFIEYLLCGGPSSGAP
PPS;

(4-amino-F)-Aib-QGTFTSDYSI-αMeL-LDK-K(Ac)-AQ-Aib-CFIEYLLCGGPSS
GAPPPS;

Y-s-QGTFTSDYSI-αMeL-LDK-K(Ac)-AQ-Aib-CFIEYLLCGGPSSGAPPPS;

Y-Aib-QGT-αMeF-TSDYSI-αMeL-LDK-K(Ac)-AQ-Aib-CFIEYLLCGGPSSGAP
PPS;

Y-Aib-QGTFT-HoS-DYSI-αMeL-LDK-K(Ac)-AQ-Aib-CFIEYLLCGGPSSGAPP
PS;

Y-Aib-QGTFTSDYSI-αMeL-HoL-DK-K(Ac)-AQ-Aib-CFIEYLLCGGPSSGAPP
PS;

Y-Aib-QGTFTSDYSI-αMeL-Npg-DK-K(Ac)-AQ-Aib-CFIEYLLCGGPSSGAPP
PS;

Y-Aib-QGTFTSDYSI-αMeL-Tle-DK-K(Ac)-AQ-Aib-CFIEYLLCGGPSSGAPPP
S;

Y-Aib-QGTFTSDYSI-αMeL-LDK-K(Ac)-AQ-Aib-CFIEWLLCGGPSSGAPPPS;
Y-Aib-QGTFTSDYSILLDK-K(Ac)-AQ-Aib-CFIEWLLCGGPSSGAPPPS;
Y-Aib-QGTFTSDYSI-αMeL-LDKQAQ-Aib-CFIEYLLCGGPSSGAPPPS;
Y-Aib-QGTFTSDYSI-αMeL-LDK-K(Ac)-AQ-a-CFIEYLLCGGPSSGAPPPS;
Y-Aib-QGTFTSDYSI-αMeL-LDK-K(Ac)-AQHCFIEYLLCGGPSSGAPPPS;
Y-Aib-QGTFTSDYSI-αMeL-LDK-K(Ac)-AQQCFIEYLLCGGPSSGAPPPS;
Y-Aib-QGTFTSDYSI-αMeL-LDK-K(Ac)-A-Aib-QCFIEYLLCGGPSSGAPPPS;
Y-Aib-QGTFTSDYSI-αMeL-LDKRAQ-Aib-CFIEYLLCGGPSSGAPPPS;
Y-Aib-QGTFTSDYSI-αMeL-LEK-K(Ac)-AQ-Aib-CFIEYLLCGGPSSGAPPPS;

Y-Aib-QGTFTSDYSI-αMeL-L-Aad-K-K(Ac)-AQ-Aib-CFIEYLLCGGPSSGAPP
PS;

Y-Aib-QGTFTSD-4-Pal-SI-αMeL-LDK-K(Ac)-AQ-Aib-CFIEYLLCGGPSSGAP
PPS;

Y-Aib-QGTFT-αMeS-DYSI-αMeL-LDK-K(Ac)-AQ-Aib-CFIEYLLCGGPSSGAP
PPS;

Y-Aib-QGT-F(2-F)-TSDYSI-αMeL-LDK-K(Ac)-AQ-Aib-CFIEYLLCGGPSSGA
PPPS;

Y-Aib-Dab(Ac)-GTFTSDYSI-αMeL-LDK-K(Ac)-AQ-Aib-CFIEYLLCGGPSSG
APPPS;

Y-Aib-HGTFTSDYSI-αMeL-LDK-K(Ac)-AQ-Aib-CFIEYLLCGGPSSGAPPPS;
Y-Ac4c-QGTFTSDYSI-αMeL-LDK-K(Ac)-AQ-Aib-CFIEYLLCGGPSSGAPPPS ;
Y-Aib-QGTFTSDYSILLDKCAQH-Aib-FIEYLLCGGPSSGAPPPS;
Y-Aib-QGTFTSDYSILLDK-HoC-AQH-Aib-FIEYLL-HoC-GGPSSGAPPPS;
Y-Aib-QGT-F(2-F)-TSDYSILLDKCAQQAFICYLLAGGPSSGAPPPS;
Y-Aib-QGT-F(2-F)-TSDYSILLDKQAQQCFIEYLLCGGPSSGAPPPS;
Y-Aib-QGT-F(2-F)-TSDYSI-Aib-LDK-K(Ac)-AQQCFIEYLLCGGPSSGAPPPS;
Y-Aib-QGT-F(2-F)-TSDYSILCDKQAQQCFIEYLLAGGPSSGAPPPS;

Y-Aib-QGT-F(2-F)-TSDYSILLD-αMeK-K(Ac)-AQQCFIEYLLCGGPSSGAPPP
S;

Y-Aib-QGT-F(2-F)-TSDYSILLD-αMeK-K(Ac)-AQ-Aib-CFIEYLICGGPSSGAP
PPS;

Y-Aib-QGT-F(2-F)-TSDYSI-Iva-LDK-K(Ac)-AQ-Aib-CFIEYLLCGGPSSGAPP
PS;

Y-Aib-QGT-F(2-F)-TSDY-αMeS-ILLD-αMeK-K(Ac)-AQQCFIEYLLCGGPSSG
APPPS;

Y-Aib-QGT-F(2-F)-TSDYSI-αMeL-LD-αMeK-K(Ac)-AQQCFIEYLLCGGPSSG
APPPS;

Y-Aib-QGT-F(2-F)-TSDYSI-αMeL-LD-αMeK-K(Ac)-AQQC-HoF-IEYLLCGGP
SSGAPPPS;

Y-Aib-QGT-F(2-F)-TSDYSI-αMeL-LDKCAQCAFIEYLLAGGPSSGAPPPS;
Y-Aib-QGT-F(2-F)-TSDYSI-Iva-LDKCAQCAFIEYLLAGGPSSGAPPPS;

Y-Aib-QGT-F(2-F)-TSDY-αMeS-I-αMeL-LD-αMeK-K(Ac)-AQ-Aib-CFIEYLLC
GGPSSGAPPPS;

Y-Aib-QGT-F(2-F)-TSDY-αMeS-I-αMeL-LD-αMeK-CAQCAFIEYLLAGGPSSG
APPPS;

H-Aib-HGT-F(2-F)-TSDYSIYLE-K(Ac)-KYA-Aib-CFVQWLLCGGPSSGAPPPS ;
H-Aib-HGT-F(2-F)-TSDYSIQ-K(Ac)-EEIAQ-Aib-CFIEWLLCGGPSSGAPPPS;
H-Aib-HGT-F(2-F)-TSDYSIY-K(Ac)-EERAQ-Aib-CFIEWLLCGGPSSGAPPPS;

H-Aib-HGT-F(2-F)-TSDYSI-Iva-LDK-K(Ac)-AQ-Aib-CFIEYLLCGGPSSGAPP
PS;

Y-Aib-QGT-F(2-F)-TSDYSI-αMeL-LDKCAHCAFIEYLLAGGPSSGAPPPS;

Y-Aib-QGT-F(2-F)-TSDYSI-αMeL-LDKCA-K(Ac)-CAFIEYLLAGGPSSGAPPP
S;

Y-Aib-QGT-F(2-F)-TSDYSI-αMeL-LDKCAQCAFIEYLLAGGPSSGAPPPS;
H-Aib-HGT-F(2-F)-TSDYSI-αMeL-LDKCAQCAFIEYLLAGGPSSGAPPPS;
Y-s-QGT-F(2-F)-TSDYSI-αMeL-LDKCAQCAFIEYLLAGGPSSGAPPPS;
Y-Aib-QGTFTSDYSILLDKQAQQCFIEYLLCGGPSSGAPPPS;
Y-Ac4c-QGT-F(2-F)-TSDYSILLDKCAQCAFIEYLLEGGPSSGAPPPS;
Y-AC4c-QGT-F(2-F)-TSDYSILLDK-K(Ac)-AQ-Aib-CFIEYLLCGGPSSGAPPS ;
H-Ac4c-HGT-F(2-F)-TSDYSIQLEEIAQ-Aib-CFIEYLLCGGPSSGAPPPS;
Y-Aib-QGTFTSDYSILLDK-K(Ac)-AQ-Aib-c-FIEYLL-c-GGPSSGAPPPS;
Y-Aib-QGTFTSDYSILLDK-K(Ac)-AQ-Aib-Hoc-FIEYLL-Hoc-GGPSSGAPPPS;

Y-Aib-QGTFTSDYSILLDK-K(Ac)-AQ-Aib-αMeC-FIEYLL-αMeC-GGPSSGAP
PPS;

Y-Aib-QGT-F(2-F)-TSDYSIQLDK-K(Ac)-AQ-Aib-CFIEYLLCGGPSSGAPPPS;

Y-Aib-QGT-F(2-F)-TSDYSI-αMeL-LD-αMeK-K(Ac)-AQ-Aib-KFIEYLLKGGPS
SGAPPPS;

Y-Aib-QGT-F(2-F)-TSDYSI-αMeL-LDK-K(Ac)-AQ-Aib-KFIEYLLKGGPSSGA
PPPS;

Y-Aib-QGTFTSDYSILLDK-K(Ac)-AQ-Aib-Orn-FIEYLL-Orn-GGPSSGAPPPS;
Y-Aib-QGT-F(2-F)-TSDYSI-αMeL-LDKKAQKAFIEYLLAGGPSSGAPPPS;
Y-Aib-QGT-F(2-F)-TSDYSIQLDKKAQKAFIEYLLAGGPSSGAPPPS;
Y-Aib-QGTFTSDYSILLDKKAQKAFIEYLLAGGPSSGAPPPS;
Y-Aib-QGT-F(2-F)-TSDYSI-αMeL-LDKKAQKAFIEYLLAGGPSSGAPPPS.

It should be noted that the "-" between amino acids in the above-mentioned polypeptide illustrates an amide bond; illustratively, the "-" in "Y-Aib-Q" represents an amide bond.

As used herein, "(3-iodo-Y)" means 3-iodo-Y, "(4-Pal)" means 4-Pal, and "(4-amino-F)" means 4-amino-F. The "-" in F(2-F), (3-iodo-Y), (4-Pal), and (4-amino-F) does not represent an amide bond.

According to an embodiment of the present disclosure, it further includes a modification group.

According to an embodiment of the present disclosure, the modification group is bound to one or both of the two amino acids Z₁ with the side chain containing -SH, or one or both of the two amino acids Z₂ with the side chain containing -NH₂, in the polypeptide or the derivative thereof represented by Formula (I).

According to an embodiment of the present disclosure, the modification group is bound to the both two amino acids Z₁ with the side chain containing -SH in the polypeptide or the derivative thereof represented by Formula (I); or
the modification group is bound to the both two amino acids Z₂ with the side chain containing -NH₂ in the polypeptide or the derivative thereof represented by Formula (I).

According to an embodiment of the present disclosure, the modification group is bound to the -SH of the side chain of amino acid C via a sulfur-carbon bond.

According to an embodiment of the present disclosure, the modification group is bound to the ε-amino group of the side chain of amino acid K via an amide bond.

According to an embodiment of the present disclosure, the modification group has a structure represented by Formula (XI): 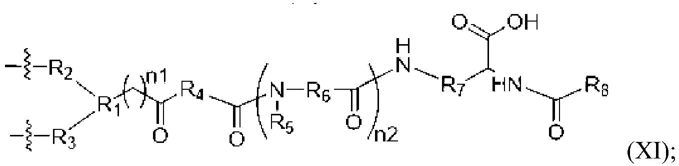
where R₁ is C, N, -C₃₋₁₀ heteroalkylene, -C₆₋₁₀ arylene, or -C₅₋₁₀ heteroarylene;
R₂ and R₃ are each independently -C₁₋₆ alkylene optionally substituted with one or more R₁ₐ, -NH-C(O)-C₁₋₆ alkylene optionally substituted with one or more R₁ₐ, or -C₁₋₆ alkylene-NH-C(O)-C₁₋₆ alkylene optionally substituted with one or more R₁ₐ, wherein each R₁ₐ is independently halogen, -OH, -SH, -NH₂, -NO₂, -CN, phenyl, -C₁₋₆ alkyl, -C₁₋₆ haloalkyl, or -C₁₋₆ alkoxy;
R₄ is absent, or -C₁₋₆ alkylene-NH-C(O)-C₁₋₆ alkylene optionally substituted with one or more R₂ₐ, wherein each R₂ₐ is independently halogen, -OH, -SH, -NH₂, -NO₂, -CN, phenyl, -C₁₋₆ alkyl, -C₁₋₆ haloalkyl, or -C₁₋₆ alkoxy;
R₅ is H, -C₁₋₆ alkoxy, or -C₁₋₆ alkyl, wherein the -C₁₋₆ alkyl and -C₁₋₆ alkoxy are each independently optionally substituted with one or more halogen, -OH, -C(O)OH, -C(O)-, -SH, -NH₂, -NO₂, or -CN;
R₆ is -C₁₋₆ alkylene- optionally substituted with one or more R₃ₐ, or -(C₁₋₃ alkylene -O)ₘ₁-C₁₋₆ alkylene- optionally substituted with one or more R₃ₐ, wherein each R₃ₐ is independently halogen, -OH, -SH, -NH₂, -NO₂, -CN, phenyl, -C₁₋₆ alkyl, -C₁₋₆ haloalkyl, or -C₁₋₆ alkoxy;
R₇ is -C₁₋₆ alkylene- optionally substituted with one or more R₄ₐ, wherein each R₄ₐ is independently halogen, -OH, -SH, -NH₂, -NO₂, -CN, phenyl, -C₁₋₆ alkyl, -C₁₋₆ haloalkyl, or -C₁₋₆ alkoxy;
R₈ is -C₁₀₋₂₀ alkyl optionally substituted with one or more R₅ₐ, -C₁₀₋₂₀ alkylene-R₉ optionally substituted with one or more R₅ₐ, -C₅₋₁₀ alkylene-O-C₆₋₁₀ arylene-R₉ optionally substituted with one or more R₅ₐ, or -C₅₋₁₀ alkylene-O-C₅₋₁₀ heteroarylene-R₉ optionally substituted with one or more R₅ₐ, wherein each R₅ₐ is independently halogen, -OH, -SH, -NH₂, -NO₂, -CN, phenyl, -C₁₋₆ alkyl, -C₁₋₆ haloalkyl, or -C₁₋₆ alkoxy;
R₉ is -COOH, -C₃₋₇ heteroaryl, -S(O)₂OH, or -PO(OH)₂;
m1 is any integer from 1 to 6;
n1 is any integer from 0 to 6;
n2 is any integer from 1 to 10.

It is to be noted that "-" in the modification group in the present disclosure represents a chemical bond that connects chemical groups, such as a covalent bond between an atom (or an atom in a group) and an atom (or an atom in a group).

According to an embodiment of the present disclosure, R₁ is C, N, -C₅₋₇ heteroalkylene, -C₅₋₇ arylene, or -C₅₋₇ heteroarylene.

According to an embodiment of the present disclosure, R₁ is N, phenylene, or pyridinylene.

According to an embodiment of the present disclosure, R₁ is 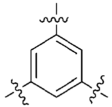 or

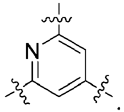

According to an embodiment of the present disclosure, R₂ and R₃ are each independently -C₁₋₆ alkylene-, or -C₁₋₆ alkylene-NH-C(O)-C₁₋₆ alkylene-.

According to an embodiment of the present disclosure, R₂ and R₃ are each independently -C₁₋₃ alkylene-, or -C₁₋₃ alkylene-NH-C(O)-C₁₋₃ alkylene-.

According to an embodiment of the present disclosure, each n1 is independently 0, 1, 2, 3, 4, or 5.

According to an embodiment of the present disclosure, n1 is 0.

According to an embodiment of the present disclosure, R₄ is absent, or -C₁₋₆ alkylene-NH-C(O)-C₁₋₆ alkylene-.

According to an embodiment of the present disclosure, R₄ is absent, or -C₁₋₃ alkylene-NH-C(O)-C₁₋₃ alkylene-.

According to an embodiment of the present disclosure, R₅ is H, -C₁₋₃ alkoxy, or -C₁₋₃ alkyl.

According to an embodiment of the present disclosure, R₆ is -C₁₋₆ alkylene-, -(C₁₋₃ alkylene-O)ₘ₁-C₁₋₆ alkylene-.

According to an embodiment of the present disclosure, R₆ is -C₁₋₃ alkylene-, -(C₁₋₃ alkylen -O)ₘ₁-C₁₋₃ alkylene-.

According to an embodiment of the present disclosure, m1 is 2, 3, 4, or 5.

According to an embodiment of the present disclosure, R₇ is -C₁₋₆ alkylene-.

According to an embodiment of the present disclosure, R₇ is -C₂₋₄ alkylene-.

According to an embodiment of the present disclosure, R₈ is -C₁₀₋₂₀ alkyl, -C₁₀₋₂₀ alkylene-R₉, -C₅₋₁₀ alkylene-O-C₆₋₁₀ arylene-R₉, or -C₅₋₁₀ alkylene-O-C₅₋₁₀ heteroarylene-R₉.

According to an embodiment of the present disclosure, R₈ is -C₁₀₋₁₈ alkyl, -C₁₄₋₁₈ alkylene-R₉, or -C₇₋₉ alkylene-O-C₅₋₇ arylene-COOH.

According to an embodiment of the present disclosure, R₉ is -COOH, -C₅₋₆ heteroaryl, -S(O)₂OH, or -PO(OH)₂.

According to an embodiment of the present disclosure, R₉ is -COOH, -S(O)₂OH, -PO(OH)₂, or

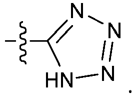

According to an embodiment of the present disclosure, n2 is 1, 2, or 3.

According to an embodiment of the present disclosure, n2 is 3, 4, 5, or 6.

According to an embodiment of the present disclosure, the modification group represented by Formula (XI) has a structure represented by Formula (XIa): 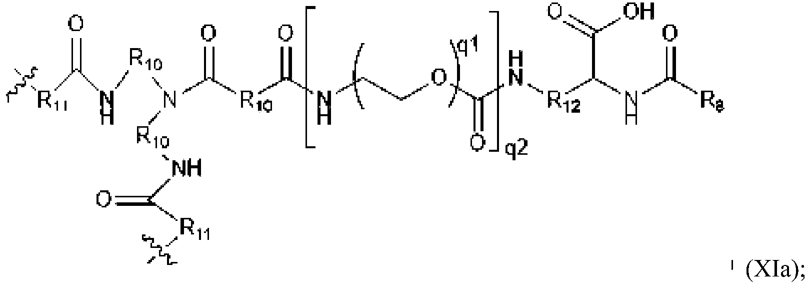
wherein each R₁₀ and R₁₁ are each independently -C₀₋₃ alkylene-;
R₁₂ is -C₁₋₆ alkylene-;
R₈ is -C₁₀₋₂₁ alkyl, -C₁₀₋₂₁ alkylene-COOH, -C₁₀₋₂₁ alkylene-PO(OH)₂, -C₁₀₋₂₁ alkylene-C₅₋₆ heteroaryl, or -C₇₋₁₀ alkylene-O-C₅₋₇ arylene-COOH;
q1 is 1, 2, 3, or 4;
q2 is 1, 2, or 3.

According to an embodiment of the present disclosure, in the structure represented by Formula (XIa), each R₁₀ and R₁₁ is independently -C₀₋₃ alkylene-;
R₁₂ is -C₁₋₆ alkylene-;
R₈ is -C₁₄₋₁₉ alkyl or -C₁₀₋₁₉ alkylene-COOH;
q1 is 1, 2, 3, or 4;
q2 is 1, 2, or 3.

According to an embodiment of the present disclosure, the modification group represented by Formula (XIa) has one of the structures as shown below:

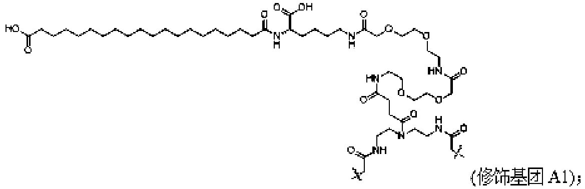

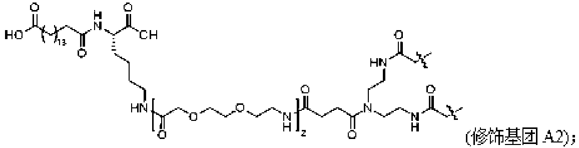

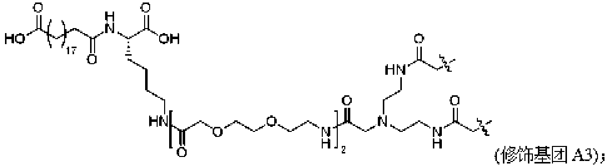

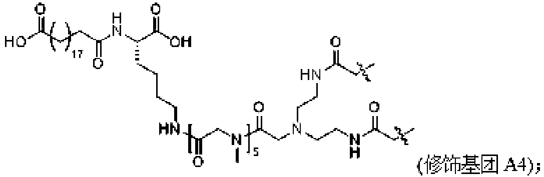

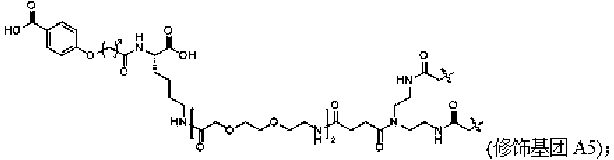

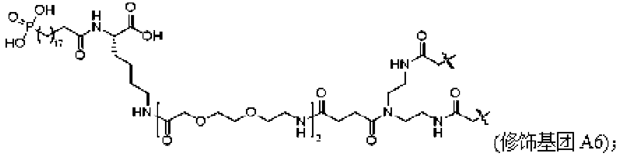

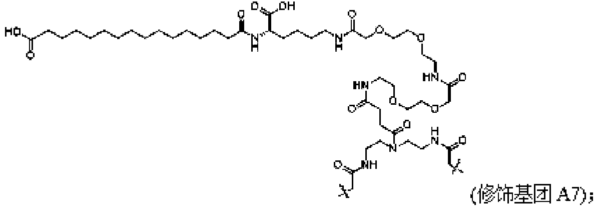

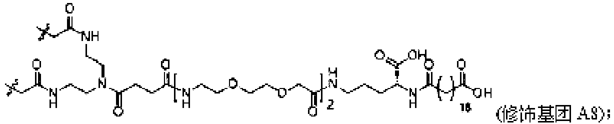

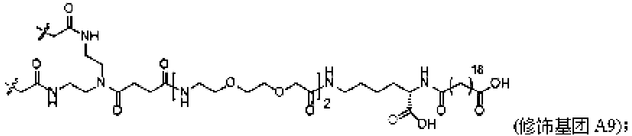

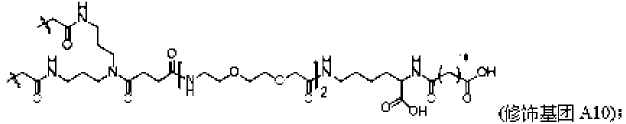

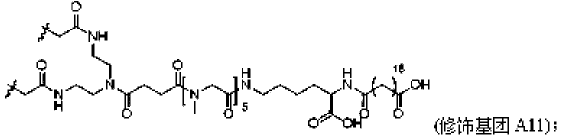

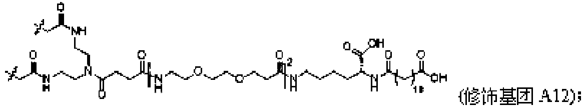

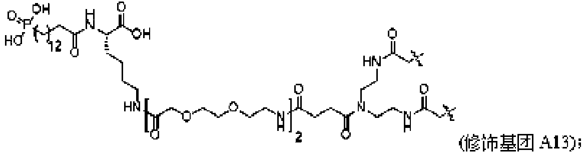

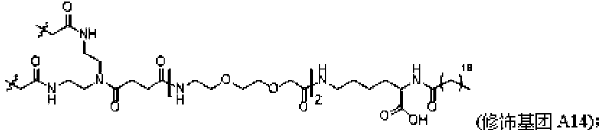

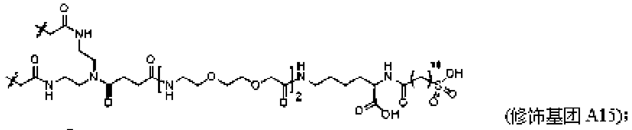

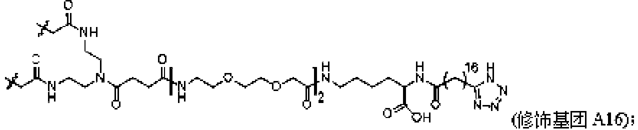

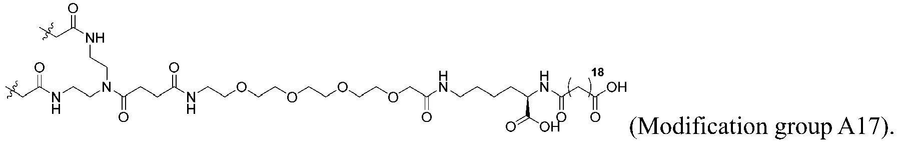

According to an embodiment of the present disclosure, the modification group represented by Formula (XI) has a structure represented by Formula (XIb): 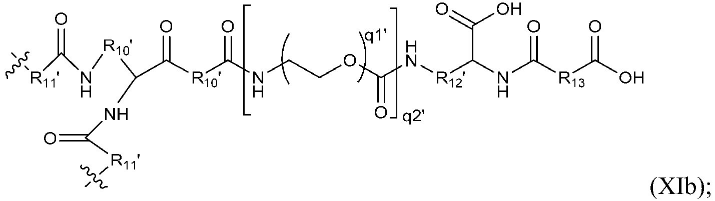
where each R₁₀' and R₁₁' are each independently -C₀₋₃ alkylene-;
R₁₂' is -C₁₋₆ alkylene-;
R₁₃ is -C₁₀₋₂₀ alkylene-, preferably -C₁₄₋₁₈ alkylene-;
q1' is 1, 2, 3, or 4;
q2' is 1, 2, or 3.

According to an embodiment of the present disclosure, in the structure represented by Formula (XIb), each R₁₀' and R₁₁' are each independently -C₀₋₃ alkylene-;
R₁₂' is -C₁₋₆ alkylene-;
R₁₃ is -C₁₄₋₁₈ alkylene-;
q1' is 1, 2, 3, or 4;
q2' is 1, 2, or 3.

According to an embodiment of the present disclosure, the modification group represented by Formula (XIb) has one of the structures as shown below:

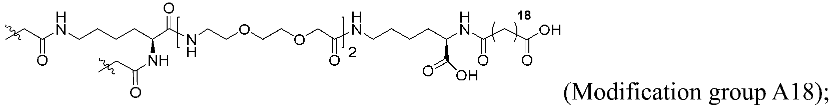

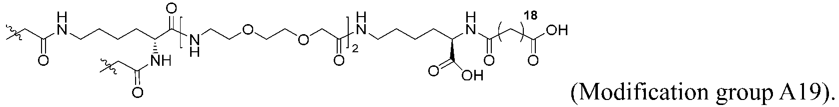

According to an embodiment of the present disclosure, the modification group represented by Formula (XI) has a structure represented by Formula (XIc): 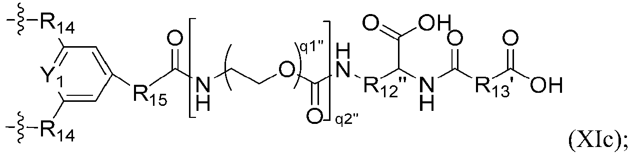
where each R₁₄ is independently -C₁₋₃ alkylene- or -NH-C(O)-C₁₋₃ alkylene-;
R₁₅ is -C₀₋₃ alkylene- or -C(O)-NH-C₁₋₃ alkylene-;
R₁₂'' is -C₁₋₆ alkylene-;
R₁₃' is -C₁₀₋₂₀ alkylene-, preferably -C₁₄₋₁₈ alkylene-;
q1'' is 1, 2, 3, or 4;
q2'' is 1, 2, or 3;
Y₁ is C or N.

According to an embodiment of the present disclosure, in the structure represented by Formula (XIc), each R₁₄ is independently -C₁₋₃ alkylene- or -NH-C(O)-C₁₋₃ alkylene-;
R₁₅ is -C₀₋₃ alkylene- or -C(O)-NH-C₁₋₃ alkylene-;
R₁₂'' is -C₁₋₆ alkylene-;
R₁₃' is -C₁₄₋₁₈ alkylene-;
q1'' is 1, 2, 3, or 4;
q2'' is 1, 2, or 3;
Y₁ is C or N.

According to an embodiment of the present disclosure, the modification group represented by Formula (XIc) has one of the structures as shown below:

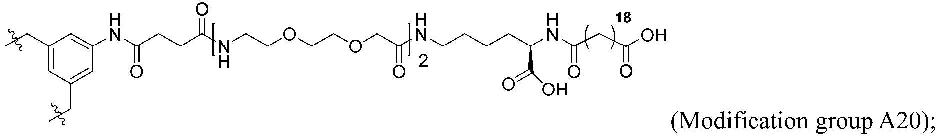

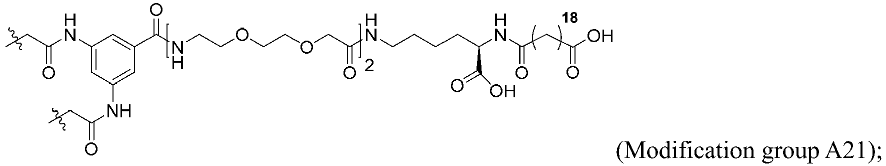

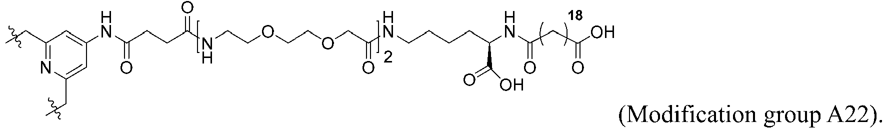

According to an embodiment of the present disclosure, the polypeptide or the derivative thereof represented by Formula (I) has two amino acids Z₁ with the side chain containing -SH, and the modification group has a structure represented by Formula (XI).

According to an embodiment of the present disclosure, the modification group has a structure represented by Formula (XII): 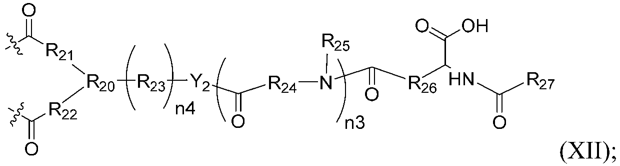
where R₂₀ is C, N, -C₆₋₁₀ arylene, or -C₅₋₁₀ heteroarylene;
R₂₁ and R₂₂ are each independently absent, -C₁₋₆ alkylene- optionally substituted with one or more R_{1b}, or -C₁₋₆ oxyalkylene- optionally substituted with one or more R_{1b}, wherein each R_{1b} is independently halogen, -OH, -SH, -NH₂, -NO₂, -CN, phenyl, -C₁₋₆ alkyl, -C₁₋₆ haloalkyl, or -C₁₋₆ alkoxy;
R₂₃ is absent, -C₁₋₆ alkylene- optionally substituted with one or more R_{2b}, -C(O)-C₁₋₆ alkylene- optionally substituted with one or more R_{2b}, -NH-C(O)-C₁₋₆ alkyleneoptionally substituted with one or more R_{2b}, or -C₁₋₆ alkylene-C(O)-NH-C₁₋₆ alkyleneoptionally substituted with one or more R_{2b}, wherein each R_{2b} is independently halogen, -OH, -SH, -NH₂, -NO₂, -CN, phenyl, -C₁₋₆ alkyl, -C₁₋₆ haloalkyl, or -C₁₋₆ alkoxy;
R₂₄ is -C₁₋₆ alkylene- optionally substituted with one or more R_{3b}, or -(C₁₋₃ alkylene-O)ₘ₂-C₁₋₆ alkylene- optionally substituted with one or more R_{3b}, wherein each R_{3b} is independently halogen, -OH, -SH, -NH₂, -NO₂, -CN, phenyl, -C₁₋₆ alkyl, -C₁₋₆ haloalkyl, or -C₁₋₆ alkoxy;
R₂₅ is H, -C₁₋₆ alkoxy, or -C₁₋₆ alkyl, wherein the -C₁₋₆ alkyl and the -C₁₋₆ alkoxy are each independently optionally substituted with one or more halogen, -OH, -C(O)OH, -C(O)-, -SH, -NH₂, -NO₂, or -CN;
R₂₆ is -C₁₋₆ alkylene- optionally substituted with one or more R_{4b}, wherein each R_{4b} is independently halogen, -OH, -SH, -NH₂, -NO₂, -CN, phenyl, -C₁₋₆ alkyl, -C₁₋₆ haloalkyl, or -C₁₋₆ alkoxy;
R₂₇ is -C₁₀₋₂₀ alkyl optionally substituted with one or more R_{5b}, -C₁₀₋₂₀ alkylene-R₂₈ optionally substituted with one or more R_{5b}, -C₅₋₁₀ alkylene-O-C₆₋₁₀ arylene-R₂₈ optionally substituted with one or more R_{5b}, or -C₅₋₁₀ alkylene-O-C₅₋₁₀ heteroarylene-R₂₈ optionally substituted with one or more R_{5b}, wherein each R_{5b} is independently halogen, -OH, -SH, -NH₂, -NO₂, -CN, phenyl, -C₁₋₆ alkyl, -C₁₋₆ haloalkyl, or -C₁₋₆ alkoxy;
R₂₈ is -COOH, -C₃₋₇ heteroaryl, -S(O)₂OH, or -PO(OH)₂;
m2 is any integer from 1 to 6;
n3 is any integer from 1 to 10;
n3 is 0, 1, or 2;
Y₂ is absent or NH.

According to an embodiment of the present disclosure, R₂₀ is C, N, -C₅₋₇ arylene or -C₅₋₇ heteroarylene.

According to an embodiment of the present disclosure, R₂₀ is C, N, phenylene, or pyridinylene.

According to an embodiment of the present disclosure, R₂₀ is 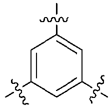 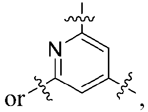 preferably

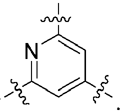

According to an embodiment of the present disclosure, R₂₁ and R₂₂ are each independently absent, -C₁₋₆ alkylene-, or -C₁₋₆ oxyalkylene-.

According to an embodiment of the present disclosure, R₂₁ and R₂₂ are each independently absent or -C₁₋₃ alkylene-.

According to an embodiment of the present disclosure, R₂₃ is absent, -C₁₋₆ alkylene-, -C₁₋₆ alkylene-C(O)-NH-C₁₋₆ alkylene-, or -C(O)-C₁₋₆ alkylene-.

According to an embodiment of the present disclosure, R₂₃ is absent, -C₁₋₃ alkylene-, or -C(O)-C₂₋₄ alkylene-.

According to an embodiment of the present disclosure, R₂₄ is -C₁₋₆ alkylene-, or -(C₁₋₃ alkylene-O)ₘ₂-C₁₋₆ alkylene-.

According to an embodiment of the present disclosure, R₂₄ is -C₁₋₃ alkylene-, or -(C₁₋₃ alkylene-O)ₘ₂-C₁₋₃ alkylene-.

According to an embodiment of the present disclosure, m2 is 2, 3, 4, or 5.

According to an embodiment of the present disclosure, R₂₅ is H, -C₁₋₆ alkoxy or -C₁₋₆ alkyl.

According to an embodiment of the present disclosure, R₂₅ is H or -C₁₋₃ alkyl.

According to an embodiment of the present disclosure, R₂₆ is -C₁₋₆ alkylene-.

According to an embodiment of the present disclosure, R₂₆ is -C₁₋₃ alkylene-.

According to an embodiment of the present disclosure, R₂₇ is -C₁₀₋₂₀ alkyl, -C₁₀₋₂₀ alkylene-R₂₈, -C₅₋₁₀ alkylene-O-C₆₋₁₀ arylene-R₂₈, or -C₅₋₁₀ alkylene-O-C₅₋₁₀ heteroarylene-R₂₈.

According to an embodiment of the present disclosure, R₂₇ is -C₁₀₋₁₈ alkyl, -C₁₄₋₁₈ alkylene-R₂₈, or -C₇₋₉ alkylene-O-C₅₋₇ arylene-COOH.

According to an embodiment of the present disclosure, R₂₈ is -COOH, -C₅₋₆ heteroaryl, -S(O)₂OH, or -PO(OH)₂.

According to an embodiment of the present disclosure, R₂₈ is -COOH, -S(O)₂OH, -PO(OH)₂, or

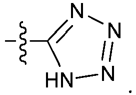

According to an embodiment of the present disclosure, p is 1, 2, or 3.

According to an embodiment of the present disclosure, p is 3, 4, 5, or 6.

According to an embodiment of the present disclosure, the modification group represented by Formula (XII) has a structure represented by Formula (XIIa): 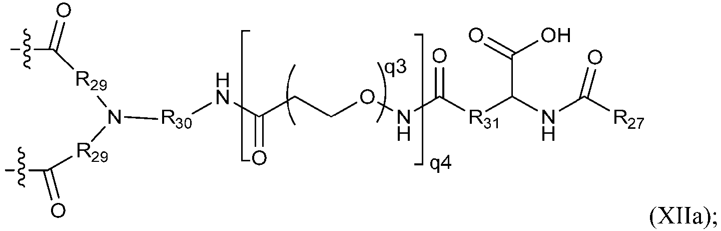
where R₂₇ is -C₁₀₋₂₀ alkyl, -C₁₀₋₂₀ alkylene-COOH, -C₁₀₋₂₀ alkylene-S(O)₂OH, -C₁₀₋₂₀ alkylene-PO(OH)₂, -C₁₀₋₂₀ alkylene-C₅₋₆ heteroaryl, or -C₅₋₁₀ alkylene-O-C₆₋₁₀ arylene-COOH;
each R₂₉ is independently -C₁₋₃ alkylene-;
R₃₀ is -C(O)-C₁₋₆ alkylene-, or -C₁₋₃ alkylene-C(O)-NH-C₁₋₃ alkylene-;
R₃₁ is -C₁₋₆ alkylene-;
q3 is 1, 2, 3, or 4;
q4 is 1, 2, or 3.

According to an embodiment of the present disclosure, in the structure represented by Formula (XIIa), R₂₇ is -C₁₂₋₁₉ alkyl, -C₁₂₋₁₉ alkylene-COOH, or -C₅₋₁₀ alkylene-O-C₆₋₈ arylene-COOH;
each R₂₉ is independently -C₁₋₃ alkylene-;
R₃₀ is -C(O)-C₁₋₆ alkylene-, or -C₁₋₃ alkylene-C(O)-NH-C₁₋₃ alkylene-;
R₃₁ is -C₁₋₆ alkylene-;
q3 is 1, 2, 3, or 4;
q4 is 1, 2, or 3.

According to an embodiment of the present disclosure, the modification group represented by Formula (XIIa) has one of the structures as shown below:

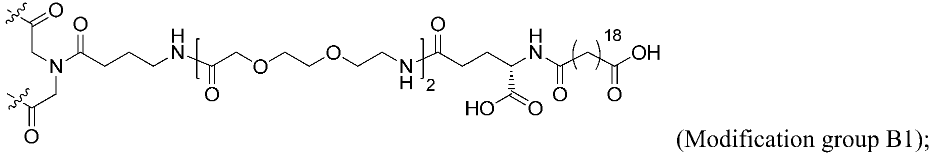

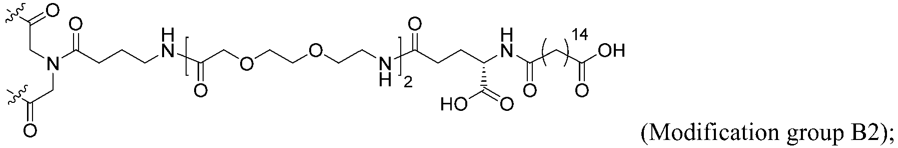

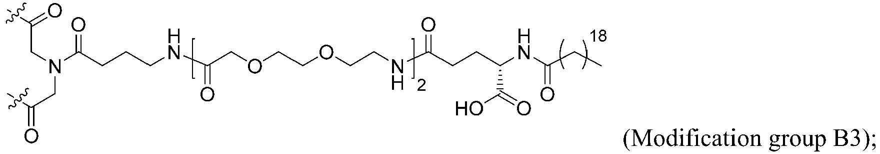

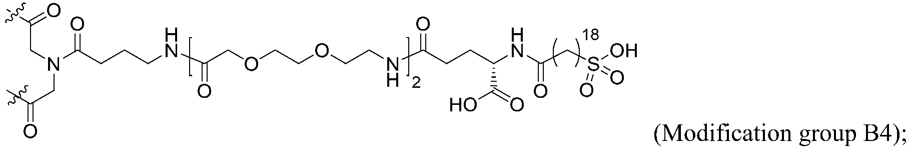

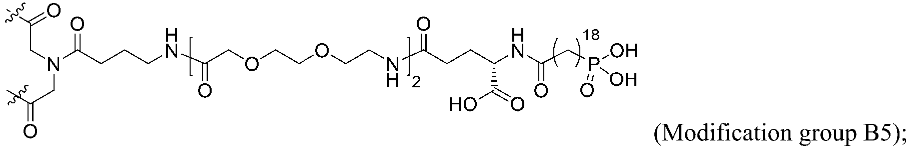

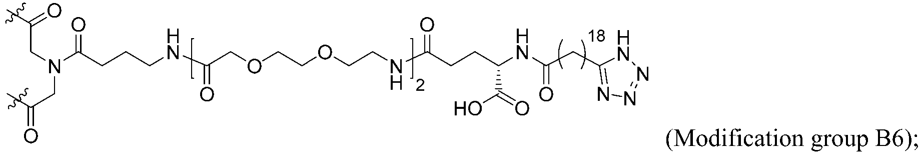

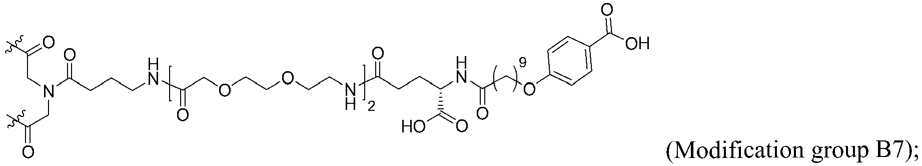

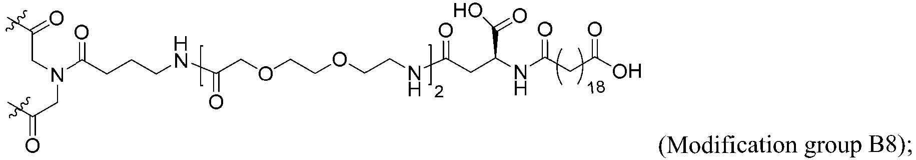

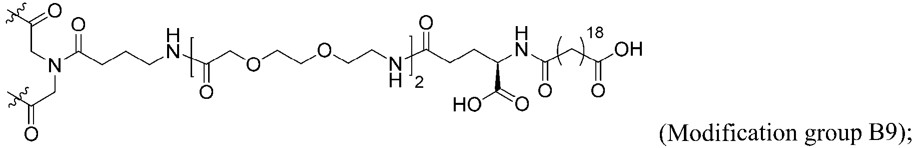

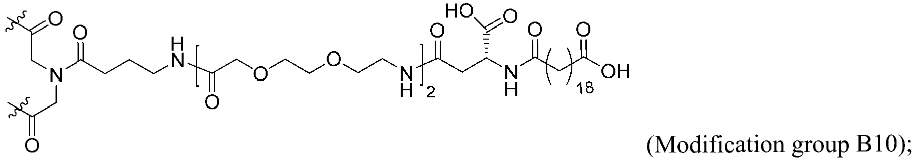

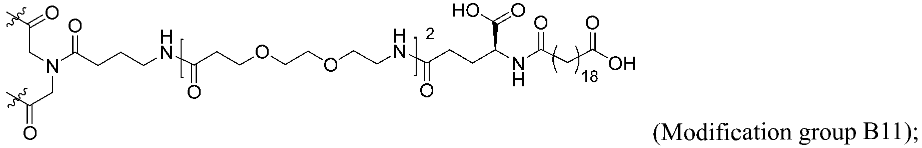

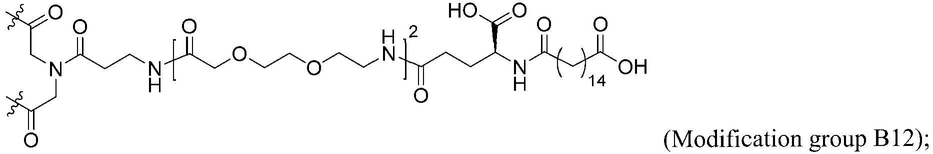

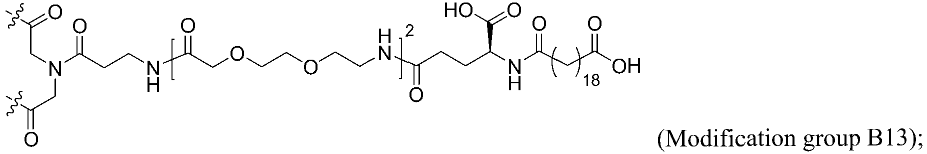

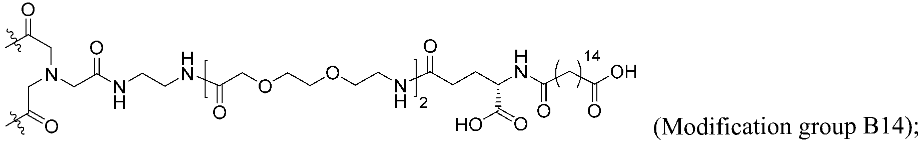

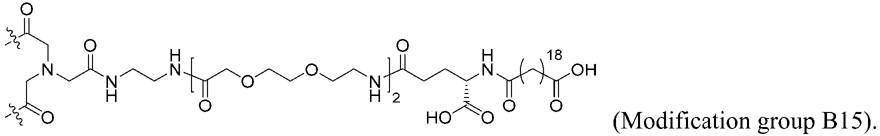

According to an embodiment of the present disclosure, the modification group represented by Formula (XII) has a structure represented by Formula (XIIb): 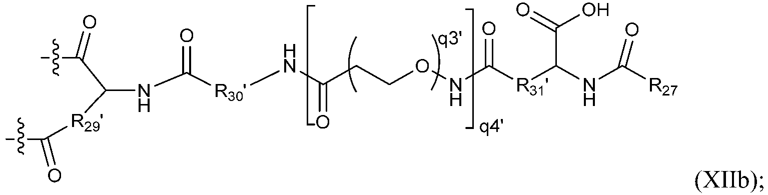
where R₂₇ is -C₁₀₋₂₀ alkyl, -C₁₀₋₂₀ alkylene-COOH, -C₁₀₋₂₀ alkylene-S(O)₂OH, -C₁₀₋₂₀ alkylene-PO(OH)₂, -C₁₀₋₂₀ alkylene-C₅₋₆ heteroaryl, or -C₅₋₁₀ alkylene-O-C₆₋₁₀ arylene-COOH;
each R₂₉' is independently -C₁₋₃ alkylene-;
R₃₀' is -C₁₋₆ alkylene-;
R₃₁' is -C₁₋₆ alkylene-;
q3' is 1, 2, 3, or 4, preferably 2;
q4' is 1, 2, or 3, preferably 2.

According to an embodiment of the present disclosure, in the structure represented by Formula (XIIb), R₂₇ is -C₁₂₋₁₉ alkyl, or -C₁₂₋₁₉ alkylene-COOH;
each R₂₉' is independently -C₁₋₃ alkylene-;
R₃₀' is -C₁₋₆ alkylene-;
R₃₁' is -C₁₋₆ alkylene-;
q3' is 2;
q4' is 2.

According to an embodiment of the present disclosure, R₂₇ is -C₁₀₋₂₀ alkylene-COOH or -C₁₀₋₂₀ alkyl.

According to an embodiment of the present disclosure, R₂₇ is -C₁₄₋₁₈ alkylene-COOH.

According to an embodiment of the present disclosure, the modification group represented by Formula (XIIb) has one of the structures as shown below:

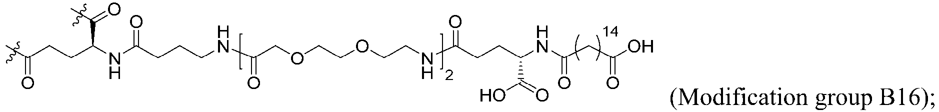

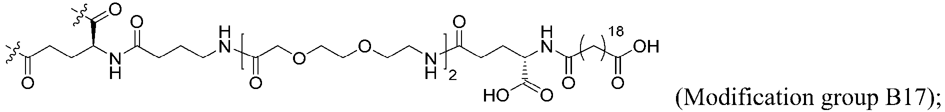

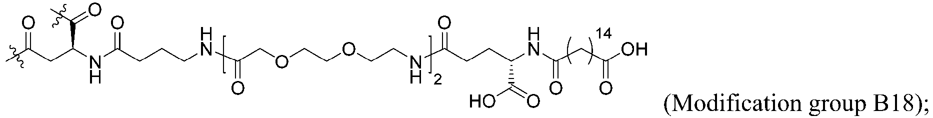

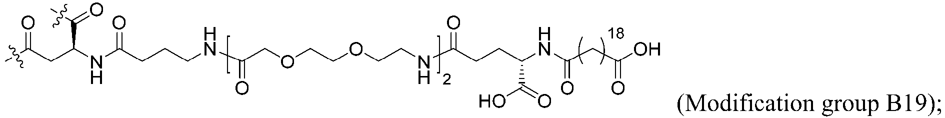

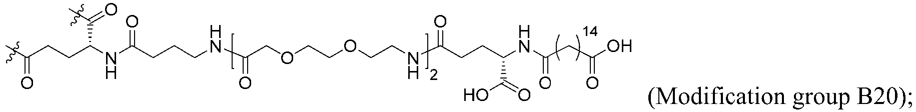

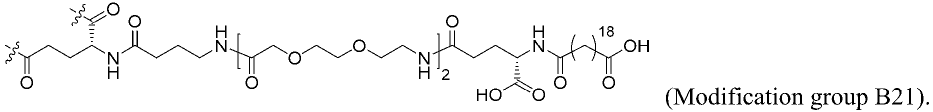

According to an embodiment of the present disclosure, the modification group represented by Formula (XII) has a structure represented by Formula (XIIc): 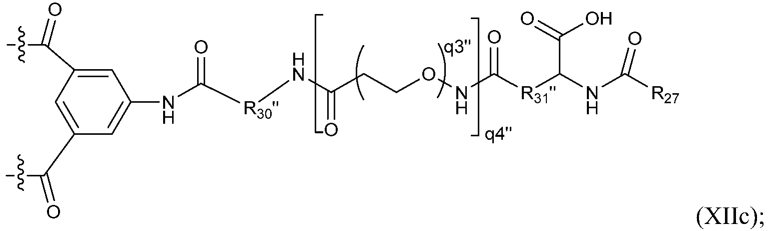
where R₂₇ is -C₁₀₋₂₀ alkyl, -C₁₀₋₂₀ alkylene-COOH, -C₁₀₋₂₀ alkylene-S(O)₂OH, -C₁₀₋₂₀ alkylene-PO(OH)₂, -C₁₀₋₂₀ alkylene-C₅₋₆ heteroaryl, or -C₅₋₁₀ alkylene-O-C₆₋₁₀ arylene-COOH;
R₃₀" is -C₁₋₆ alkylene-;
R₃₁" is -C₁₋₆ alkylene-;
q3 is 1, 2, 3, or 4;
q4 is 1, 2, or 3.

According to an embodiment of the present disclosure, in the structure represented by Formula (XIIc), R₂₇ is -C₁₀₋₂₀ alkylene-COOH or -C₁₀₋₂₀ alkyl;
R₃₀" is -C₁₋₆ alkylene-;
R₃₁" is -C₁₋₆ alkylene-;
q3 is 1, 2, 3, or 4;
q4 is 1, 2, or 3.

According to an embodiment of the present disclosure, in the structure represented by Formula (XIIc), R₂₇ is -C₁₄₋₁₈ alkylene-COOH or -C₁₀₋₁₉ alkyl;
R₃₀" is -C₁₋₆ alkylene-;
R₃₁" is -C₁₋₆ alkylene-;
q3 is 1, 2, 3, or 4;
q4 is 1, 2, or 3.

According to an embodiment of the present disclosure, the modification group represented by Formula (XIIc) has one of the structures as shown below:

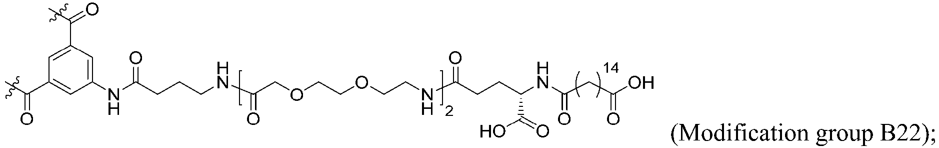

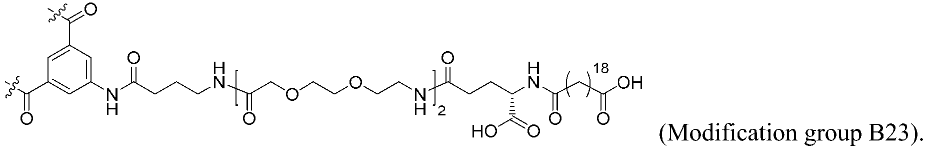

According to an embodiment of the present disclosure, the polypeptide or the derivative thereof represented by Formula (I) includes two amino acids Z₂ with the side chain containing -NH₂, and the modification group has a structure represented by Formula (XIIc).

In some alternative embodiments of the present disclosure, the polypeptide or the derivative thereof represented by Formula (I) has at least one of the following structures in Table A:

**Table A**

| Name of Polype ptide | Amino acid sequence of a polypeptide | SEQ ID NO: | Sites where modifi cation groups are bound | Modifi cation group |
|---|---|---|---|---|
| TG1 | Y-Aib-QGTFTSDYSKYCDKRAAQCFVQWLLAGGPSSGAPPPS | 1 | 14,21 | A1 |
| TG2 | Y-Aib-QGTFTSDYSKYCDEKRAKCFVQWLLDHHPSSGQPPPS | 2 | 14,21 | A1 |
| TG3 | Y-Aib-QGTFTSDCSKYCDERAAQDFVQWLLAGGPSSGAPPPS | 3 | 10,14 | A1 |
| TG4 | Y-Aib-QGTFTSDCSKYLDECAAQDFVQWLLAGGPSSGAPPPS | 4 | 10,17 | A1 |
| TG5 | Y-Aib-QGTFTSDCSKYLDERAAQCFVQWLLAGGPSSGAPPPS | 5 | 10,21 | A1 |
| TG6 | H-Aib-HGTFTSDYSIYCEKKYAQCFVQWLLAGGPSSGAPPPS | 6 | 14,21 | A1 |
| TG7 | H-Aib-HGTFTSDYSIYCEKRYAQCFVQWLLAGGPSSGAPPPS | 7 | 14,21 | A1 |
| TG8 | H-Aib-HGTFTSDYSIYCEKRYA-Aib-CFVQWLLEGGPSSGAPPP S | 8 | 14,21 | A1 |
| TG9 | Y-Aib-QGTFTSDYSKYLDKCAAQDFVCWLLAGGPSSGAPPPS | 9 | 17,24 | A1 |
| TG10 | H-Aib-HGTFTSDYSKYLDKCYAQDFVCWLLEGGPSSGAPPPS | 10 | 17,24 | A1 |
| TG11 | Y-Aib-QGTFTSDYSILLDKCAQHAFICYLLEGGPSSGAPPPS | 11 | 17,24 | A1 |
| TG12 | Y-Aib-QGTFTSDYSILLDKCAQ-Aib-AFICYLLEGGPSSGAPPPS | 12 | 17,24 | A1 |
| TG13 | H-Aib-HGTFTSDYSRALEKCAARLFICWLLEGGPSSGAPPPS | 13 | 17,24 | A1 |
| TG14 | H-Aib-HGTFTSDYSKYLE-K(Ac)-KYA-Aib-CFVQWLLCGGPSS GAPPPS | 14 | 21,28 | A1 |
| TG15 | Y-Aib-QGTFTSDYSI-αMeL-LDK-K(Ac)-AQ-Aib-CFIEYLLCGGP SSGAPPPS | 15 | 21,28 | A1 |
| TG16 | H-Aib-HGTFTSDLSKL-K(Ac)-EEQRQ-Aib-CFIEWLKCGGPPS- Aib-KPPPK | 16 | 21,28 | A1 |
| TG17 | H-Aib-QGTFTSDLSKQ-K(Ac)-DEQRAKCFIEWLICGGPSSGAPP PS | 17 | 21,28 | A1 |
| TG18 | H-Aib-QGTFTSDYSI-αMeL-LDK-K(Ac)-AQ-Aib-CFIEYLLCGGP SSGAPPPS | 18 | 21,28 | A1 |
| TG19 | (3-iodo-Y)-Aib-QGTFTSDYSI-αMeL-LDK-K(Ac)-AQ-Aib-CFIEY LLCGGPSSGAPPPS | 19 | 21,28 | A1 |
| TG20 | (4-Pal)-Aib-QGTFTSDYSI-αMeL-LDK-K(Ac)-AQ-Aib-CFIEYLL CGGPSSGAPPPS | 20 | 21,28 | A1 |
| TG21 | (4-amino-F)-Aib-QGTFTSDYSI-αMeL-LDK-K(Ac)-AQ-Aib-CFIE YLLCGGPSSGAPPPS | 21 | 21,28 | A1 |
| TG22 | Y-s-QGTFTSDYSI-αMeL-LDK-K(Ac)-AQ-Aib-CFIEYLLCGGPSS GAPPPS | 22 | 21,28 | A1 |
| TG23 | Y-Aib-QGT-αMeF-TSDYSI-αMeL-LDK-K(Ac)-AQ-Aib-CFIEYLL CGGPSSGAPPPS | 23 | 21,28 | A1 |
| TG24 | Y-Aib-QGTFT-HoS-DYSI-αMeL-LDK-K(Ac)-AQ-Aib-CFIEYLLC GGPSSGAPPPS | 24 | 21,28 | A1 |
| TG25 | Y-Aib-QGTFTSDYSI-αMeL-HoL-DK-K(Ac)-AQ-Aib-CFIEYLLC GGPSSGAPPPS | 25 | 21,28 | A1 |
| TG26 | Y-Aib-QGTFTSDYSI-αMeL-Npg-DK-K(Ac)-AQ-Aib-CFIEYLLC GGPSSGAPPPS | 26 | 21,28 | A1 |
| TG27 | Y-Aib-QGTFTSDYSI-αMeL-Tle-DK-K(Ac)-AQ-Aib-CFIEYLLCG GPSSGAPPPS | 27 | 21,28 | A1 |
| TG28 | Y-Aib-QGTFTSDYSI-αMeL-LDK-K(Ac)-AQ-Aib-CFIEWLLCGG PSSGAPPPS | 28 | 21,28 | A1 |
| TG29 | Y-Aib-QGTFTSDYSILLDK-K(Ac)-AQ-Aib-CFIEWLLCGGPSSG APPPS | 29 | 21,28 | A1 |
| TG30 | Y-Aib-QGTFTSDYSI-αMeL-LDKQAQ-Aib-CFIEYLLCGGPSSGA PPPS | 30 | 21,28 | A1 |
| TG31 | Y-Aib-QGTFTSDYSI-αMeL-LDK-K(Ac)-AQ-a-CFIEYLLCGGPSS GAPPPS | 31 | 21,28 | A1 |
| TG32 | Y-Aib-QGTFTSDYSI-αMeL-LDK-K(Ac)-AQHCFIEYLLCGGPSS GAPPPS | 32 | 21,28 | A1 |
| TG33 | Y-Aib-QGTFTSDYSI-αMeL-LDK-K(Ac)-AQQCFIEYLLCGGPSS GAPPPS | 33 | 21,28 | A1 |
| TG34 | Y-Aib-QGTFTSDYSI-αMeL-LDK-K(Ac)-A-Aib-QCFIEYLLCGGP SSGAPPPS | 34 | 21,28 | A1 |
| TG35 | Y-Aib-QGTFTSDYSI-αMeL-LDKRAQ-Aib-CFIEYLLCGGPSSGA PPPS | 35 | 21,28 | A1 |
| TG36 | Y-Aib-QGTFTSDYSI-αMeL-LEK-K(Ac)-AQ-Aib-CFIEYLLCGGP SSGAPPPS | 36 | 21,28 | A1 |
| TG37 | Y-Aib-QGTFTSDYSI-αMeL-L-Aad-K-K(Ac)-AQ-Aib-CFIEYLLC GGPSSGAPPPS | 37 | 21,28 | A1 |
| TG38 | Y-Aib-QGTFTSD-4-Pal-SI-αMeL-LDK-K(Ac)-AQ-Aib-CFIEYLLC GGPSSGAPPPS | 38 | 21,28 | A1 |
| TG39 | Y-Aib-QGTFT-αMeS-DYSI-αMeL-LDK-K(Ac)-AQ-Aib-CFIEYLL CGGPSSGAPPPS | 39 | 21,28 | A1 |
| TG40 | Y-Aib-QGT-F(2-F)-TSDYSI-αMeL-LDK-K(Ac)-AQ-Aib-CFIEYLL CGGPSSGAPPPS | 40 | 21,28 | A1 |
| TG41 | Y-Aib-Dab(Ac)-GTFTSDYSI-αMeL-LDK-K(Ac)-AQ-Aib-CFIEYL LCGGPSSGAPPPS | 41 | 21,28 | A1 |
| TG42 | Y-Aib-HGTFTSDYSI-αMeL-LDK-K(Ac)-AQ-Aib-CFIEYLLCGGP SSGAPPPS | 42 | 21,28 | A1 |
| TG43 | Y-Ac4c-QGTFTSDYSI-αMeL-LDK-K(Ac)-AQ-Aib-CFIEYLLCGG PSSGAPPPS | 43 | 21,28 | A1 |
| TG44 | Y-Aib-QGTFTSDYSILLDKCAQH-Aib-FIEYLLCGGPSSGAPPPS | 44 | 17,28 | A1 |
| TG45 | Y-Aib-QGTFTSDYSILLDK-HoC-AQH-Aib-FIEYLL-HoC-GGPSS GAPPPS | 45 | 17,28 | A1 |
| TG46 | Y-Aib-QGT-F(2-F)-TSDYSILLDKCAQQAFICYLLAGGPSSGAPP PS | 46 | 17,24 | A1 |
| TG47 | Y-Aib-QGT-F(2-F)-TSDYSILLDKQAQQCFIEYLLCGGPSSGAPP PS | 47 | 21,28 | A1 |
| TG48 | Y-Aib-QGT-F(2-F)-TSDYSI-Aib-LDK-K(Ac)-AQQCFIEYLLCGG PSSGAPPPS | 48 | 21,28 | A1 |
| TG49 | Y-Aib-QGT-F(2-F)-TSDYSILCDKQAQQCFIEYLLAGGPSSGAPP PS | 49 | 14,21 | A1 |
| TG50 | Y-Aib-QGT-F(2-F)-TSDYSILLD-αMeK-K(Ac)-AQQCFIEYLLCG GPSSGAPPPS | 50 | 21,28 | A1 |
| TG51 | Y-Aib-QGT-F(2-F)-TSDYSILLD-αMeK-K(Ac)-AQ-Aib-CFIEYLIC GGPSSGAPPPS | 51 | 21,28 | A1 |
| TG52 | Y-Aib-QGT-F(2-F)-TSDYSI-Iva-LDK-K(Ac)-AQ-Aib-CFIEYLLC GGPSSGAPPPS | 52 | 21,28 | A1 |
| TG53 | Y-Aib-QGT-F(2-F)-TSDYSI-Iva-LDK-K(Ac)-AQ-Aib-CFIEYLLC GGPSSGAPPPS | 53 | 21,28 | A5 |
| TG54 | Y-Aib-QGT-F(2-F)-TSDYSI-Iva-LDK-K(Ac)-AQ-Aib-CFIEYLLC GGPSSGAPPPS | 54 | 21,28 | A8 |
| TG55 | Y-Aib-QGT-F(2-F)-TSDYSI-Iva-LDK-K(Ac)-AQ-Aib-CFIEYLLC GGPSSGAPPPS | 55 | 21,28 | A11 |
| TG56 | Y-Aib-QGT-F(2-F)-TSDY-αMeS-ILLD-αMeK-K(Ac)-AQQCFIEYL LCGGPSSGAPPPS | 56 | 21,28 | A1 |
| TG57 | Y-Aib-QGT-F(2-F)-TSDYSI-αMeL-LD-αMeK-K(Ac)-AQQCFIEYL LCGGPSSGAPPPS | 57 | 21,28 | A1 |
| TG58 | Y-Aib-QGT-F(2-F)-TSDYSI-αMeL-LD-αMeK-K(Ac)-AQQC-HoF-I EYLLCGGPSSGAPPPS | 58 | 21,28 | A1 |
| TG59 | Y-Aib-QGT-F(2-F)-TSDYSI-αMeL-LDKCAQCAFIEYLLAGGPSS GAPPPS | 59 | 17,20 | A1 |
| TG60 | Y-Aib-QGT-F(2-F)-TSDYSI-Iva-LDKCAQCAFIEYLLAGGPSSGA PPPS | 60 | 17,20 | A1 |
| TG61 | Y-Aib-QGT-F(2-F)-TSDY-αMeS-I-αMeL-LD-αMeK-K(Ac)-AQ-Aib -CFIEYLLCGGPSSGAPPPS | 61 | 21,28 | A1 |
| TG62 | Y-Aib-QGT-F(2-F)-TSDY-αMeS-I-αMeL-LD-αMeK-CAQCAFIEY LLAGGPSSGAPPPS | 62 | 17,20 | A1 |
| TG63 | H-Aib-HGT-F(2-F)-TSDYSIYLE-K(Ac)-KYA-Aib-CFVQWLLCG GPSSGAPPPS | 63 | 21,28 | A1 |
| TG64 | H-Aib-HGT-F(2-F)-TSDYSIQ-K(Ac)-EEIAQ-Aib-CFIEWLLCGGP SSGAPPPS | 64 | 21,28 | A1 |
| TG65 | H-Aib-HGT-F(2-F)-TSDYSIY-K(Ac)-EERAQ-Aib-CFIEWLLCGG PSSGAPPPS | 65 | 21,28 | A1 |
| TG66 | H-Aib-HGT-F(2-F)-TSDYSI-Iva-LDK-K(Ac)-AQ-Aib-CFIEYLLC GGPSSGAPPPS | 66 | 21,28 | A1 |
| TG67 | Y-Aib-QGT-F(2-F)-TSDYSI-αMeL-LDKCAHCAFIEYLLAGGPSS GAPPPS | 67 | 17,20 | A1 |
| TG68 | Y-Aib-QGT-F(2-F)-TSDYSI-αMeL-LDKCA-K(Ac)-CAFIEYLLAG GPSSGAPPPS | 68 | 17,20 | A1 |
| TG69 | Y-Aib-QGT-F(2-F)-TSDYSI-αMeL-LDKCAQCAFIEYLLAGGPSS GAPPPS | 69 | 17,20 | A1 |
| TG70 | H-Aib-HGT-F(2-F)-TSDYSI-αMeL-LDKCAQCAFIEYLLAGGPSS GAPPPS | 70 | 17,20 | A1 |
| TG71 | Y-s-QGT-F(2-F)-TSDYSI-αMeL-LDKCAQCAFIEYLLAGGPSSGA PPPS | 71 | 17,20 | A1 |
| TG72 | Y-Aib-QGTFTSDYSILLDKQAQQCFIEYLLCGGPSSGAPPPS | 72 | 21,28 | A1 |
| TG73 | Y-Ac4c-QGT-F(2-F)-TSDYSILLDKCAQCAFIEYLLEGGPSSGAP PPS | 73 | 17,20 | A1 |
| TG74 | Y-Ac4c-QGT-F(2-F)-TSDYSILLDK-K(Ac)-AQ-Aib-CFIEYLLCG GPSSGAPPPS | 74 | 21,28 | A1 |
| TG75 | H-Ac4c-HGT-F(2-F)-TSDYSIQLEEIAQ-Aib-CFIEYLLCGGPSSG APPPS | 75 | 21,28 | A1 |
| TG76 | Y-Aib-QGTFTSDYSILLDK-K(Ac)-AQ-Aib-c-FIEYLL-c-GGPSSG APPPS | 76 | 21,28 | A1 |
| TG77 | Y-Aib-QGTFTSDYSILLDK-K(Ac)-AQ-Aib-Hoc-FIEYLL-Hoc-GG PSSGAPPPS | 77 | 21,28 | A1 |
| TG78 | Y-Aib-QGTFTSDYSILLDK-K(Ac)-AQ-Aib-αMeC-FIEYLL-αMeC -GGPSSGAPPPS | 78 | 21,28 | A1 |
| TG79 | Y-Aib-QGT-F(2-F)-TSDYSIQLDK-K(Ac)-AQ-Aib-CFIEYLLCGG PSSGAPPPS | 79 | 21,28 | A1 |
| TG80 | Y-Aib-QGT-F(2-F)-TSDYSI-αMeL-LDK-K(Ac)-AQ-Aib-CFIEYLL CGGPSSGAPPPS | 80 | 21,28 | A11 |
| TG81 | Y-Aib-QGT-F(2-F)-TSDYSI-αMeL-LDK-K(Ac)-AQ-Aib-CFIEYLL CGGPSSGAPPPS | 81 | 21,28 | A18 |
| TG82 | Y-Aib-QGT-F(2-F)-TSDYSI-αMeL-LDK-K(Ac)-AQ-Aib-CFIEYLL CGGPSSGAPPPS | 82 | 21,28 | A20 |
| TG83 | Y-Aib-QGT-F(2-F)-TSDYSI-αMeL-LDK-K(Ac)-AQ-Aib-CFIEYLL CGGPSSGAPPPS | 83 | 21,28 | A22 |
| TG84 | Y-Aib-QGT-F(2-F)-TSDYSI-αMeL-LD-αMeK-K(Ac)-AQ-Aib-KFI EYLLKGGPSSGAPPPS | 84 | 21,28 | B1 |
| TG85 | Y-Aib-QGT-F(2-F)-TSDYSI-αMeL-LDK-K(Ac)-AQ-Aib-KFIEYLL KGGPSSGAPPPS | 85 | 21,28 | B1 |
| TG86 | Y-Aib-QGTFTSDYSILLDK-K(Ac)-AQ-Aib-Orn-FIEYLL-Orn-GG PSSGAPPPS | 86 | 21,28 | B1 |
| TG87 | Y-Aib-QGT-F(2-F)-TSDYSI-αMeL-LDKKAQKAFIEYLLAGGPSS GAPPPS | 87 | 17,20 | B1 |
| TG88 | Y-Aib-QGT-F(2-F)-TSDYSIQLDKKAQKAFIEYLLAGGPSSGAPP PS | 88 | 17,20 | B1 |
| TG89 | Y-Aib-QGTFTSDYSILLDKKAQKAFIEYLLAGGPSSGAPPPS | 89 | 17,20 | B1 |
| TG90 | Y-Aib-QGT-F(2-F)-TSDYSI-αMeL-LDKKAQKAFIEYLLAGGPSS GAPPPS | 90 | 17,20 | B1 |
| TG91 | Y-Aib-QGT-F(2-F)-TSDYSI-αMeL-LDKKAQKAFIEYLLAGGPSS GAPPPS | 91 | 17,20 | B7 |
| TG92 | Y-Aib-QGT-F(2-F)-TSDYSI-αMeL-LDKKAQKAFIEYLLAGGPSS GAPPPS | 92 | 17,20 | B16 |
| TG93 | Y-Aib-QGT-F(2-F)-TSDYSI-αMeL-LDKKAQKAFIEYLLAGGPSS GAPPPS | 93 | 17,20 | B20 |
| TG94 | Y-Aib-QGT-F(2-F)-TSDYSI-αMeL-LDKKAQKAFIEYLLAGGPSS GAPPPS | 94 | 17,20 | B22 |

| | | | | |
|---|---|---|---|---|
| Note that the "Sites where modification groups are bound" in Table A is specifically the site of i in Xᵢ as described in the present disclosure. | | | | |

### Pharmaceutical compositions, use, and method

In a second aspect of the present disclosure, a pharmaceutical composition is provided. According to an embodiment of the present disclosure, the pharmaceutical composition includes the polypeptide or the derivative thereof or the pharmaceutically acceptable salt thereof according to the first aspect. It can be seen from the foregoing that the polypeptide or the derivative thereof or the pharmaceutically acceptable salt thereof of the present disclosure exhibits strong agonistic activity on three receptor targets of GLP-1R, GCGR, and GIPR, and is effective in controlling blood sugar and reducing body weight. Thus, the use of a drug containing the polypeptide or the derivative thereof or the pharmaceutically acceptable salt thereof is effective in preventing or treating metabolic disorder related diseases, bone-related diseases, cardiovascular diseases, neurodegenerative diseases, and the like.

According to an embodiment of the present disclosure, it further includes a pharmaceutically acceptable excipient.

In a third aspect of the present disclosure, provided is use of the polypeptide or the derivative thereof or the pharmaceutically acceptable salt thereof according to the first aspect, or the pharmaceutical composition according to the second aspect in the manufacture of a medicament for treating or preventing at least one of a metabolic disorder related disease, a bone-related disease, a cardiovascular disease, or a neurodegenerative disease.

According to an embodiment of the present application, provided is use of the polypeptide or the derivative thereof or the pharmaceutically acceptable salt thereof according to the first aspect, or a pharmaceutical composition according to the second aspect in the treatment or prevention of at least one of a metabolic disorder related disease, a bone-related disease, a cardiovascular disease, or a neurodegenerative disease.

According to an embodiment of the present application, provided is the polypeptide or the derivative thereof or the pharmaceutically acceptable salt thereof according to the first aspect, or a pharmaceutical composition according to the second aspect for use in treating or preventing at least one of a metabolic disorder related disease, a bone-related disease, a cardiovascular disease, or a neurodegenerative disease.

According to an embodiment of the present disclosure, the metabolic disorder related disease includes at least one of obesity, diabetes, dyslipidemia-related disease, fatty liver disease, metabolic syndrome, metabolic liver disease, non-alcoholic steatohepatitis, or non-alcoholic fatty liver disease.

According to an embodiment of the present disclosure, the neurodegenerative disease includes at least one of Alzheimer's disease or Parkinson's disease.

In a fourth aspect, the present disclosure provides a method for preventing and/or treating a metabolic disorder related disease, a bone-related disease, a cardiovascular disease, and/or a neurodegenerative disease. According to an embodiment of the present disclosure, the method includes: administering to a subject a pharmaceutically acceptable amount of the polypeptide or the derivative thereof or the pharmaceutically acceptable salt thereof according to the first aspect, or the pharmaceutical composition according to the second aspect. The method of the present disclosure is effective in preventing and/or treating metabolic disorder related diseases, bone-related diseases, cardiovascular diseases, and/or neurodegenerative diseases.

According to an embodiment of the present disclosure, the metabolic disorder related disease includes at least one of obesity, diabetes, dyslipidemia-related disease, fatty liver disease, metabolic syndrome, metabolic liver disease, non-alcoholic steatohepatitis, or non-alcoholic fatty liver disease.

According to an embodiment of the present disclosure, the neurodegenerative disease includes at least one of Alzheimer's disease or Parkinson's disease.

The effective amount of the polypeptide or derivative thereof, or pharmaceutical composition of the present disclosure, may vary depending on, among other things, the mode of administration and the severity of the disease to be treated. Selection of a preferred effective amount can be determined by one of ordinary skill in the art based on a variety of factors (e.g., through clinical trials). Such factors include, but are not limited to: pharmacokinetic parameters of the active ingredient, such as bioavailability, metabolism, half-life, and the like; the severity of the disease to be treated in the patient, the weight of the patient, the immune status of the patient, the route of administration, and the like. For example, several divided doses may be administered daily, or the dose may be proportionally reduced as required by the therapeutic situation.

The polypeptide or the derivative thereof, or the pharmaceutical composition of the present disclosure, may be incorporated into a drug suitable for parenteral administration (e.g., intravenous, subcutaneous, intraperitoneal, intramuscular). These drugs can be prepared in various forms, such as liquid, semi-solid, and solid dose forms, including but not limited to liquid solutions (e.g., injection solutions and infusion solutions) or lyophilized powders. These drugs are typically in the form of an injection solution or an infusion solution. The aforementioned polypeptide or the derivative thereof, or the pharmaceutical composition, may be administered by intravenous infusion or injection, or by intramuscular or subcutaneous injection.

The embodiments of the present disclosure will be described with reference to the following examples. Those skilled in the art will understand that the following embodiment is for illustration only and should not be construed as limiting the scope of the present disclosure. Where specific techniques or conditions are not specified in the embodiments, they should be performed according to the techniques or conditions described in the literature of the art or according to the product specification. The reagents or instruments used, which are not specified by the manufacturer, are conventional products available commercially.

### Example 1: Preparation method for polypeptides or derivatives thereof

1. The linear peptide synthesis of Polypeptide 1 to Polypeptide 83 (referred to as TG01 to TG83) in Table A was performed using the classical Fmoc-tBu solid-phase synthesis method with a Symphony^{®} X peptide synthesizer under the following reaction conditions:
(1) Resin swelling: The Rink Amide MBHA resin was added to DCM. The reaction was sparged with N₂ at room temperature for 1 h and filtered by suction. The resin was then washed 2-3 times with DMF.
(2) Fmoc protecting group removal: A solution of 20% piperidine in DMF was added to the resin. The reaction was sparged with N₂ at room temperature for 10 min, filtered by suction, and the operation was repeated until complete deprotection was achieved. The resin was then washed 2-3 times with DMF.
(3) Amino acid coupling: Reagents were added according to the reaction ratio of resin: amino acids: DIC: Oxyma Pure = 1:5:5:5. The reactants, DIC, and Oxyma Pure were dissolved in DMF in advance. The reaction was sparged with N₂ at room temperature for 10 min, then transferred to a resin reaction vessel and sparged with N₂ for 1-3 h at room temperature. After the reaction, the mixture was filtered by suction, and the resin was washed 2-3 times with DMF. After the synthesis was completed, the resin was washed 2-3 times with DCM and dried under vacuum to obtain the peptide resin.
   The above methods are applicable to the amino acids (D or L forms) or synthesis reagents, including but not limited to: Fmoc-AEEA-OH, Fmoc-Aad(tBu)-OH, Fmoc-Ac4c-OH, Fmoc-Aib-OH, Fmoc-Ala-OH, Fmoc-Arg(Pbf)-OH, Fmoc-Asp(tBu)-OH, Fmoc-Cys(Trt)-OH, Fmoc-Dab(Ac)-OH, Fmoc-Dap(Ac)-OH, Fmoc-Gly-OH, Fmoc-Glu(tBu)-OH, Fmoc-Gln(Trt)-OH, Fmoc-His(Trt)-OH, Fmoc-Ile-OH, Fmoc-Iva-OH, Fmoc-Leu-OH, Fmoc-Lys(Ac)-OH, Fmoc-Lys(Boc)-OH, Fmoc-Lys(Mtt)-OH, Fmoc-Lys(Alloc)-OH, Fmoc-Lys(ivDde)-OH, Fmoc-Met-OH, Fmoc-Nal-OH, Fmoc-Nle-OH, Fmoc-Orn(Boc)-OH, Fmoc-Pal-OH, Fmoc-Pro-OH, Fmoc-Phe-OH, Fmoc-Phe(2-F)-OH, Fmoc-Phe(3-F)-OH, Fmoc-Phe(4-F)-OH, Fmoc-Ser(tBu)-OH, Fmoc-Thr(tBu)-OH, Fmoc-Tle-OH, Fmoc-Trp(Boc)-OH, Fmoc-Tyr(tBu)-OH, Fmoc-Val-OH, Fmoc-α-methyl-Leu-OH, Fmoc-α-methyl-Lys(Boc)-OH, Fmoc-α-methyl-Phe-OH, Fmoc-α-methyl-Phe(2-F)-OH, Fmoc-AEEA-OH, Fmoc-Glu-OtBu, Fmoc-Asp-OtBu, Fmoc-Ida(Allyl)-OH, Boc-L-His(Trt)-OH, Boc-L-Tyr(tBu)-OH, myristic acid, myristic acid, stearic acid, arachidic acid, docosanoic acid, 18-(tert-butoxy)-18-oxooctadecanoic acid, 20-(tert-Butoxy)-20-oxoicosanoic acid, and the like.

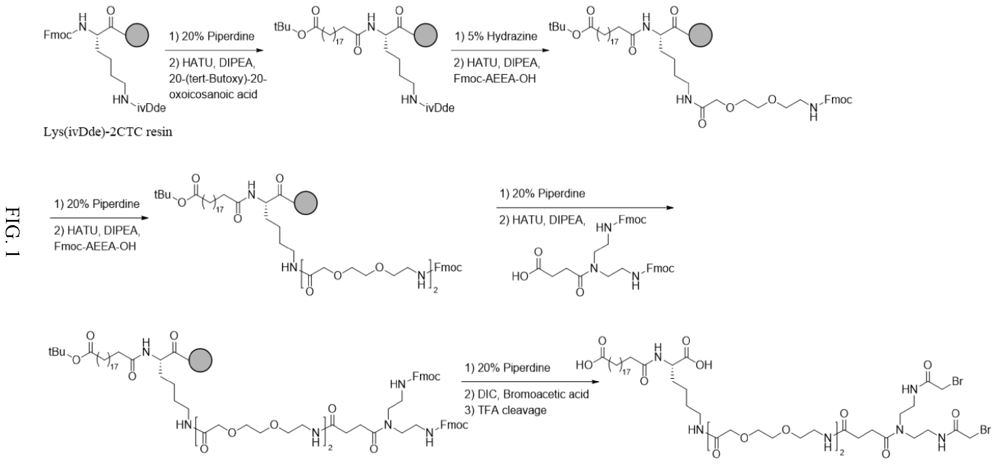

2. Linear peptide cleavage and drying: The polypeptide cleavage solution was prepared at a volume ratio of TFA: TIPS: H₂O: EDT = 95:2:2:1. The cleavage solution was added to the dried peptide resin at 10 mL/g resin, and then placed on a shaker for thorough shaking for 3 h. The resin residue was filtered out. Ten volumes of cold MTBE were added to the filtrate. The resulting suspension was cooled at -20°C for 1 h, then centrifuged at 3500 rpm. The precipitate was washed 3-5 times with cold MTBE and dried under vacuum to obtain the crude peptide.
3. Purification of linear peptides: The dissolution solution was prepared according to a volume ratio of mobile phase A (0.1% TFA-water): mobile phase B (0.1% TFA-acetonitrile) = 1.5:1. The crude peptide was dissolved in the dissolution solution to prepare the stock solution. A 0.45-micron filter membrane was used to filter the solution. A C18 reverse-phase preparative column (20 * 250 mm, particle size: 5 µm) was used to perform gradient elution with mobile phase A and mobile phase B at a flow rate of 10 mL/min. The target peak was collected and lyophilized to obtain the target peptide compound (i.e., linear peptide).
4. The preparation of the Staple linker (or modification group A) is performed using the classical Fmoc-tBu solid-phase synthesis method. In this step, Staple-A1 (or modification group A1) is used as an example, and the reaction conditions are as follows (see FIG. 1 for the specific process):
(1) Resin swelling: The Fmoc-L-Lys(ivDde)-2CTC resin was added to DCM. The reaction was sparged with N₂ at room temperature for 1 h and filtered by suction. The resin was then washed 2-3 times with DMF.
(2) Fmoc protecting group removal: A solution of 20% piperidine in DMF was added to the resin. The reaction was sparged with N₂ at room temperature for 10 min, filtered by suction, and the operation was repeated until complete deprotection was achieved. The resin was then washed 2-3 times with DMF.
(3) Fatty acid chain coupling: Reagents were added according to the reaction ratio of resin: fatty acid: HATU: DIPEA = 1:5:5:10. Fatty acid mono-tert-butyl ester, HATU, and DIPEA were pre-dissolved in DMF. The reaction was sparged with N₂ at room temperature for 10 min, then transferred to a resin reaction vessel. The reaction was sparged with N₂ at room temperature for 1-3 h. After the reaction, the mixture was filtered by suction and washed 2-3 times with DMF.
(4) ivDde protecting group removal: A solution of 5% hydrazine in DMF was added to the resin, and the reaction was sparged with N₂ at room temperature for 10 min. This operation was repeated 2-3 times until complete removal was achieved. The resin was then washed 2-3 times with DMF.
(5) Coupling of amino acid, AEEA, and bromoacetic acid: Reagents were added according to the reaction ratio of resin: Fmoc-AA-OH: DIC: Oxyma Pure = 1:5:5:5. The reactants, DIC and Oxyma pure were pre-dissolved in DMF. The reaction was sparged with N₂ at room temperature for 10 min, then transferred to a resin reaction vessel. The reaction was sparged with N₂ at room temperature for 1-3 h. After the reaction, the mixture was filtered by suction and washed 2-3 times with DMF. After the synthesis was completed, the resin was washed 2-3 times with DCM and dried under vacuum to obtain the linker resin.
   The Staple linker may also be selected from the following structures:

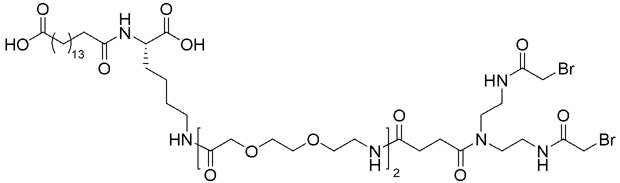

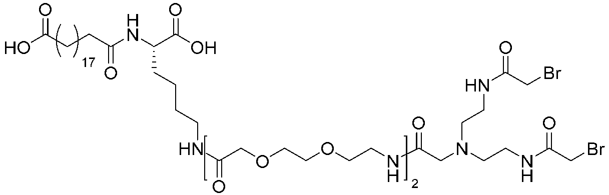

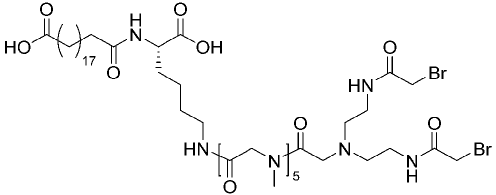

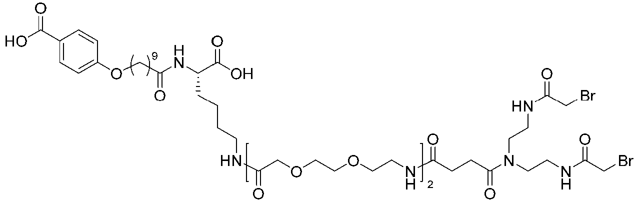

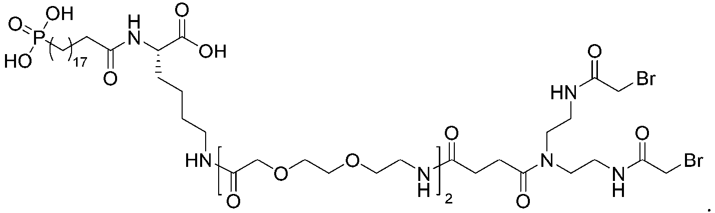

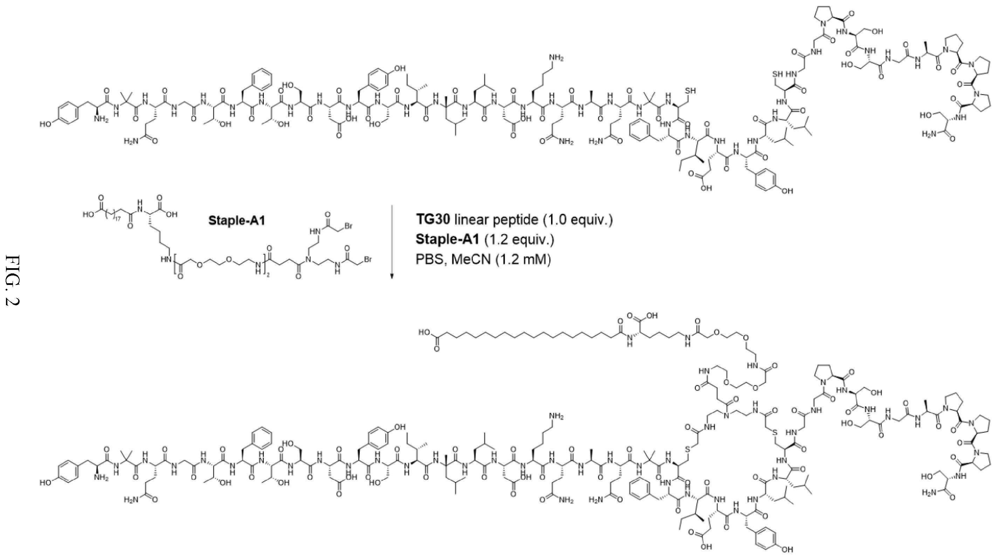

5. Coupling of linear peptide to staple linker:
1.0 eq. of linear peptide (i.e., the target peptide compound obtained in step 3; in this step, Polypeptide 30 (referred to as TG30) in Table A is used as an example) and 1.2 eq. of Staple linker (i.e., the Staple linker obtained in step 4) were dissolved in a reaction solution at a volume ratio of PBS: acetonitrile = 1:1.5 (final concentration: 1.2 mM). The pH of the reaction solution was adjusted to 8.0 using a 10% NaOH aqueous solution. The solution was placed on a shaker at room temperature and shaken thoroughly for 3-8 h until the linear peptide was completely consumed as detected by LC-MS. The reaction solution was neutralized to pH 6.5 using a 5% TFA aqueous solution. The solution was filtered using a 0.45-micron filter membrane. A C18 reverse-phase preparative column (20 * 250 mm, particle size: 5 µm) was used to perform gradient elution with mobile phase A and mobile phase B at a flow rate of 10 mL/min. The target peak was collected and lyophilized to obtain the target coupling compound (i.e., a polypeptide derivative). See FIG. 2 for the specific process. The purity and mass spectrometry identification of each polypeptide derivative were conducted. The purity was more than 90% as detected by HPLC, and the molecular weights of polypeptides identified by mass spectrometry identification were basically consistent with the theoretical molecular weights (all within the allowable range of error). In this example, the molecular weights of some polypeptide molecules were shown by way of example, see Table 1 for details.

**Table 1: Compound molecular weight**

| Polypeptid e name | Calc MS [M+5H]⁵ + | Found MS [M+5H]⁵ + | Modificatio n group | Polypeptid e name | Calc MS [M+5H]⁵ + | Found MS [M+5H]⁵ + | Modificatio n group |
|---|---|---|---|---|---|---|---|
| TG1 | 1040.17 | 1040.37 | A1 | TG39 | 1045.20 | 1045.33 | A1 |
| TG2 | 1104.03 | 1104.22 | A1 | TG40 | 1045.99 | 1045.72 | A1 |
| TG3 | 1030.74 | 1031.09 | A1 | TG41 | 1045.20 | 1045.33 | A1 |
| TG4 | 1022.14 | 1022.37 | A1 | TG42 | 1044.19 | 1044.13 | A1 |
| TG5 | 1030.36 | 1030.91 | A1 | TG43 | 1044.79 | 1044.7 | A1 |
| TG6 | 1049.39 | 1049.56 | A1 | TG44 | 1032.97 | 1032.87 | A1 |
| TG7 | 1054.99 | 1055.03 | A1 | TG45 | 1038.58 | 1038.54 | A1 |
| TG8 | 1057.99 | 1058.19 | A1 | TG46 | 1020.36 | 1020.36 | A1 |
| TG9 | 1029.75 | 1029.24 | A1 | TG47 | 1043.37 | 1043.63 | A1 |
| TG10 | 1055.58 | 1055.96 | A1 | TG48 | 1046.18 | 1046.17 | A1 |
| TG11 | 1030.17 | 1030.53 | A1 | TG49 | 1034.96 | 1035.17 | A1 |
| TG12 | 1019.76 | 1019.96 | A1 | TG50 | 1054.59 | 1054.43 | A1 |
| TG13 | 1035.18 | 1035.36 | A1 | TG51 | 1045.99 | 1046.04 | A1 |
| TG14 | 1060.61 | 1060.79 | A1 | TG52 | 1040.38 | 1040.37 | A1 |
| TG15 | 1042.39 | 1042.69 | A1 | TG53 | 1033.57 | 1033.57 | A5 |
| TG16 | 1087.06 | 1087.42 | A1 | TG55 | 1053.40 | 1053.27 | A8 |
| TG17 | 1052.39 | 1052.62 | A1 | TG56 | 1057.40 | 1057.47 | A1 |
| TG18 | 1037.18 | 1037.49 | A1 | TG57 | 1057.40 | 1057.53 | A1 |
| TG19 | 1067.57 | 1068.03 | A1 | TG58 | 1060.21 | 1060.08 | A1 |
| TG20 | 1039.39 | 1039.91 | A1 | TG59 | 1023.36 | 1023.65 | A1 |
| TG21 | 1042.19 | 1042.41 | A1 | TG61 | 1051.60 | 1051.75 | A1 |
| TG22 | 1042.79 | 1043.06 | A1 | TG62 | 1028.97 | 1028.73 | A1 |
| TG23 | 1045.20 | 1045.20 | A1 | TG63 | 1061.20 | 1061.41 | A1 |
| TG24 | 1045.20 | 1045.57 | A1 | TG64 | 1050.77 | 1050.41 | A1 |
| TG25 | 1045.20 | 1045.57 | A1 | TG65 | 1066.38 | 1065.99 | A1 |
| TG26 | 1045.20 | 1045.29 | A1 | TG67 | 1025.18 | 1025.17 | A1 |
| TG27 | 1050.81 | 1051.04 | A1 | TG68 | 1031.80 | 1031.53 | A1 |
| TG28 | 1047.00 | 1047.61 | A1 | TG69 | 1025.80 | 1025.56 | A1 |
| TG29 | 1039.59 | 1040.19 | A1 | TG70 | 1020.00 | 1020.09 | A1 |
| TG30 | 1033.98 | 1034.06 | A1 | TG71 | 1023.80 | 1023.71 | A1 |
| TG31 | 1039.59 | 1039.61 | A1 | TG72 | 1039.58 | 1039.61 | A1 |
| TG32 | 1052.80 | 1052.9 | A1 | TG73 | 1034.56 | 1034.71 | A1 |
| TG33 | 1051.00 | 1050.86 | A1 | TG74 | 1045.59 | 1045.52 | A1 |
| TG34 | 1042.39 | 1042.47 | A1 | TG75 | 1036.76 | 1036.87 | A1 |
| TG35 | 1039.59 | 1039.42 | A1 | TG76 | 1039.60 | 1039.61 | A1 |
| TG36 | 1045.20 | 1045.27 | A1 | TG77 | 1039.60 | 1239.61 | A1 |
| TG37 | 1048.00 | 1047.94 | A1 | TG78 | 1045.21 | 1245.33 | A1 |
| TG38 | 1039.39 | 1039.48 | A1 | TG79 | 1046.20 | 1046.15 | A1 |

### Example 2: Preparation method for polypeptides or derivatives thereof

1. The linear peptide synthesis of Polypeptide 84 to Polypeptide 94 (referred to as TG84 to TG94) in Table A was performed using the classical Fmoc-tBu solid-phase synthesis method with a Symphony^{®} X peptide synthesizer under the following reaction conditions:
(1) Resin swelling: The Rink Amide MBHA resin was added to DCM. The reaction was sparged with N₂ at room temperature for 1 h and filtered by suction. The resin was then washed 2-3 times with DMF.
(2) Fmoc protecting group removal: A solution of 20% piperidine in DMF was added to the resin. The reaction was sparged with N₂ at room temperature for 10 min, filtered by suction, and the operation was repeated until complete deprotection was achieved. The resin was then washed 2-3 times with DMF.
(3) Amino acid coupling: Reagents were added according to the reaction ratio of resin: Fmoc-AA-OH: DIC: Oxyma Pure = 1:5:5:5. Fmoc-AA-OH, DIC, and Oxyma pure were pre-dissolved in DMF. The reaction was sparged with N₂ at room temperature for 10 min, then transferred to a resin reaction vessel. The reaction was sparged with N₂ at room temperature for 1-3 h. After the reaction, the mixture was filtered by suction and washed 2-3 times with DMF. The amino acids with Boc-L-His(Trt)-OH or Boc-L-Tyr(tBu)-OH at the N-terminus were also synthesized using the method described above. After the synthesis was completed, the resin was washed with DCM 2-3 times and dried under vacuum to obtain peptide resin containing linear peptide.
   The above methods are applicable to the amino acids (D or L forms) or synthesis reagents, including but not limited to: Fmoc-AEEA-OH, Fmoc-Aad(tBu)-OH, Fmoc-Ac4c-OH, Fmoc-Aib-OH, Fmoc-Ala-OH, Fmoc-Arg(Pbf)-OH, Fmoc-Asp(tBu)-OH, Fmoc-Cys(Trt)-OH, Fmoc-Dab(Ac)-OH, Fmoc-Dap(Ac)-OH, Fmoc-Gly-OH, Fmoc-Glu(tBu)-OH, Fmoc-Gln(Trt)-OH, Fmoc-His(Trt)-OH, Fmoc-Ile-OH, Fmoc-Iva-OH, Fmoc-Leu-OH, Fmoc-Lys(Ac)-OH, Fmoc-Lys(Boc)-OH, Fmoc-Lys(Mtt)-OH, Fmoc-Lys(Alloc)-OH, Fmoc-Lys(ivDde)-OH, Fmoc-Met-OH, Fmoc-Nal-OH, Fmoc-Nle-OH, Fmoc-Orn(Boc)-OH, Fmoc-Pal-OH, Fmoc-Pro-OH, Fmoc-Phe-OH, Fmoc-Phe(2-F)-OH, Fmoc-Phe(3-F)-OH, Fmoc-Phe(4-F)-OH, Fmoc-Ser(tBu)-OH, Fmoc-Thr(tBu)-OH, Fmoc-Tle-OH, Fmoc-Trp(Boc)-OH, Fmoc-Tyr(tBu)-OH, Fmoc-Val-OH, Fmoc-α-methyl-Leu-OH, Fmoc-α-methyl-Lys(Boc)-OH, Fmoc-α-methyl-Phe-OH, Fmoc-α-methyl-Phe(2-F)-OH, Fmoc-AEEA-OH, Fmoc-Glu-OtBu, Fmoc-Asp-OtBu, Fmoc-Ida(Allyl)-OH, Boc-L-His(Trt)-OH, Boc-L-Tyr(tBu)-OH, myristic acid, stearic acid, arachidic acid, docosanoic acid, 18-(tert-butoxy)-18-oxooctadecanoic acid, 20-(tert-butoxy)-20-oxoicosanoic acid, and the like.

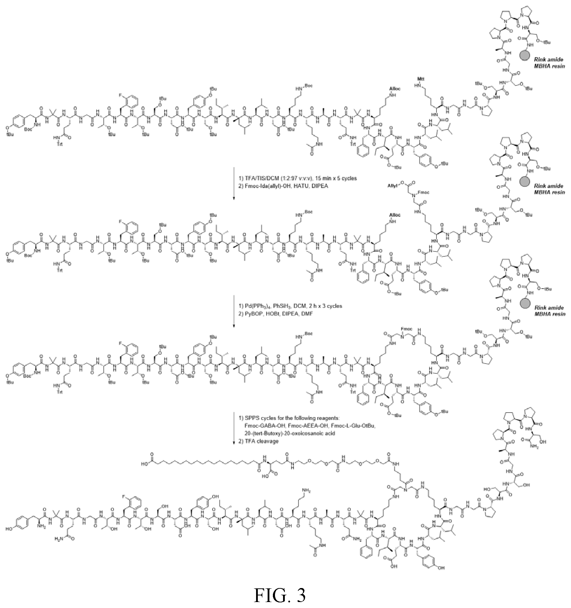

2. The coupling of the Staple linker (or modification group B) and the peptide resin containing a linear peptide (TG85) obtained in step 1 was as follows (for the specific process, see FIG. 3):
2.1 Removal of Mtt protecting group from the peptide resin: The deprotection solution was prepared at a volume ratio of TFA: TIPS: DCM=1:2:97, and Mtt removal solution was added to the resin at 10 mL/g resin. The mixture was shaken thoroughly on a shaker for 15 min, after which the resin was filtered by suction. This process was repeated 3-5 times until the removal solution no longer turned bright yellow upon addition, indicating complete deprotection. The resin was then washed with DMF 2-3 times.
2.2 Removal of the Alloc and Allyl protecting groups from the peptide resin: DCM (10 mL/g resin), PhSiH3 (10 eq.), and Pd(PPh3)4 (0.1 eq.) were added to the resin. The reaction was sparged with N₂ at room temperature for 1 h, and the resin was then filtered by suction. The process was repeated 1-2 times, and the resin was washed with DMF 2-3 times.
2.3 Ring closing reaction on peptide resin: DMF (10 mL/g resin), PyBOP (5 eq.), HOBt (5 eq.), and DIPEA (10 eq.) were added to the resin from which the Alloc and Allyl protecting groups had been removed. The reaction was sparged with N₂ at room temperature for 4 h, and the resin was then filtered by suction. The process was repeated 1-2 times, and the resin was washed 2-3 times with DMF.
2.4 Solid-phase side chain introduction: Standard solid-phase synthesis procedures were followed, in which the side chain was of the following structure:

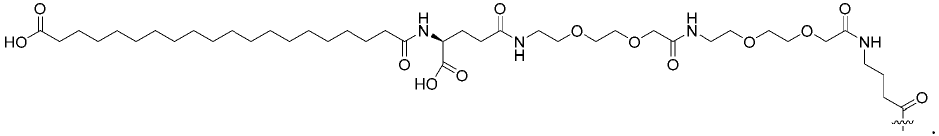

3. Cleavage and drying: The polypeptide cleavage solution was prepared at a volume ratio of TFA: TIPS: H₂O: EDT = 95:2:2:1. The cleavage solution was added to the dried resin at 10 mL/g resin, and then placed on a shaker for thorough shaking for 3 h. The resin residue was filtered out. Ten volumes of cold MTBE were added to the filtrate. The resulting suspension was cooled at -20°C for 1 h and then centrifuged at 3500 rpm. The precipitate was washed 3-5 times with cold MTBE and dried under vacuum to obtain the crude peptide.
4. Polypeptide purification: The dissolution solution was prepared according to a volume ratio of mobile phase A (0.1% TFA-water) and mobile phase B (0.1% TFA-acetonitrile) = 1.5:1. The crude peptide was dissolved in the dissolution solution to prepare the stock solution. A 0.45-micron filter membrane was used to filter the solution. A C18 reverse-phase preparative column (20 * 250 mm, particle size: 5 µm) was used to perform gradient elution with mobile phase A and mobile phase B at a flow rate of 10 mL/min. The target peak was collected and lyophilized to obtain the target peptide derivative, i.e., Polypeptide 84 to Polypeptide 94 (referred to as TG84 to TG94). The purity and mass spectrometry identification of each polypeptide derivative were conducted. The purity was more than 90% as detected by HPLC, and the molecular weights of polypeptides identified by mass spectrometry identification were basically consistent with the theoretical molecular weights (all within the allowable range of error). In this example, the molecular weights of some polypeptide molecules were shown by way of example, see Table 2 for details.

**Table 2: Compound molecular weight**

| Polypeptide name | Calc MS [M+5H]⁵⁺ | Found MS [M+5H]⁵⁺ | Modification group |
|---|---|---|---|
| TG84 | 1021.75 | 1021.74 | B1 |
| TG85 | 1035.97 | 1036.24 | B1 |
| TG86 | 1012.75 | 1012.78 | B2 |
| TG87 | 1013.34 | 1013.48 | B1 |

### Test example 1: Determination of in vitro activity

1. Insulinotropic analogues can bind to the target receptor on the cell membrane, activate the cAMP response element (CRE), and initiate the expression of downstream luciferase. The expression level is positively correlated with the biological activity of the analogue being tested. After activation, luciferase substrate is added for chemiluminescence detection, and the luminescence intensity is determined to characterize the biological activity of the test compounds. Therefore, the target peptide derivatives (or polypeptide derivatives, i.e., TG1-TG94) prepared in Example 1 and Example 2 can be tested by the CreLuc method. The specific steps are as follows:
The stably transfected cell lines HEK293/pGM-CREB-L-Luc/GLP-1R pool, HEK293/pGM-CREB-L-Luc/GIPR pool, and 293HEK/pGM-CREB-L-Luc/GCGR pool were constructed. The target peptide derivatives (TG1 to TG94) prepared in Example 1 and Example 2 were diluted 5-fold from a maximum of 100 nM to prepare a dilution series including 11 concentration gradients. Human GLP-1, GIP, and Glucagon were selected as the test positive controls, and were diluted using the same method. The corresponding test cells were digested (1 min), dispersed by pipetting, and centrifuged (1000 rpm, 5 min). The culture medium was discarded, and the cells were resuspended in freestyle medium, centrifuged again, collected, and counted. The cells were then diluted to a cell density of 5 x 10⁵ cells/mL and then added to the detection wells of a 384-well plate (40 µL/20,000 cells/well). Echo was used to add the corresponding analyte dilution series. The cell plate was incubated at 37°C and 5% CO₂ for 6 h, then ONE-GLO (20 µL/well) was added for detection. It was kept in the dark for 3 min, then a chemiluminescent microplate reader was used for determination. The plate was read within 30 min, and the determination results were recorded. The EC₅₀ values of the target peptide derivatives were calculated by plotting activation curves using GraphPad Prism software, and the results were shown in Table 3. The results indicated that the polypeptide derivatives of the present disclosure show binding activity to GLP-1R, GIPR, and GCGR.

**Table 3: EC₅₀ values for target peptide derivatives**

| Polypeptide name | EC₅₀ (pM) for each receptor | | |
|---|---|---|---|
| | GLP-1R | GIPR | GCGR |
| GLP-1 | 96.4 | NA | NA |
| GIP | NA | 55.5 | NA |
| Glucagon | NA | NA | 427.9 |
| TG8 | 243.4 | 425.7 | 835.6 |
| TG10 | 358.7 | 367.8 | 148.8 |
| TG12 | 204.5 | 66.6 | 325.3 |
| TG14 | 310.6 | 752.1 | 276.9 |
| TG15 | 116.3 | 85.6 | 328.8 |
| TG18 | 66.9 | 25.9 | 801.5 |
| TG22 | 664.8 | 18.3 | 204.9 |
| TG23 | 92.9 | 75.6 | 109 |
| TG25 | 54 | 24.4 | 311.3 |
| TG26 | 149.2 | 59.4 | 359.7 |
| TG27 | 136.4 | 46.5 | 363.1 |
| TG28 | 60.2 | 30 | 481.9 |
| TG29 | 98.3 | 16.7 | 312 |
| TG30 | 47.8 | 45.3 | 346.7 |
| TG31 | 32.7 | 11.4 | 440.5 |
| TG32 | 34 | 11.7 | 196.7 |
| TG33 | 23.7 | 7.6 | 96.8 |
| TG35 | 40.1 | 15.5 | 273.2 |
| TG36 | 34.1 | 10.8 | 812.8 |
| TG38 | 70.2 | 14.5 | 232.4 |
| TG40 | 33.9 | 12.5 | 92.9 |
| TG41 | 282.1 | 19.6 | 76.3 |
| TG42 | 477.1 | 21.3 | 99.4 |
| TG43 | 57.1 | 13.6 | 78.7 |
| TG52 | 111.9 | 18.9 | 173.3 |
| TG56 | 196.6 | 39.5 | 681.3 |
| TG59 | 51.8 | 30.7 | 392.5 |
| TG61 | 263.5 | 12.6 | 267.7 |
| TG62 | 370.9 | 27.9 | 783.1 |
| TG64 | 92.9 | 14.9 | 52.8 |
| TG66 | 435.1 | 34.7 | 201.4 |
| TG85 | 254.5 | 61.2 | 330 |
| TG87 | 171.3 | 27.6 | 171.5 |

2. Insulinotropic analogues can bind to target receptors on the cell membrane, activate the receptors, and induce release. The activity of the test substance can be determined using the HTRF method with a cAMP detection kit. Therefore, the cAMP method can be used to detect the target peptide derivatives (or polypeptide derivatives, i.e., TG1-TG86) prepared in Example 1 and Example 2. The specific steps are as follows:
The stably transfected cell lines HEK293/pGM-CREB-L-Luc/GLP-1 pool, HEK293/pGM-CREB-L-Luc/GIP pool, and HEK293/pGM-CREB-L-Luc/GCG pool were constructed. The target peptide derivatives (TG1 to TG86) prepared in Example 1 and Example 2 were diluted 5-fold from a maximum of 100 nM to prepare a dilution series including 11 concentration gradients. Human GLP-1, GIP, and Glucagon were selected as the test positive controls, and were diluted using the same method. The test cell line was selected, treated with cAMP-specific trypsin for digestion. 3 mL of serum-free DMEM culture medium was added, and the cells were pipetted, resuspended, and counted. 5 µL of IBMX (prepared with serum-free DMEM medium, 0.5 mM) and test cells (5 µL/7500 cells/well) were added in a 384-well plate sequentially. The plate was incubated at 37°C under 5% CO₂ for 30 min, followed by the addition of cAMP-d2 (5 µL, 1x) and Anti-cAMP-Cryptate (5 µL, 1x). The mixture was kept at room temperature for 1 h, after which the HTRF value was measured using a chemiluminescent microplate reader, and the determination result was recorded. The EC₅₀ values of the target peptide derivatives were calculated by plotting activation curves using GraphPad Prism software, and the results were shown in Table 4. The results indicated that the polypeptide derivatives of the present disclosure show binding activity to GLP-1R, GIPR, and GCGR.

**Table 4: EC₅₀ values for target peptide derivatives**

| Polypeptide name | EC₅₀ (pM) for each receptor | | |
|---|---|---|---|
| | GLP-1R | GIPR | GCGR |
| GLP-1 | 1.8 | NA | NA |
| GIP | NA | 1.6 | NA |
| Glucagon | NA | NA | 1.4 |
| TG10 | 2.5 | 9.7 | 8.5 |
| TG12 | 5.8 | 12.1 | 40.3 |
| TG15 | 1.9 | 3.3 | 40.3 |
| TG18 | 1.4 | 1.7 | 94.4 |
| TG25 | 2.2 | 0.4 | 65.1 |
| TG28 | 2.2 | 0.7 | 83.7 |
| TG29 | 3.7 | 1.1 | 65.1 |
| TG30 | 2.5 | 3.2 | 28.7 |
| TG40 | 0.7 | 3.1 | 2.1 |
| TG43 | 1.3 | 6.4 | 3.8 |
| TG52 | 4.1 | 3.8 | 8.8 |
| TG59 | 0.4 | 3.9 | 9.1 |
| TG61 | 0.5 | 5.9 | 12.4 |
| TG62 | 0.8 | 6.0 | 62.2 |

The polypeptide derivative disclosed in Patent PCT/CN2022/097622 was prepared using the methods described in Example 1 and Example 2, and the activity of this polypeptide derivative was evaluated using the CreLuc method. The results show that the polypeptide derivative had binding activity only to two of the receptors GLP-1R, GIPR, and GCCR, and could not have agonistic activity to all three receptors (GLP-1R, GIPR, and GCCR) simultaneously.

### Test example 2: In vivo pharmacokinetic evaluation

The pharmacokinetic behavior of target peptide derivatives (or polypeptide derivatives) prepared in Example 1 and Example 2, i.e., TG1-TG94 in rodents was tested. The specific steps are as follows:

The target peptide derivatives (TG1-TG94) to be tested were administered to male SD rats via subcutaneous injection (SC, 3 mg/kg; drug vehicle: PBS, concentration: 1.5 mg/mL; administration volume: 2 mL) and intravenous injection (IV, 1 mg/kg; drug vehicle: PBS, concentration: 0.5 mg/mL; administration volume: 2 mL) as a single dose, respectively. Whole blood samples were collected from the jugular vein or other appropriate vein at the following time points after administration: for SC, 15 min, 30 min, 1 h, 2 h, 4 h, 6 h, 8 h, 24 h, 48 h, 72 h, 96 h, 120 h, and 168 h; for IV, 5 min, 15 min, 30 min, 1 h, 2 h, 4 h, 8 h, 24 h, 48 h, 72 h, 96 h, 120 h, and 168 h. 0.2 mL of blood was collected and transferred into a labeled EDTA-K2 anticoagulant tube. The tube was gently inverted to ensure thorough mixing of the anticoagulant (EDTA-K2) with the blood and immediately placed on wet ice. Plasma separation was performed as soon as possible by centrifugation under the following conditions: 4°C, 6800xg, for 6 min. The plasma samples were stored in a refrigerator at no higher than -20°C and used for analysis when needed.

Under the condition of yellow light in an ice-water bath, except for blank samples, 300 µL of precipitant containing an internal standard was added to a 96-well plate containing 20 µL of standard curve sample, quality control sample, or unknown sample; 300 µL of acetonitrile was added to the blank sample. The mixture was vortexed, mixed well, and then centrifuged. 150 µL of the supernatant after centrifugation was transferred to a new 96-well plate, and 150 µL of ultrapure water was added and mixed well. The above samples were subjected to quantitative analysis using LC-MS analysis with XB-C18 (2.1 * 100 mm, 3 µm) chromatography for gradient elution. The mobile phases used were: mobile phase A = 0.1% formic acid-water; mobile phase B = acetonitrile. Phoenix WinNonlin software was used to calculate the pharmacokinetic parameters. The results show that the polypeptide derivatives of the present disclosure exhibited good in vivo absorption, high blood drug concentration, and long half-life in rats. The testing results of pharmacokinetic parameters for some polypeptide derivatives were shown in Tables 5A and 5B.

**Table 5A: Pharmacokinetic parameters of target peptide derivatives**

| iv | | TG40 | | TG87 | | TG52 | |
|---|---|---|---|---|---|---|---|
| | | Mean | SD | Mean | SD | Mean | SD |
| t_{1/2} | h | 15.0 | 0.965 | 13.9 | 1.63 | 21.5 | 1.71 |
| Kel | h | 0.0462 | 0.00299 | 0.0504 | 0.00610 | 0.0323 | 0.00245 |
| Vdss | L/kg | 0.134 | 0.00895 | 0.0891 | 0.0286 | 0.0920 | 0.00571 |
| MRT_{inf} | h | 22.1 | 1.56 | 21.4 | 2.27 | 26.5 | 1.64 |
| Cl | mL/min/kg | 0.101 | 0.00783 | 0.0706 | 0.0270 | 0.0580 | 0.00532 |
| AUC₀₋ₜ | h*ng/mL | 164493 | 12199 | 254082 | 80563 | 265341 | 21980 |
| AUC_{0-inf} | h*ng/mL | 164976 | 12235 | 256761 | 81098 | 289225 | 27203 |
| AUC₀₋ₜ /AUC_{0-inf} | / | 0.997 | 0.00101 | 0.989 | 0.00365 | 0.918 | 0.0103 |

**Table 5B: Pharmacokinetic parameters of target peptide derivatives**

| sc | | TG40 | | TG87 | | TG52 | |
|---|---|---|---|---|---|---|---|
| | | Mean | SD | Mean | SD | Mean | SD |
| t_{1/2} | h | 13.1 | 1.43 | 15.6 | 0.662 | 26.4 | NA |
| Tₘₐₓ | h | 24 | NA | 24 | NA | 24 | NA |
| Cₘₐₓ | ng/mL | 4773 | 1053 | 4880 | 335 | 8207 | 584 |
| MRT_{inf} | h | 36.9 | 1.79 | 32.3 | 1.53 | 42.8 | NA |
| Cl/F | mL/min/kg | 0.205 | 0.0298 | 0.204 | 0.00818 | 0.0877 | NA |
| Vz/F | L/kg | 0.232 | 0.0386 | 0.276 | 0.0165 | 0.200 | NA |
| AUC₀₋ₜ | h*ng/mL | 246700 | 38755 | 243284 | 9691 | 410455 | 56438 |
| AUC_{0-inf} | h*ng/mL | 247646 | 39237 | 245013 | 9612 | 570292 | NA |
| F(%) | / | 50.0 | 7.93 | 31.8 | 1.25 | 50.9 | NA |
| AUC₀₋ₜ /AUC_{0-inf} | / | 0.996 | 0.00196 | 0.993 | 0.000615 | 0.834 | NA |

The pharmacokinetic behavior of TG52 in non-rodents was tested. The specific steps are as follows:

The target peptide derivative TG52 to be tested was administered to adult male cynomolgus monkeys via subcutaneous and intravenous injections (0.2 mg/kg, with PBS as the drug vehicle) as a single dose, respectively. Whole blood samples were collected from the jugular vein or other appropriate vein at the following time points after administration: for SC, 0 min, 30 min, 1 h, 3 h, 7 h, 24 h, 48 h, 72 h, 120 h, 168 h, 216 h, and 264 h; for IV, 0 min, 5 min, 30 min, 1 h, 3 h, 7 h, 24 h, 48 h, 72 h, 120 h, 168 h, 216 h, and 264 h. 0.2 mL of blood was collected and transferred into a labeled EDTA-K2 anticoagulant tube. The tube was gently inverted to ensure thorough mixing of the anticoagulant (EDTA-K2) with the blood and immediately placed on wet ice. Plasma separation was performed as soon as possible by centrifugation under the following conditions: 4°C, 6800xg, for 6 min. The plasma samples were stored in a refrigerator at no higher than -20°C and used for analysis when needed. Analysis of the drug content in the blood samples was performed using the analytical method described above.

**Table 5C: Pharmacokinetic parameters of TG52 in cynomolgus monkeys**

| sc | | | iv | | |
|---|---|---|---|---|---|
| T_{1/2} | h | 97.2 | T_{1/2} | h | 88.3 |
| Tₘₐₓ | h | 18.3 | Vdss | L/kg | 0.0626 |
| Cₘₐₓ | ng/mL | 1757 | C1 | mL/min/kg | 0.0111 |
| AUC₀₋ₜ | h*ng/mL | 198975 | AUC₀₋ₜ | h*ng/mL | 277564 |
| F (%) | / | 75.7 | AUC_{0-inf} | h*ng/mL | 302154 |

### Test example 3: In vivo pharmacodynamic study

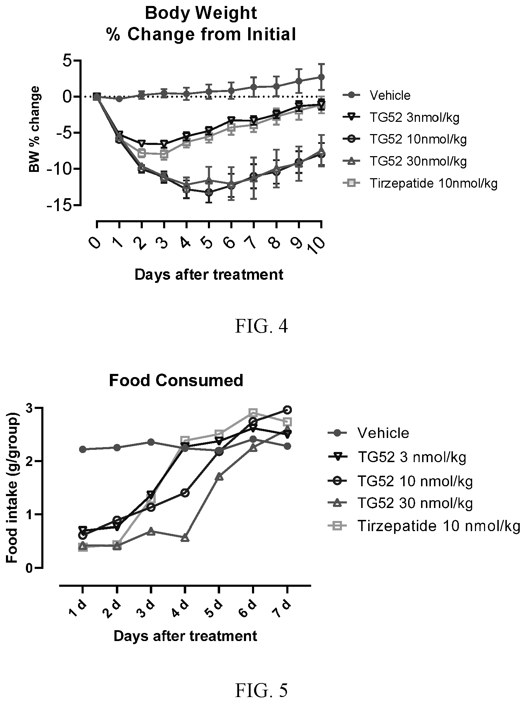

DIO male C57BL/6J mice aged 23-26 weeks, weighing 45-55 g and with blood glucose levels ranging from 8-12 mmol/L, were selected. The DIO mice were randomly divided into groups, with 6 mice per cage, and underwent a 1-week acclimatization period on a high-fat diet with free access to water. After the pre-acclimatization period, the basal blood glucose and body weight of each mouse were measured before drug administration (Day 0). Subsequently, according to the experimental groups, the mice were subcutaneously injected (s.c.) with vehicle control (Vehicle), Tirzepatide (10 nmol/kg), and three doses of the compound of the present disclosure, TG52 (3 nmol/kg, 10 nmol/kg, and 30 nmol/kg). After administration, the mice were provided with a normal high-fat diet and water. On Days 1, 2, 3, 4, 5, 6, and 7 post-administration, the blood glucose levels, body weights, and total daily food intake of each mouse were measured. The changes in blood glucose, body weight (as a percentage of the initial value), and total daily food intake for each group were calculated and plotted. The results were shown in FIG. 4 to FIG. 6.

### Test example 4: Study on the stability against enzymatic hydrolysis

The stability of the target peptide derivatives (or polypeptide derivatives, i.e., TG1-TG94) prepared in Example 1 and Example 2 and Retatrutide (positive control) was tested in simulated gastric fluid (SGF) and simulated intestinal fluid (SIF), respectively. The specific steps are as follows:

The target peptide derivatives to be tested TG1-TG94 and Retatrutide (positive control) were dissolved in PBS to prepare a stock solution (60 µM). Then, 15 µL of the stock solution was diluted with 285 µL of commercial simulated gastric fluid (SGF) and incubated in a water bath at 37°C. Equal volumes of solution (30 µL) were collected at 0 min, 5 min, 15 min, 30 min, 1 h, 2 h, 4 h, and 8 h, respectively. The reactions were neutralized with 0.2 M Na₂CO₃ aqueous solution (30 µL). The remaining peptide amounts were analyzed by LC-MS, and the relative percentages of peptide amounts at different time points compared to 0 min were calculated. The degradation curves were plotted using GraphPad Prism, and the half-life (T_{1/2}) of the target peptide derivative in SGF was calculated.

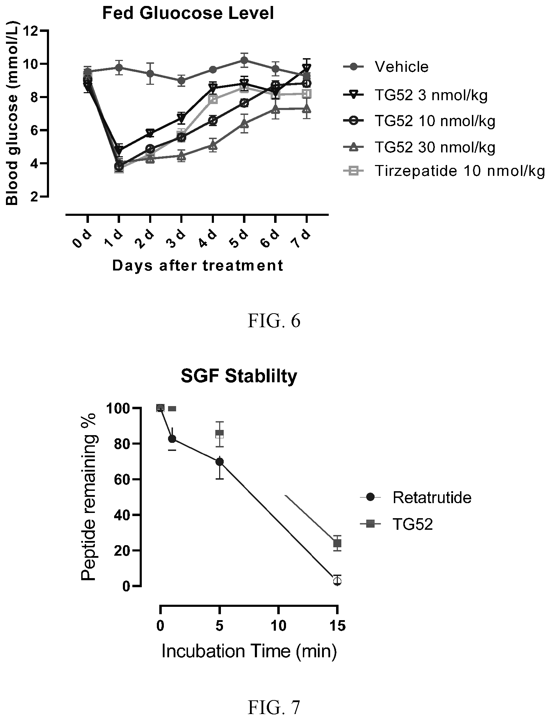

The test compounds were dissolved in PBS to prepare a stock solution (60 µM). Then, 15 µL of the stock solution was diluted with 285 µL of commercial simulated gastric fluid (SGF) and incubated in a water bath at 37°C. Equal volumes of solution (30 µL) were collected at 0 min, 5 min, 15 min, 30 min, 1 h, 2 h, 4 h, and 8 h, respectively. The reactions were neutralized with 10% TFA aqueous solution (30 µL). The remaining peptide amounts were analyzed by LC-MS, and the relative percentages of peptide amounts at different time points compared to the 0 min time point were calculated. The degradation curves were plotted using GraphPad Prism, and the half-life (T_{1/2}) of each target peptide derivative in SIF was calculated. The results show that all the target peptide derivatives have good stability against enzymatic hydrolysis. The stability test results of TG52 and Retatrutide (positive control) in simulated gastric fluid are shown in FIG. 7.

### Test example 5: Comparison of serum albumin binding capacity

To further illustrate that the agonist of the present application can provide stronger serum albumin binding capacity after cyclization, the binding capacities of Semaglutide and the target peptide derivative TG052 to serum albumin were tested based on Step 1 of Test Example 1 in the present application (the only difference from Step 1 of Test Example 1 is the addition of 10% FBS to the cell culture system). The steps are as follows:

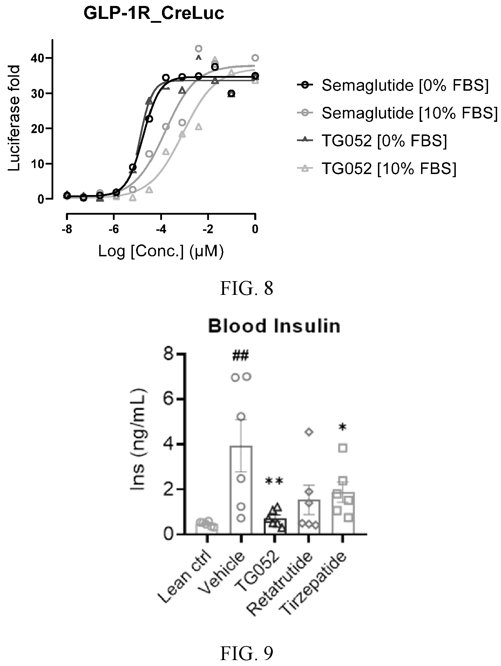

The stably transfected cell line HEK293/pGM-CREB-L-Luc/GLP-1R pool was constructed. TG052 and the positive control Semaglutide were diluted 5-fold from a maximum concentration of 100 nM to prepare a dilution series including 11 concentration gradients. The corresponding test cells were selected, digested (for 1 min), dispersed by pipetting, and centrifuged (1000 rpm, 5 min). The culture medium was discarded, and the cells were suspended using Freestyle culture medium, centrifuged and counted. The cells were diluted to a cell density of 5 x 10⁵ cells/mL using freestyle culture medium (0% FBS) and culture medium containing 10% FBS, respectively. The cells were then added to the detection wells of a 384-well plate (40 µL/20,000 cells/well). Echo was used to add the corresponding analyte dilution series. The cell plate was incubated at 37°C and 5% CO₂ for 6 h, and then ONE-GLO (20 µL/well) was added for detection. It was kept in the dark for 3 min, and then a chemiluminescent microplate reader was used for determination. The plate was read within 30 min, and the determination results were recorded. The activation curves were plotted using GraphPad Prism software, and the EC₅₀ values of the target peptides under 0% and 10% FBS incubation were calculated, respectively. The specific detection results are shown in FIG. 8 and Table 6.

**Table 6**

| **Polypeptide** | **EC₅₀ (pM)** | | **Fold (10%/0%)** |
|---|---|---|---|
| | **0% FBS** | **10% FBS** | |
| Semaglutide | 17.6 | 151.4 | 8.6 |
| **TG052** | **13.1** | **860.8** | **65.7** |

It can be seen from the above that, compared with the positive control Semaglutide (i.e., a linear agonist) modified with a single side-chain fatty acid, the activity reduction (Fold) of the target peptide derivative TG052 in this application is more pronounced in the presence of 10% FBS. This illustrates that TG052 has a stronger binding capacity to FBS and a stronger serum albumin binding capacity. Thus, it can be further illustrated that the cyclic agonist of this application (e.g., TG052) binds more to FBS compared to a linear agonist (e.g., the positive control semaglutide), and TG052 has a stronger binding capacity.

### Test example 6: Comparison of in vivo efficacy

This Test Example compares the therapeutic effects of the target peptide derivative (TG052) in this application, as well as commercially available linear agonists (Glucagon and Retatrutide), in DIO mouse model and nonalcoholic steatohepatitis (NASH) mouse model.

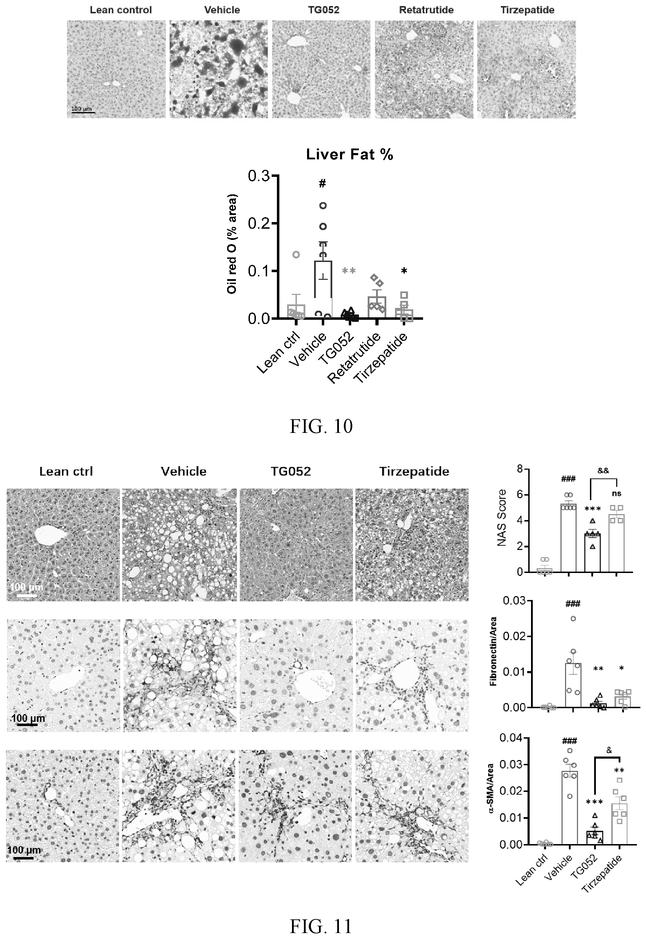

4.1 DIO male C57BL/6J mice aged 23-26 weeks, weighing 45-55 g, and with blood glucose levels ranging from 8-12 mmol/L were selected. The DIO mice were randomly divided into groups, with 6 mice per cage, and underwent a 1-week acclimatization period on a high-fat diet with free access to water. After the animal pre-acclimation period, the basal blood glucose and body weight values of each mouse were measured before administration (Day 0). Subsequently, according to the experimental groups, the mice were subcutaneously injected (s.c.) with the vehicle control (Vehicle), Tirzepatide (10 nmol/kg), Retatrutide (10 mg/kg), and the compound of this application, TG52 (10 nmol/kg). Additionally, normal male C57BL/6J mice aged 23-26 weeks were used as a healthy control (Lean control) and administered the vehicle. After administration, the mice were provided with a normal high-fat diet and water, and were subcutaneously injected once every three days (Q3D) for 21 consecutive days. On Day 22 of the experiment, all animals were euthanized, and blood and organ samples were collected for analysis. A commercially available insulin detection kit was used to determine the insulin content in the blood of each mouse. Each mouse's liver tissue was fixed with a 4% paraformaldehyde fixative solution (4% PFA), embedded in paraffin, sectioned to obtain liver tissue sections. Each mouse's liver tissue was stained with Oil Red O staining to obtain stained sections. In addition, the fat content in the liver tissue was counted. The detailed test results are shown in FIG. 9 and FIG. 10.

As can be seen from FIG. 9, the target peptide derivative (TG052) in this application can more effectively improve the insulin resistance status and reduce blood insulin levels in obese mice compared to the positive control drugs Retatrutide and Tirzepatide.

As can be seen from FIG. 10, the target peptide derivative (TG052) in this application can more significantly and effectively clear fat accumulation in the liver and improve liver metabolic status in obese mice compared to the positive control drugs Retatrutide and Tirzepatide.

4.2 DIO male C57BL/6J mice aged 18-20 weeks were fed with a high-fat diet (HFD) for 10 weeks, and then the mice were divided into 3 groups of 6 mice each for the test, namely a control group (administered with vehicle + carbon tetrachloride), a TG52 group (administered with TG52 10 nmol/kg + carbon tetrachloride), and a Tirzepatide group (administered with Tirzepatide 10 nmol/kg + carbon tetrachloride). Meanwhile, a healthy control (Lean control) group was set up, where normal male C57BL/6J mice aged 18-20 weeks were fed with a normal diet for 10 weeks for the test (administered with vehicle + vehicle). In the above four groups, the mice were administered once every three days (Q3D) for 8 weeks. Subsequently, all mice were euthanized, and liver tissues were collected for the test. Each mouse liver's tissue was fixed with 4% paraformaldehyde (4% PFA), embedded in paraffin, sectioned to obtain liver tissue sections, and then stained with hematoxylin-eosin (H&E). Stained sections were obtained by staining the target proteins with commercially available antibodies (Fibronectin and α-smooth muscle actin (α-SMA)). Finally, statistical analysis was performed on each stained section to obtain quantitative analysis results (FIG. 11, right side). The specific detection results are shown in FIG. 11.

As can be seen from FIG. 11, compared to the positive control drug Tirzepatide, the target peptide derivative (TG052) of the present application can more significantly and effectively improve the liver inflammatory state, more significantly reverse the liver fibrosis state, and significantly reduce the levels of liver fibrosis markers.

In the specification of this specification, references to terms such as "one embodiment", "some embodiments", "example", "specific example", or "some examples" indicate that the specific features, structures, materials, or characteristics described in connection with the embodiment or example are included in at least one embodiment or example of the present disclosure. In this specification, the schematic representations of the aforementioned terms do not necessarily pertain to the same embodiment or example. Furthermore, the specific features, structures, materials, or characteristics described may be combined in any suitable manner in one or more embodiments or examples. Furthermore, without contradiction, those skilled in the art may combine and assemble different embodiments or examples described in this specification, as well as the features of those embodiments or examples.

Although embodiments of the present disclosure have been shown and described, it should be understood that these embodiments are illustrative and not restrictive. Those of ordinary skill in the art may make changes, modifications, substitutions, and variations to the aforementioned embodiments within the scope of the present disclosure.

## Claims

1. A polypeptide or a derivative or pharmaceutically acceptable salt thereof, wherein the polypeptide or the derivative thereof has a structure represented by Formula (I):
X₁X₂X₃GTX₆X₇X₈DX₁₀X₁₁X₁₂X₁₃X₁₄X₁₅X₁₆X₁₇X₁₈X₁₉X₂₀X₂₁X₂₂X₂₃X₂₄X₂₅LX₂₇X₂₈X₂₉X ₃₀PX₃₂SX₃₄X₃₅PPPX₃₉ (I),
wherein:
X₁ is Y, H, 3-iodo-Y, 4-Pal, or 4-amino-F;
X₂ is Aib, s, or Ac4c;
X₃ is Q, H, or Dab(Ac);
X₆ is F, F(2-F), or αMeF;
X₈ is S, HoS, or αMeS;
X₁₀ is Z₂, Y, Z₁, L, or 4-Pal;
X₁₁ is S or αMeS;
X₁₂ is K, R, or I;
X₁₃ is Z₂, Z₁, Y, L, A, αMeL, Q, Aib, or Iva;
X₁₄ is Z₂, Z₁, L, K(Ac), HoL, Npg, or Tle;
X₁₅ is D, E, or Aad;
X₁₆ is K, E, K(Ac), or αMeK;
X₁₇ is R, Z₂, Z₁, K(Ac), Q, or I;
X₁₈ is Z₂, Z₁, A, R, or Y;
X₁₉ is A, Q, Aib, H, or K(Ac);
X₂₀ is Q, Z₂, Aib, H, R, a, or Z₁;
X₂₁ is Z₁, D, A, L, Z₂, or Aib;
X₂₂ is F or HoF;
X₂₃ is V or I;
X₂₄ is Z₂, Q, Z₁, or E;
X₂₅ is Z₂, Z₁, W, or Y;
X₂₇ is L, K, or I;
X₂₈ is A, D, E, Z₁, or Z₂;
X₂₉ is G or H;
X₃₀ is G or H;
X₃₂ is Z₂, Z₁, S, or P;
X₃₄ is G or Aib;
X₃₅ is A, Q, or K;
X₃₉ is S or K,
wherein, in the structure represented by Formula (I), two sites in any of the following groups are each independently selected from amino acid Z₁ with a side chain containing -SH, or wherein, in the structure represented by Formula (I), two sites in any of the following groups are each independently selected from amino acid Z₂ with a side chain containing -NH₂:
1) i and i+3;
2) i and i+4;
3) i and i+7; or
4) i and i+11, wherein:
i is 10, 14, 17, or 21;
the two amino acids Z₁ with the side chain containing -SH are each independently selected from C, c, αMeC, HoC, Hoc, Pen, or N-Me-C; and
the two amino acids Z₂ with the side chain containing -NH₂ are each independently selected from K, k, αMeK, HoK, Dap, Dab, Orn, or N-Me-K.

2. The polypeptide or the derivative or pharmaceutically acceptable salt thereof according to claim 1, wherein the two sites i and i+3, i and i+4, i and i+7, or i and i+11 in the structure represented by Formula (I) are the amino acids Z₁ with the side chain containing -SH, i being 10, 14, 17, or 21;
optionally, the two sites i and i+3 in the structure represented by Formula (I) are the amino acids Z₁ with the side chain containing -SH, i being 10, 14, 17, or 21, and preferably 17;
optionally, the two sites i and i+4 in the structure represented by Formula (I) are the amino acids Z₁ with the side chain containing -SH, i being 10, 14, 17, or 21, and preferably 10;
optionally, the two sites i and i+7 in the structure represented by Formula (I) are the amino acids Z₁ with the side chain containing -SH, i being 10, 14, 17, or 21, and preferably 14, 17, or 21;
optionally, the two sites i and i+11 in the structure represented by Formula (I) are the amino acids Z₁ with the side chain containing -SH, i being 10, 14, 17, or 21, and preferably 10 or 17;
optionally, wherein the two sites i and i+3, i and i+4, i and i+7, or i and i+11 in the structure represented by Formula (I) are the amino acids Z₂ with the side chain containing -NH₂, i being 10, 14, 17, or 21;
optionally, the two sites i and i+3, or i and i+7 in the structure represented by Formula (I) are the amino acids Z₂ with the side chain containing -NH₂, i being 17 or 21;
optionally, the two sites 17 and 20 or 21 and 28 in the structure represented by Formula (I) are the amino acids Z₂ with the side chain containing -NH₂.

3. The polypeptide or the derivative or pharmaceutically acceptable salt thereof according to claim 1, wherein X₁ is Y or H;
optionally, X₂ is Aib or Ac4c;
optionally, X₃ is Q or H;
optionally, X₆ is F or F(2-F);
optionally, X₆ is F(2-F);
optionally, X₁₀ is Y, C, L, or 4-Pal;
optionally, X₁₀ is Y, L, or 4-Pal;
optionally, X₁₀ is Y or 4-Pal;
optionally, X₁₁ is S or αMeS;
optionally, X₁₂ is K or I;
optionally, X₁₃ is Y, L, A, αMeL, Q, Aib, or Iva;
optionally, X₁₃ is Y, L, αMeL, Q, Aib, or Iva;
optionally, X₁₃ is Y, L, αMeL, Q, or Iva;
optionally, X₁₃ is L or αMeL;
optionally, X₁₃ is Y, L, or A;
optionally, X₁₃ is αMeL, Y, or Iva;
optionally, X₁₃ is αMeL, Q, or L;
optionally, X₁₃ is Y or L;
optionally, X₁₄ is L, K(Ac), HoL, Npg, or Tle;
optionally, X₁₄ is C, L, K(Ac), HoL, Npg, or Tle;
optionally, X₁₅ is D or E;
optionally, X₁₆ is K or E;
optionally, X₁₆ is E, K, αMeK, or K(Ac);
optionally, X₁₆ is K or αMeK;
optionally, X₁₇ is C, Q, K(Ac), R, or I;
optionally, X₁₇ is R, K, K(Ac), Q, or I;
optionally, X₁₇ is K(Ac) or K;
optionally, X₁₇ is C or K(Ac);
optionally, X₁₇ is R, K, or Q;
optionally, X₁₈ is A, R, or Y;
optionally, X₁₈ is A or Y;
optionally, X₁₉ is A, Q, or Aib;
optionally, X₁₉ is Q or A;
optionally, X₁₉ is Q or H;
optionally, X₂₀ is Q, K, Aib, H, a, or C;
optionally, X₂₀ is Aib, Q, H, C, or a;
optionally, X₂₀ is Q, Aib, H, or R;
optionally, X₂₀ is Q, K, or Aib;
optionally, X₂₀ is Aib or Q;
optionally, X₂₀ is Aib or K;
optionally, X₂₁ is C, D, A, L, or Aib;
optionally, X₂₁ is D, A, or C;
optionally, X₂₁ is D, A, or L;
optionally, X₂₁ is K or A;
optionally, X₂₁ is D or C;
optionally, X₂₂ is F or HoF;
optionally, X₂₃ is I or V;
optionally, X₂₄ is Q, C, or E;
optionally, X₂₄ is E, C, or Q;
optionally, X₂₄ is E or C;
optionally, X₂₄ is Q or E;
optionally, X₂₅ is W or Y;
optionally, X₂₈ is A, D, E, C, or HoC;
optionally, X₂₈ is A, D, or E;
optionally, X₂₈ is A or E;
optionally, X₂₈ is E or C;
optionally, X₂₈ is K or A;
optionally, X₃₂ is S or P;
optionally, X₃₅ is A or Q;
optionally, X₃₅ is A or K.

4. The polypeptide or the derivative or pharmaceutically acceptable salt thereof according to any one of claims 1 to 3, wherein the polypeptide or the derivative thereof represented by Formula (I) has a structure represented by Formula (Ia):
X₁X₂X₃GTX₆TSDX₁₀X₁₁X₁₂X₁₃X₁₄X₁₅X₁₆X₁₇X₁₈X₁₉X₂₀X₂₁FX₂₃X₂₄X₂₅LLX₂₈GGPSSGA PPPS (Ia),
optionally, sites 14 and 21, 17 and 24, 21 and 28, or 17 and 20 in the structure represented by Formula (Ia) are the two amino acids Z₁ with the side chain containing -SH;
optionally, sites 21 and 28, or 17 and 20 in the structure represented by Formula (Ia) are the two amino acids Z₂ with the side chain containing -NH₂;
optionally, wherein the polypeptide or the derivative thereof represented by Formula (I) has a structure represented by Formula (Ib):
X₁X₂X₃GTX₆TSDYX₁₁X₁₂X₁₃LDX₁₆X₁₇X₁₈X₁₉X₂₀X₂₁FX₂₃X₂₄X₂₅LLX₂₈GGPSSGAPPPS (Ib),
optionally, sites 17 and 24, or 21 and 28 in the structure represented by Formula (Ib) are the two amino acids Z₁ with the side chain containing -SH.

5. The polypeptide or the derivative or pharmaceutically acceptable salt thereof according to any one of claims 1 to 3, wherein the polypeptide or the derivative thereof represented by Formula (I) has a structure represented by Formula (II):
X₁X₂X₃GTX₆X₇X₈DX₁₀X₁₁X₁₂X₁₃X₁₄X₁₅X₁₆X₁₇X₁₈X₁₉X₂₀X₂₁FX₂₃X₂₄X₂₅LX₂₇X₂₈X₂9 X₃₀PX₃₂SX₃₄X₃₅PPPX₃₉ (II),
wherein: optionally, sites of the following combinations in the structure represented by Formula (II) are the two amino acids Z₁ with the side chain containing -SH:
i and i+3, i being 17;
i and i+4, i being 10;
i and i+7, i being 10, 14, 17, or 21; or
i and i+11, i being 10 or 17;
optionally, wherein the polypeptide or the derivative thereof represented by Formula (I) has a structure represented by Formula (III):
X₁-Aib-X₃GTX₆TSDYSX₁₂X₁₃Z₁X₁₅X₁₆X₁₇X₁₈X₁₉X₂₀Z₁FX₂₃X₂₄X₂₅LLX₂₈X₂₉X₃₀PSSGX ₃₅PPPS (III),
optionally, wherein the polypeptide or the derivative thereof represented by Formula (I) has a structure represented by Formula (IV):
X₁X₂X₃GT-F(2-F)-TSDYX₁₁IX₁₃LDKZ₁AX₁₉Z₁AFIEYLLX₂₈GGPSSGAPPPS (IV),
optionally, wherein the polypeptide or the derivative thereof represented by Formula (II) has a structure represented by Formula (V):
X₁-Aib-X₃GTX₆TSDYSX₁₂X₁₃LX₁₅KCX₁₈X₁₉X₂₀X₂₁FX₂₃CX₂₅LLX₂₈GGPSSGAPPPS (V),
optionally, wherein the polypeptide or the derivative thereof represented by Formula (I) has a structure represented by Formula (VI):
Y-Aib-QGTFTSDYSILLDKZ₁AQH-Aib-FIEYLLZ₁GGPSSGAPPPS (VI),
optionally, the two amino acids Z₁ are each independently selected from C or HoC;
optionally, the two amino acids Z₁ are each C;
optionally, the two amino acids Z₁ are each HoC;
optionally, wherein the polypeptide or the derivative thereof represented by Formula (I) has a structure represented by Formula (VII):
X₁X₂X₃GTX₆TX₈DX₁₀X₁₁X₁₂X₁₃X₁₄X₁₅X₁₆X₁₇X₁₈X₁₉X₂₀Z₁X₂₂X₂₃X₂₄X₂₅LX₂₇Z₁ GGPX₃₂SX₃₄X₃₅PPPS (VII),
wherein:
optionally, the two amino acidsZ₁ are each independently selected from C, c, HoC, or αMeC;
optionally, the two amino acids Z₁ are each C;
optionally, the two amino acids Z₁ are each c;
optionally, the two amino acids Z₁ are each HoC;
optionally, the two amino acids Z₁ are each αMeC.

6. The polypeptide or the derivative or pharmaceutically acceptable salt thereof according to any one of claims 1 to 3, wherein the polypeptide or the derivative thereof represented by Formula (I) has a structure represented by Formula (VIII):
YX₂QGTX₆TSDYSIX₁₃LDX₁₆X₁₇AQX₂₀X₂₁FIEYLLX₂₈GGPSSGAPPPS (VIII),
optionally, sites 17 and 20, or 21 and 28 in the structure represented by Formula (VIII) are the two amino acids Z₂ with the side chain containing -NH₂;
optionally, wherein the polypeptide or the derivative thereof represented by Formula (I) has a structure represented by Formula (IX):
Y-Aib-QGTX₆TSDYSI-αMeL-LDX₁₆-K(Ac)-AQ-Aib-Z₂FIEYLLZ₂GGPSSGAPPPS (IX),
optionally, the two amino acids Z₂ are each independently selected from K or Orn;
optionally, the two amino acids Z₂ are each K;
optionally, the two amino acids Z₂ are each Orn;
optionally, the polypeptide or the derivative thereof represented by Formula (I) has a structure represented by Formula (X):
YX₂QGTX₆TSDYSIX₁₃LDKKAQKAFIEYLLAGGPSSGAPPPS (X).

7. The polypeptide or the derivative or pharmaceutically acceptable salt thereof according to claim 1, wherein the polypeptide or the derivative thereof represented by Formula (I) has at least one of the following structures:
Y-Aib-QGTFTSDYSKYCDKRAAQCFVQWLLAGGPSSGAPPPS;
Y-Aib-QGTFTSDYSKYCDEKRAKCFVQWLLDHHPSSGQPPPS;
Y-Aib-QGTFTSDCSKYCDERAAQDFVQWLLAGGPSSGAPPPS;
Y-Aib-QGTFTSDCSKYLDECAAQDFVQWLLAGGPSSGAPPPS;
Y-Aib-QGTFTSDCSKYLDERAAQCFVQWLLAGGPSSGAPPPS;
H-Aib-HGTFTSDYSIYCEKKYAQCFVQWLLAGGPSSGAPPPS;
H-Aib-HGTFTSDYSIYCEKRYAQCFVQWLLAGGPSSGAPPPS;
H-Aib-HGTFTSDYSIYCEKRYA-Aib-CFVQWLLEGGPSSGAPPPS;
Y-Aib-QGTFTSDYSKYLDKCAAQDFVCWLLAGGPSSGAPPPS;
H-Aib-HGTFTSDYSKYLDKCYAQDFVCWLLEGGPSSGAPPPS;
Y-Aib-QGTFTSDYSILLDKCAQHAFICYLLEGGPSSGAPPPS;
Y-Aib-QGTFTSDYSILLDKCAQ-Aib-AFICYLLEGGPSSGAPPPS;
H-Aib-HGTFTSDYSRALEKCAARLFICWLLEGGPSSGAPPPS;
H-Aib-HGTFTSDYSKYLE-K(Ac)-KYA-Aib-CFVQWLLCGGPSSGAPPPS;
Y-Aib-QGTFTSDYSI-αMeL-LDK-K(Ac)-AQ-Aib-CFIEYLLCGGPSSGAPPPS;
H-Aib-HGTFTSDLSKL-K(Ac)-EEQRQ-Aib-CFIEWLKCGGPPS-Aib-KPPPK;
H-Aib-QGTFTSDLSKQ-K(Ac)-DEQRAKCFIEWLICGGPSSGAPPPS;
H-Aib-QGTFTSDYSI-αMeL-LDK-K(Ac)-AQ-Aib-CFIEYLLCGGPSSGAPPPS; 
(3-iodo-Y)-Aib-QGTFTSDYSI-αMeL-LDK-K(Ac)-AQ-Aib-CFIEYLLCGGPSSGAPPP
S;
(4-Pal)-Aib-QGTFTSDYSI-αMeL-LDK-K(Ac)-AQ-Aib-CFIEYLLCGGPSSGAPPPS; 
(4-amino-F)-Aib-QGTFTSDYSI-αMeL-LDK-K(Ac)-AQ-Aib-CFIEYLLCGGPSSGAPP
PS;
Y-s-QGTFTSDYSI-αMeL-LDK-K(Ac)-AQ-Aib-CFIEYLLCGGPSSGAPPPS;
Y-Aib-QGT-αMeF-TSDYSI-αMeL-LDK-K(Ac)-AQ-Aib-CFIEYLLCGGPSSGAPPPS;
Y-Aib-QGTFT-HoS-DYSI-αMeL-LDK-K(Ac)-AQ-Aib-CFIEYLLCGGPSSGAPPPS;
Y-Aib-QGTFTSDYSI-αMeL-HoL-DK-K(Ac)-AQ-Aib-CFIEYLLCGGPSSGAPPPS;
Y-Aib-QGTFTSDYSI-αMeL-Npg-DK-K(Ac)-AQ-Aib-CFIEYLLCGGPSSGAPPPS;
Y-Aib-QGTFTSDYSI-αMeL-Tle-DK-K(Ac)-AQ-Aib-CFIEYLLCGGPSSGAPPPS;
Y-Aib-QGTFTSDYSI-αMeL-LDK-K(Ac)-AQ-Aib-CFIEWLLCGGPSSGAPPPS;
Y-Aib-QGTFTSDYSILLDK-K(Ac)-AQ-Aib-CFIEWLLCGGPSSGAPPPS;
Y-Aib-QGTFTSDYSI-αMeL-LDKQAQ-Aib-CFIEYLLCGGPSSGAPPPS;
Y-Aib-QGTFTSDYSI-αMeL-LDK-K(Ac)-AQ-a-CFIEYLLCGGPSSGAPPPS;
Y-Aib-QGTFTSDYSI-αMeL-LDK-K(Ac)-AQHCFIEYLLCGGPSSGAPPPS;
Y-Aib-QGTFTSDYSI-αMeL-LDK-K(Ac)-AQQCFIEYLLCGGPSSGAPPPS;
Y-Aib-QGTFTSDYSI-αMeL-LDK-K(Ac)-A-Aib-QCFIEYLLCGGPSSGAPPPS;
Y-Aib-QGTFTSDYSI-αMeL-LDKRAQ-Aib-CFIEYLLCGGPSSGAPPPS;
Y-Aib-QGTFTSDYSI-αMeL-LEK-K(Ac)-AQ-Aib-CFIEYLLCGGPSSGAPPPS;
Y-Aib-QGTFTSDYSI-αMeL-L-Aad-K-K(Ac)-AQ-Aib-CFIEYLLCGGPSSGAPPPS;
Y-Aib-QGTFTSD-4-Pal-SI-αMeL-LDK-K(Ac)-AQ-Aib-CFIEYLLCGGPSSGAPPPS;
Y-Aib-QGTFT-αMeS-DYSI-αMeL-LDK-K(Ac)-AQ-Aib-CFIEYLLCGGPSSGAPPPS;
Y-Aib-QGT-F(2-F)-TSDYSI-αMeL-LDK-K(Ac)-AQ-Aib-CFIEYLLCGGPSSGAPPPS;
Y-Aib-Dab(Ac)-GTFTSDYSI-αMeL-LDK-K(Ac)-AQ-Aib-CFIEYLLCGGPSSGAPPPS ;
Y-Aib-HGTFTSDYSI-αMeL-LDK-K(Ac)-AQ-Aib-CFIEYLLCGGPSSGAPPPS;
Y-Ac4c-QGTFTSDYSI-αMeL-LDK-K(Ac)-AQ-Aib-CFIEYLLCGGPSSGAPPPS;
Y-Aib-QGTFTSDYSILLDKCAQH-Aib-FIEYLLCGGPSSGAPPPS;
Y-Aib-QGTFTSDYSILLDK-HoC-AQH-Aib-FIEYLL-HoC-GGPSSGAPPPS;
Y-Aib-QGT-F(2-F)-TSDYSILLDKCAQQAFICYLLAGGPSSGAPPPS;
Y-Aib-QGT-F(2-F)-TSDYSILLDKQAQQCFIEYLLCGGPSSGAPPPS;
Y-Aib-QGT-F(2-F)-TSDYSI-Aib-LDK-K(Ac)-AQQCFIEYLLCGGPSSGAPPPS;
Y-Aib-QGT-F(2-F)-TSDYSILCDKQAQQCFIEYLLAGGPSSGAPPPS;
Y-Aib-QGT-F(2-F)-TSDYSILLD-αMeK-K(Ac)-AQQCFIEYLLCGGPSSGAPPPS;
Y-Aib-QGT-F(2-F)-TSDYSILLD-αMeK-K(Ac)-AQ-Aib-CFIEYLICGGPSSGAPPPS;
Y-Aib-QGT-F(2-F)-TSDYSI-Iva-LDK-K(Ac)-AQ-Aib-CFIEYLLCGGPSSGAPPPS;
Y-Aib-QGT-F(2-F)-TSDY-αMeS-ILLD-αMeK-K(Ac)-AQQCFIEYLLCGGPPSSGAPPS ; 
Y-Aib-QGT-F(2-F)-TSDYSI-αMeL-LD-αMeK-K(Ac)-AQQCFIEYLLCGGPSSGAPPP
S;
 
Y-Aib-QGT-F(2-F)-TSDYSI-αMeL-LD-αMeK-K(Ac)-AQQC-HoF-IEYLLCGGPSSGA
PPPS;
Y-Aib-QGT-F(2-F)-TSDYSI-αMeL-LDKCAQCAFIEYLLAGGPSSGAPPPS;
Y-Aib-QGT-F(2-F)-TSDYSI-Iva-LDKCAQCAFIEYLLAGGPSSGAPPPS; 
Y-Aib-QGT-F(2-F)-TSDY-αMeS-I-αMeL-LD-αMeK-K(Ac)-AQ-Aib-CFIEYLLCGGPS
SGAPPPS;
 
Y-Aib-QGT-F(2-F)-TSDY-αMeS-I-αMeL-LD-αMeK-CAQCAFIEYLLAGGPSSGAPPP
S;
H-Aib-HGT-F(2-F)-TSDYSIYLE-K(Ac)-KYA-Aib-CFVQWLLCGGPSSGAPPPS;
H-Aib-HGT-F(2-F)-TSDYSIQ-K(Ac)-EEIAQ-Aib-CFIEWLLCGGPSSGAPPPS;
H-Aib-HGT-F(2-F)-TSDYSIY-K(Ac)-EERAQ-Aib-CFIEWLLCGGPSSGAPPPS;
H-Aib-HGT-F(2-F)-TSDYSI-Iva-LDK-K(Ac)-AQ-Aib-CFIEYLLCGGPSSGAPPPS;
Y-Aib-QGT-F(2-F)-TSDYSI-αMeL-LDKCAHCAFIEYLLAGGPSSGAPPPS;
Y-Aib-QGT-F(2-F)-TSDYSI-αMeL-LDKCA-K(Ac)-CAFIEYLLAGGPSSGAPPPS;
Y-Aib-QGT-F(2-F)-TSDYSI-αMeL-LDKCAQCAFIEYLLAGGPSSGAPPPS;
H-Aib-HGT-F(2-F)-TSDYSI-αMeL-LDKCAQCAFIEYLLAGGPSSGAPPPS;
Y-s-QGT-F(2-F)-TSDYSI-αMeL-LDKCAQCAFIEYLLAGGPSSGAPPPS;
Y-Aib-QGTFTSDYSILLDKQAQQCFIEYLLCGGPSSGAPPPS;
Y-Ac4c-QGT-F(2-F)-TSDYSILLDKCAQCAFIEYLLEGGPSSGAPPPS;
Y-Ac4c-QGT-F(2-F)-TSDYSILLDK-K(Ac)-AQ-Aib-CFIEYLLCGGPSSGAPPPS;
H-Ac4c-HGT-F(2-F)-TSDYSIQLEEIAQ-Aib-CFIEYLLCGGPSSGAPPPS;
Y-Aib-QGTFTSDYSILLDK-K(Ac)-AQ-Aib-c-FIEYLL-c-GGPSSGAPPPS;
Y-Aib-QGTFTSDYSILLDK-K(Ac)-AQ-Aib-Hoc-FIEYLL-Hoc-GGPSSGAPPPS;
Y-Aib-QGTFTSDYSILLDK-K(Ac)-AQ-Aib-αMeC-FIEYLL-αMeC-GGPSSGAPPPS;
Y-Aib-QGT-F(2-F)-TSDYSIQLDK-K(Ac)-AQ-Aib-CFIEYLLCGGPSSGAPPPS; 
Y-Aib-QGT-F(2-F)-TSDYSI-αMeL-LD-αMeK-K(Ac)-AQ-Aib-KFIEYLLKGGPSSGAP
PPS;
Y-Aib-QGT-F(2-F)-TSDYSI-αMeL-LDK-K(Ac)-AQ-Aib-KFIEYLLKGGPSSGAPPPS;
Y-Aib-QGTFTSDYSILLDK-K(Ac)-AQ-Aib-Orn-FIEYLL-Orn-GGPSSGAPPPS;
Y-Aib-QGT-F(2-F)-TSDYSI-αMeL-LDKKAQKAFIEYLLAGGPSSGAPPPS;
Y-Aib-QGT-F(2-F)-TSDYSIQLDKKAQKAFIEYLLAGGPSSGAPPPS;
Y-Aib-QGTFTSDYSILLDKKAQKAFIEYLLAGGPSSGAPPPS; or
Y-Aib-QGT-F(2-F)-TSDYSI-αMeL-LDKKAQKAFIEYLLAGGPSSGAPPPS.

8. The polypeptide or the derivative or pharmaceutically acceptable salt thereof according to claim 1, further comprising a modification group,
optionally, the modification group is bound to one or both of the two amino acids Z₁ with the side chain containing -SH or bound to one or both of the two amino acids Z₂ with the side chain containing -NH₂ containing -NH₂ in the polypeptide or the derivative thereof represented by Formula (I);
optionally, the modification group is bound to both of the two amino acids Z₁ with the side chain containing -SH in the polypeptide or the derivative thereof represented by Formula (I), or the modification group is bound to both of the two amino acids Z₂ with the side chain containing -NH₂ in the polypeptide or the derivative thereof represented by Formula (I);
optionally, the modification group is bound to -SH of the side chain of amino acid C via a sulfur-carbon bond, or the modification group is bound to ε-amino group of the side chain of amino acid K via an amide bond.

9. The polypeptide or the derivative or pharmaceutically acceptable salt thereof according to claim 8, wherein the modification group has a structure represented by Formula (XI): 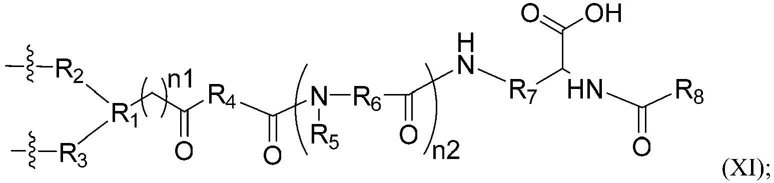
wherein R₁ is C, N, -C₃₋₁₀ heteroalkylene, -C₆₋₁₀ arylene, or -C₅₋₁₀ heteroarylene;
R₂ and R₃ are each independently -C₁₋₆ alkylene- optionally substituted with one or more R₁ₐ, -NH-C(O)-C₁₋₆ alkylene- optionally substituted with one or more R₁ₐ, or -C₁₋₆ alkylene-NH-C(O)-C₁₋₆ alkylene- optionally substituted with one or more R₁ₐ, wherein R₁ₐ is each independently halogen, -OH, -SH, -NH₂, -NO₂, -CN, phenyl, -C₁₋₆ alkyl, -C₁₋₆ haloalkyl, or -C₁₋₆ alkoxy;
R₄ is absent, or -C₁₋₆ alkylene-NH-C(O)-C₁₋₆ alkylene- optionally substituted with one or more R₂ₐ, wherein R₂ₐ is each independently halogen, -OH, -SH, -NH₂, -NO₂, -CN, phenyl, -C₁₋₆ alkyl, -C₁₋₆ haloalkyl, or -C₁₋₆ alkoxy;
R₅ is H, -C₁₋₆ alkoxy, or -C₁₋₆ alkyl, wherein the -C₁₋₆ alkyl and -C₁₋₆ alkoxy are each independently optionally substituted with one or more halogen, -OH, -C(O)OH, -C(O)-, -SH, -NH₂, -NO₂, or -CN;
R₆ is -C₁₋₆ alkylene- optionally substituted with one or more R₃ₐ, or -(C₁₋₃ alkylene -O)ₘ₁-C₁₋₆ alkylene- optionally substituted with one or more R₃ₐ, wherein R₃ₐ is each independently halogen, -OH, -SH, -NH₂, -NO₂, -CN, phenyl, -C₁₋₆ alkyl, -C₁₋₆ haloalkyl, or -C₁₋₆ alkoxy;
R₇ is -C₁₋₆ alkylene- optionally substituted with one or more R₄ₐ, wherein R₄ₐ is each independently halogen, -OH, -SH, -NH₂, -NO₂, -CN, phenyl, -C₁₋₆ alkyl, -C₁₋₆ haloalkyl, or -C₁₋₆ alkoxy;
R₈ is -C₁₀₋₂₀ alkyl optionally substituted with one or more R₅ₐ, -C₁₀₋₂₀ alkylene-R₉ optionally substituted with one or more R₅ₐ, -C₅₋₁₀ alkylene-O-C₆₋₁₀ arylene-R₉ optionally substituted with one or more R₅ₐ, or -C₅₋₁₀ alkylene-O-C₅₋₁₀ heteroarylene-R₉ optionally substituted with one or more R₅ₐ, wherein R₅ₐ is each independently halogen, -OH, -SH, -NH₂, -NO₂, -CN, phenyl, -C₁₋₆ alkyl, -C₁₋₆ haloalkyl, or -C₁₋₆ alkoxy;
R₉ is -COOH, -C₃₋₇ heteroaryl, -S(O)₂OH, or -PO(OH)₂;
m1 is any integer from 1 to 6;
n1 is any integer from 0 to 6; and
n2 is any integer from 1 to 10.

10. The polypeptide or the derivative or pharmaceutically acceptable salt thereof according to claim 9, wherein R₁ is C, N, -C₅₋₇ heteroalkylene, -C₅₋₇ arylene, or -C₅₋₇ heteroarylene;
optionally, R₁ is N, phenylene, or pyridinylene;
optionally, R₁ is 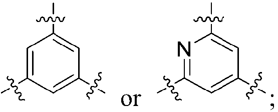
optionally, R₂ and R₃ are each independently -C₁₋₆ alkylene-, or -C₁₋₆ alkylene-NH-C(O)-C₁₋₆ alkylene-;
optionally, R₂ and R₃ are each independently -C₁₋₃ alkylene-, or -C₁₋₃ alkylene-NH-C(O)-C₁₋₃ alkylene-;
optionally, n1 is each independently 0, 1, 2, 3, 4, or 5;
optionally, n1 is 0;
optionally, R₄ is absent, or -C₁₋₆ alkylene-NH-C(O)-C₁₋₆ alkylene-;
optionally, R₄ is absent, or -C₁₋₃ alkylene-NH-C(O)-C₁₋₃ alkylene-;
optionally, R₅ is H, -C₁₋₃ alkoxy, or -C₁₋₃ alkyl;
optionally, R₆ is -C₁₋₆ alkylene-, -(C₁₋₃ alkylene-O)ₘ₁-C₁₋₆ alkylene-;
optionally, R₆ is -C₁₋₃ alkylene-, -(C₁₋₃ alkylene-O)ₘ₁-C₁₋₃ alkylene-;
optionally, m1 is 2, 3, 4, or 5;
optionally, R₇ is -C₁₋₆ alkylene-;
optionally, R₇ is -C₂₋₄ alkylene-;
optionally, R₈ is -C₁₀₋₂₀ alkyl, -C₁₀₋₂₀ alkylene-R₉, -C₅₋₁₀ alkylene-O-C₆₋₁₀ arylene-R₉, or -C₅₋₁₀ alkylene-O-C₅₋₁₀ heteroarylene-R₉;
optionally, R₈ is -C₁₀₋₁₈ alkyl, -C₁₄₋₁₈ alkylene-R₉, or -C₇₋₉ alkylene-O-C₅₋₇ arylene-COOH;
optionally, R₉ is -COOH, -C₅₋₆ heteroaryl, -S(O)₂OH, or -PO(OH)₂;
optionally, R₉ is -COOH, -S(O)₂OH, -PO(OH)₂, or 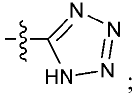
optionally, n2 is 1, 2, or 3;
optionally, n2 is 3, 4, 5, or 6;
optionally, the modification group represented by Formula (XI) has a structure represented by Formula (XIa): 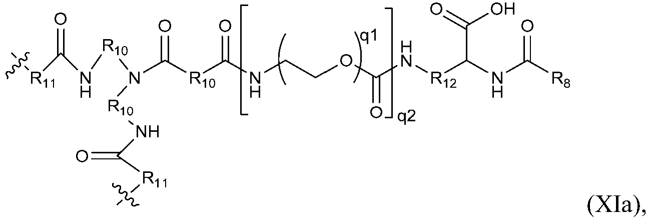
where R₁₀ and R₁₁ are each independently -C₀₋₃ alkylene-;
R₁₂ is -C₁₋₆ alkylene-;
R₈ is -C₁₀₋₂₁ alkyl, or -C₁₀₋₂₁ alkylene-COOH, preferably -C₁₄₋₁₉ alkyl or -C₁₀₋₁₈ alkylene-COOH;
q1 is 1, 2, 3, or 4; and
q2 is 1, 2, or 3;
optionally, the modification group represented by Formula (XIa) has a structure as shown below: 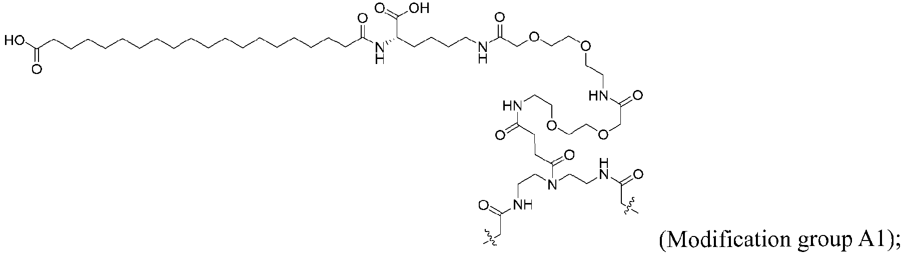 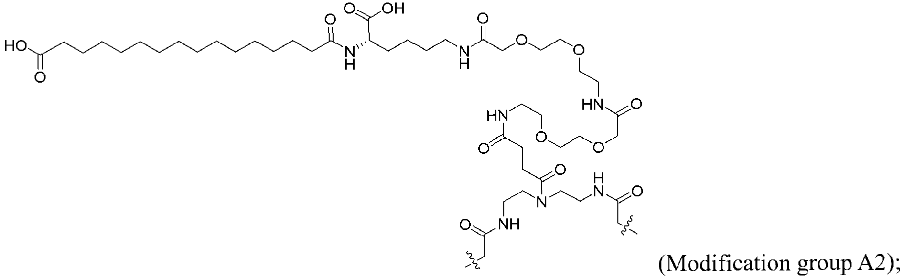 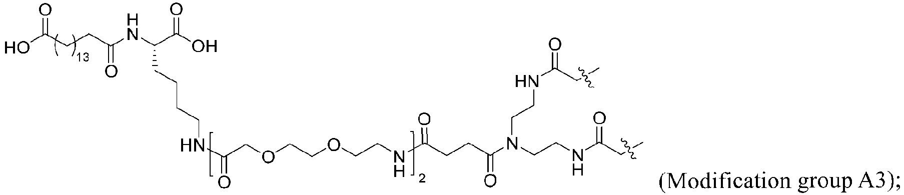 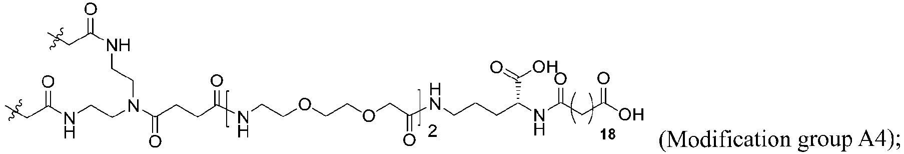 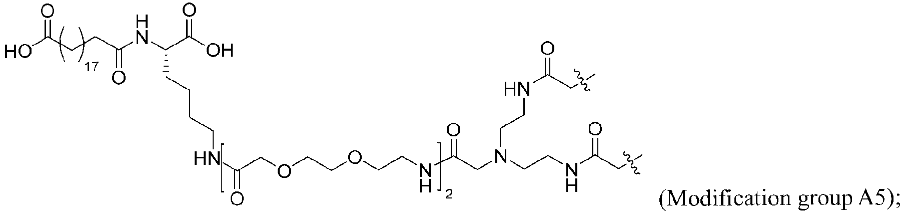 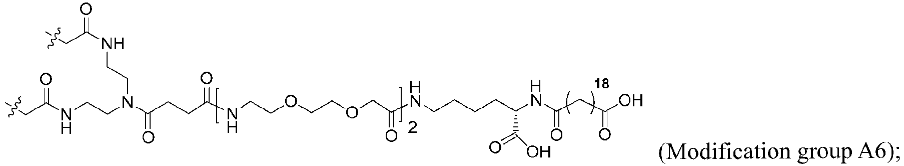 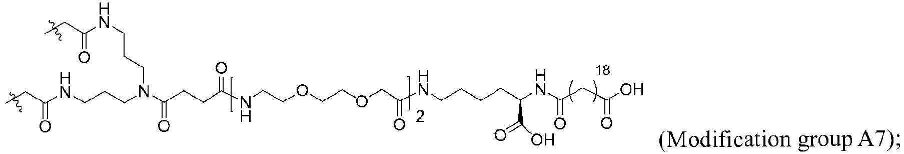 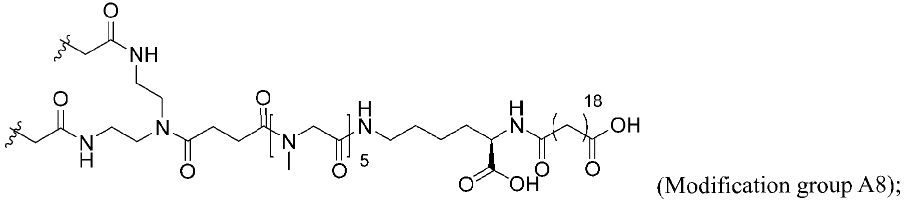 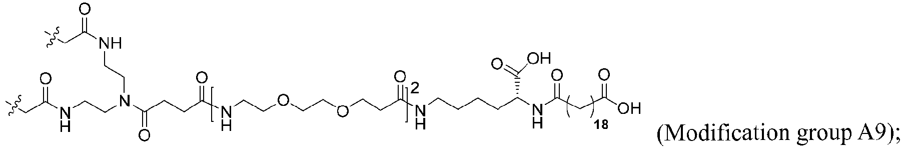 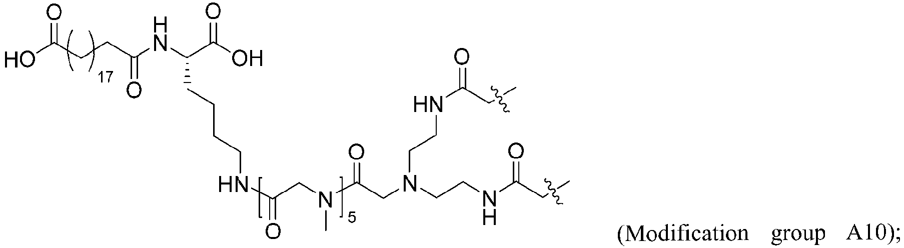 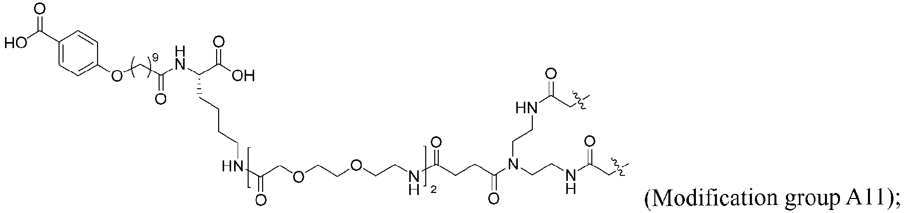 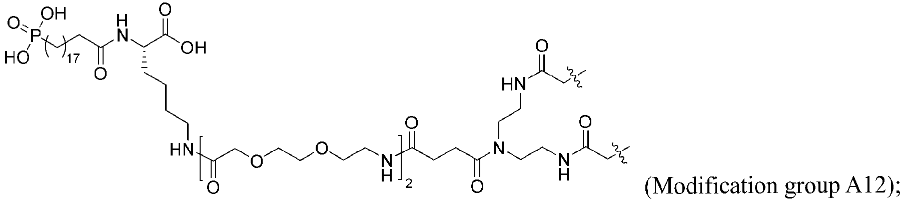 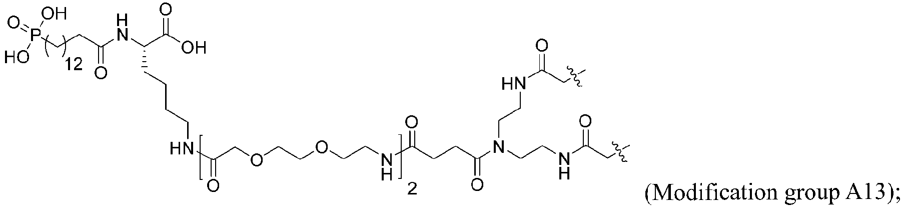 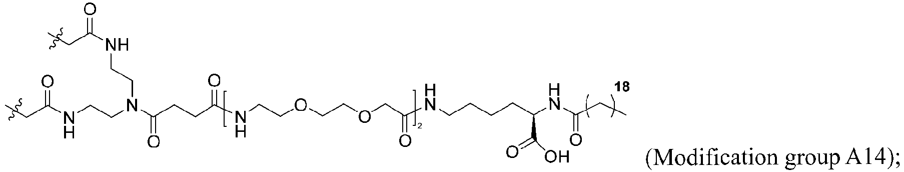 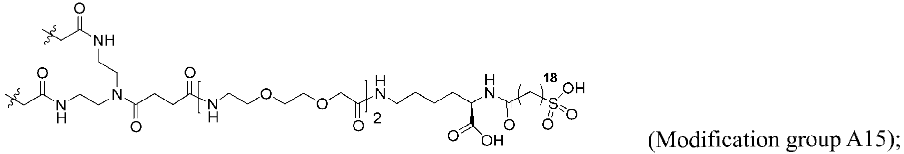 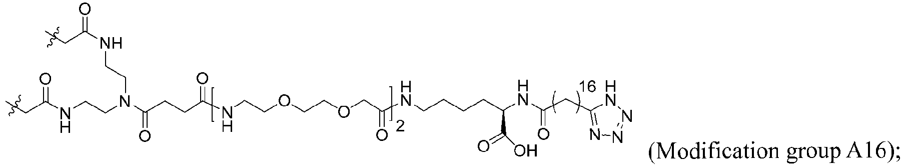 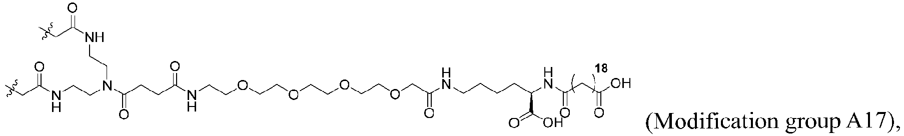
optionally, the modification group represented by Formula (XI) has a structure represented by Formula (XIb): 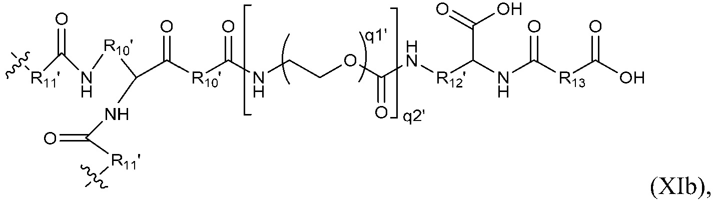
where R₁₀' and R₁₁' are each independently -C₀₋₃ alkylene-;
R₁₂' is -C₁₋₆ alkylene-;
R₁₃ is -C₁₀₋₂₀ alkylene-, and preferably -C₁₄₋₁₈ alkylene-;
q1' is 1, 2, 3, or 4; and
q2' is 1, 2, or 3;
optionally, the modification group represented by Formula (XIb) has a structure as shown below: 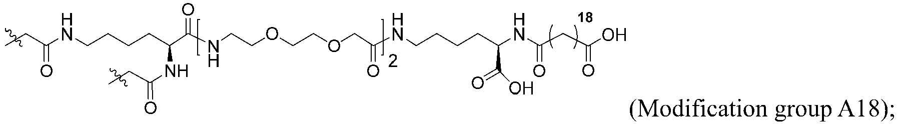 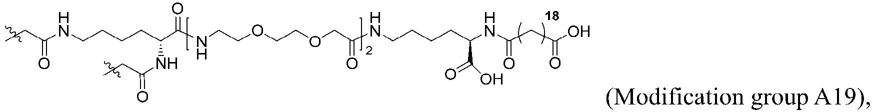
optionally, the modification group represented by Formula (XI) has a structure represented by Formula (XIc): 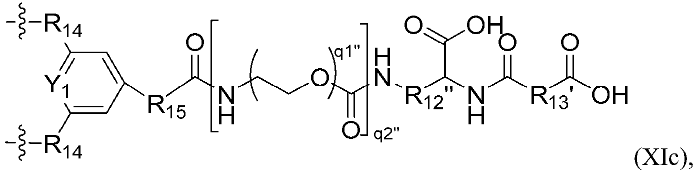
where R₁₄ is each independently -C₁₋₃ alkylene- or -NH-C(O)-C₁₋₃ alkylene-;
R₁₅ is -C₀₋₃ alkylene- or -C(O)-NH-C₁₋₃ alkylene-;
R₁₂" is -C₁₋₆ alkylene-;
R₁₃' is -C₁₀₋₂₀ alkylene-, and preferably -C₁₄₋₁₈ alkylene-;
q1" is 1, 2, 3, or 4;
q2" is 1, 2, or 3; and
Y₁ is C or N;
optionally, the modification group represented by Formula (XIc) has a structure as shown below: 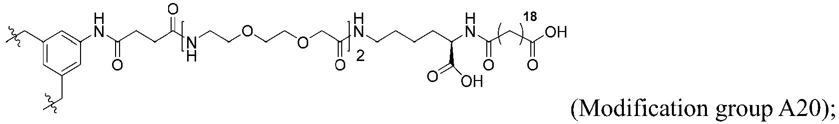 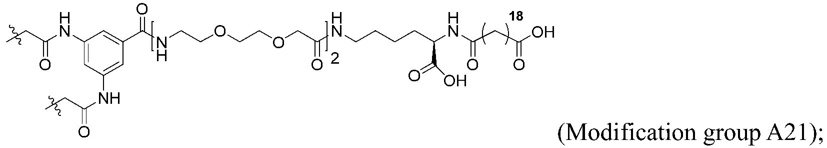 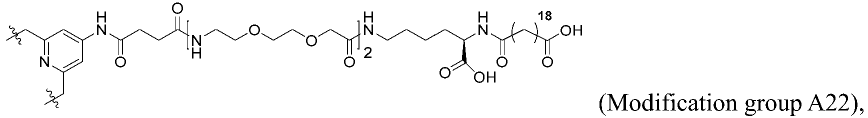
optionally, the polypeptide or the derivative thereof represented by Formula (I) has two amino acids Z₁ with the side chain containing -SH, and the modification group has a structure represented by Formula (XI).

11. The polypeptide or the derivative or pharmaceutically acceptable salt thereof according to claim 8, wherein the modification group has a structure represented by Formula (XII): 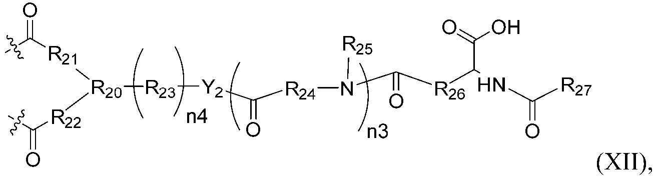
where R₂₀ is C, N, -C₆₋₁₀ arylene, or -C₅₋₁₀ heteroarylene;
R₂₁ and R₂₂ are each independently absent, or -C₁₋₆ alkylene- optionally substituted with one or more R_{1b}, or -C₁₋₆ oxyalkylene- optionally substituted with one or more R_{1b}, wherein R_{1b} is each independently halogen, -OH, -SH, -NH₂, -NO₂, -CN, phenyl, -C₁₋₆ alkyl, -C₁₋₆ haloalkyl, or -C₁₋₆ alkoxy;
R₂₃ is absent, or -C₁₋₆ alkylene- optionally substituted with one or more R_{2b}, -C(O)-C₁₋₆ alkylene- optionally substituted with one or more R_{2b}, -NH-C(O)-C₁₋₆ alkylene- optionally substituted with one or more R_{2b}, or -C₁₋₆ alkylene-C(O)-NH-C₁₋₆ alkylene- optionally substituted with one or more R_{2b}, wherein R_{2b} is each independently halogen, -OH, -SH, -NH₂, -NO₂, -CN, phenyl, -C₁₋₆ alkyl, -C₁₋₆ haloalkyl, or -C₁₋₆ alkoxy;
R₂₄ is -C₁₋₆ alkylene- optionally substituted with one or more R_{3b}, or -(C₁₋₃ alkylene-O)ₘ₂-C₁₋₆ alkylene- optionally substituted with one or more R_{3b}, wherein R_{3b} is each independently halogen, -OH, -SH, -NH₂, -NO₂, -CN, phenyl, -C₁₋₆ alkyl, -C₁₋₆ haloalkyl, or -C₁₋₆ alkoxy;
R₂₅ is H, -C₁₋₆ alkoxy, or -C₁₋₆ alkyl, wherein the -C₁₋₆ alkyl and -C₁₋₆ alkoxy are each independently optionally substituted with one or more halogen, -OH, -C(O)OH, -C(O)-, -SH, -NH₂, -NO₂, or -CN;
R₂₆ is -C₁₋₆ alkylene- optionally substituted with one or more R_{4b}, wherein R_{4b} is each independently halogen, -OH, -SH, -NH₂, -NO₂, -CN, phenyl, -C₁₋₆ alkyl, -C₁₋₆ haloalkyl, or -C₁₋₆ alkoxy;
R₂₇ is -C₁₀₋₂₀ alkyl optionally substituted with one or more R_{5b}, -C₁₀₋₂₀ alkylene-R₂₈ optionally substituted with one or more R_{5b}, -C₅₋₁₀ alkylene-O-C₆₋₁₀ arylene-R₂₈ optionally substituted with one or more R_{5b}, or -C₅₋₁₀ alkylene-O-C₅₋₁₀ heteroarylene-R₂₈ optionally substituted with one or more R_{5b}, wherein R_{5b} is each independently halogen, -OH, -SH, -NH₂, -NO₂, -CN, phenyl, -C₁₋₆ alkyl, -C₁₋₆ haloalkyl, or -C₁₋₆ alkoxy;
R₂₈ is -COOH, -C₃₋₇ heteroaryl, -S(O)₂OH, or -PO(OH)₂;
m2 is any integer from 1 to 6;
n3 is any integer from 1 to 10;
n3 is 0, 1, or 2; and
Y₂ is absent or NH.

12. The polypeptide or the derivative or pharmaceutically acceptable salt thereof according to claim 11, wherein R₂₀ is C, N, -C₅₋₇ arylene or -C₅₋₇ heteroarylene;
optionally, R₂₀ is C, N, phenylene, or pyridinylene;
optionally, R₂₀ is 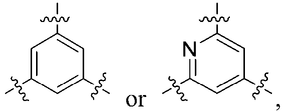
preferably 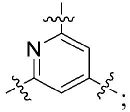
optionally, R₂₁ and R₂₂ are each independently absent, or -C₁₋₆ alkylene-, or -C₁₋₆ oxyalkylene-;
optionally, R₂₁ and R₂₂ are each independently absent or -C₁₋₃ alkylene-;
optionally, R₂₃ is absent, -C₁₋₆ alkylene-, -C₁₋₆ alkylene-C(O)-NH-C₁₋₆ alkylene-, or -C(O)-C₁₋₆ alkylene-;
optionally, R₂₃ is absent, -C₁₋₃ alkylene-, or -C(O)-C₂₋₄ alkylene-;
optionally, R₂₄ is -C₁₋₆ alkylene-, or -(C₁₋₃ alkylene-O)ₘ₂-C₁₋₆ alkylene-;
optionally, R₂₄ is -C₁₋₃ alkylene-, or -(C₁₋₃ alkylene-O)ₘ₂-C₁₋₃ alkylene-;
optionally, m2 is 2, 3, 4, or 5;
optionally, R₂₅ is H, -C₁₋₆ alkoxy, or -C₁₋₆ alkyl;
optionally, R₂₅ is H or -C₁₋₃ alkyl;
optionally, R₂₆ is -C₁₋₆ alkylene-;
optionally, R₂₆ is -C₁₋₃ alkylene-;
optionally, R₂₇ is -C₁₀₋₂₀ alkyl, -C₁₀₋₂₀ alkylene-R₂₈, -C₅₋₁₀ alkylene-O-C₆₋₁₀ arylene-R₂₈, or -C₅₋₁₀ alkylene-O-C₅₋₁₀ heteroarylene-R₂₈;
optionally, R₂₇ is -C₁₀₋₁₈ alkyl, -C₁₄₋₁₈ alkylene-R₂₈, or -C₇₋₉ alkylene-O-C₅₋₇ arylene-COOH;
optionally, R₂₈ is -COOH, -C₅₋₆ heteroaryl, -S(O)₂OH, or -PO(OH)₂;
optionally, R₂₈ is -COOH, -S(O)₂OH, -PO(OH)₂, or 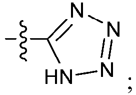
optionally, p is 1, 2, or 3;
optionally, p is 3, 4, 5, or 6;
optionally, the modification group represented by Formula (XII) has a structure represented by Formula (XIIa): 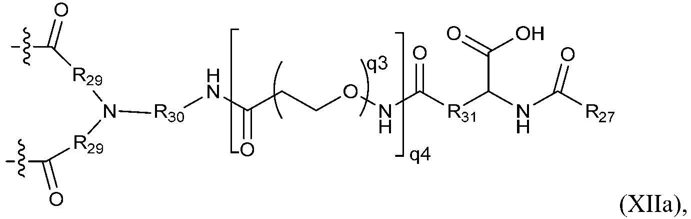
where R₂₉ is each independently -C₁₋₃ alkylene-;
R₃₀ is -C(O)-C₁₋₆ alkylene-, or -C₁₋₃ alkylene-C(O)-NH-C₁₋₃ alkylene-;
R₃₁ is -C₁₋₆ alkylene-;
q3 is 1, 2, 3, or 4; and
q4 is 1, 2, or 3;
optionally, R₂₇ is -C₁₀₋₂₀ alkylene-COOH or -C₁₀₋₂₀ alkyl, preferably -C1₄₋₁₈ alkylene-COOH or -C₁₀₋₁₉ alkyl;
optionally, wherein the modification group represented by Formula (XIIa) has a structure as shown below: 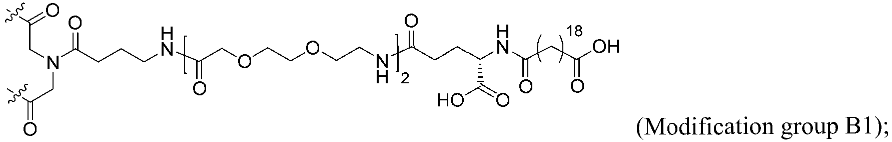 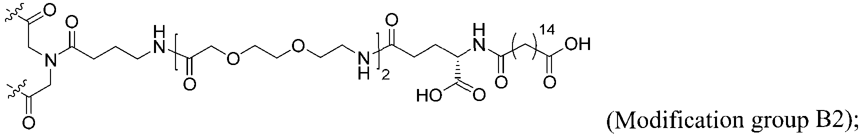 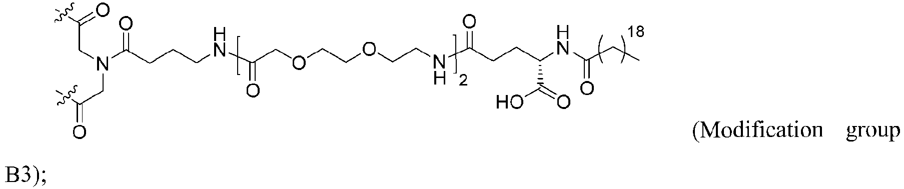 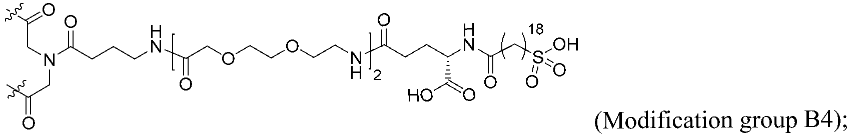 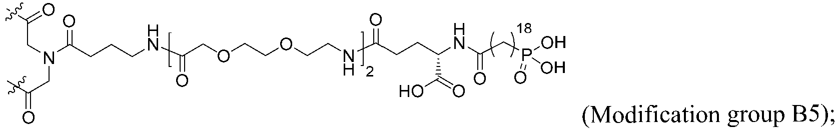 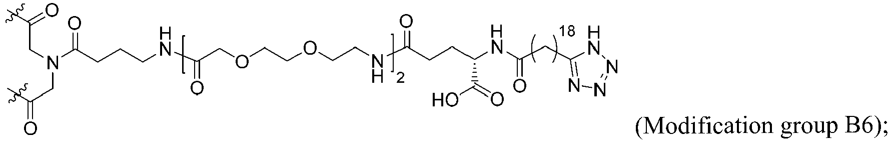 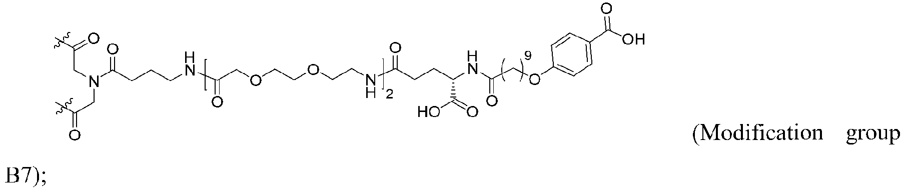 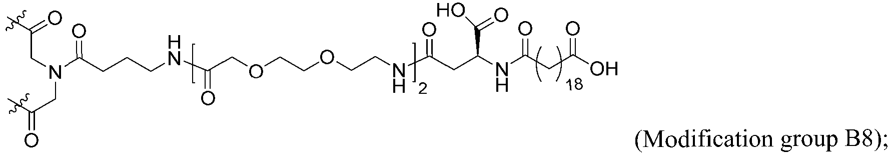 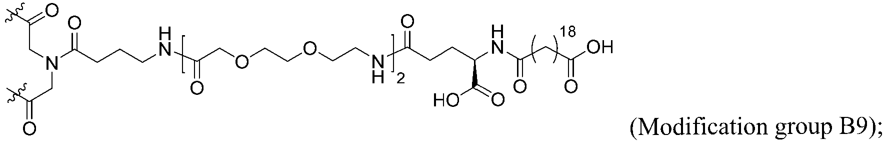 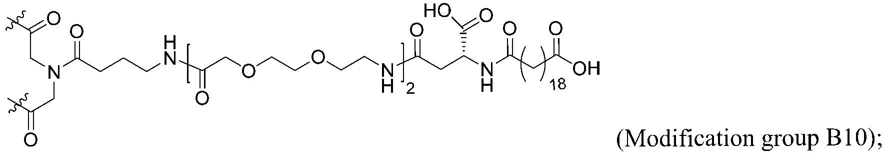 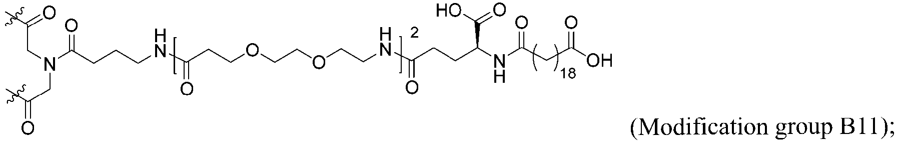 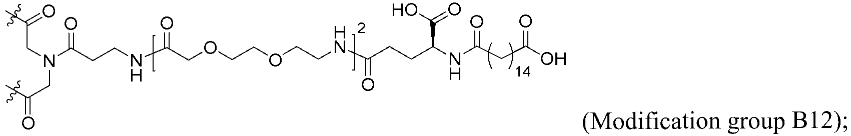 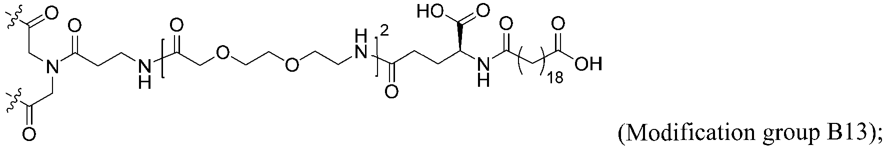 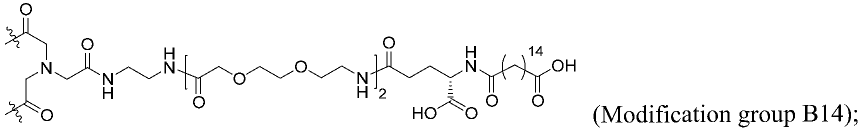 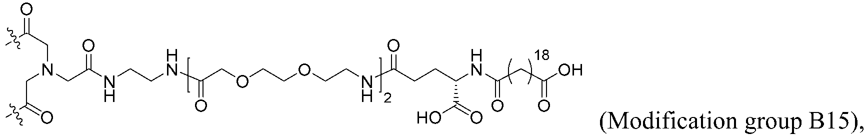
optionally, wherein the modification group represented by Formula (XII) has a structure represented by Formula (XIIb): 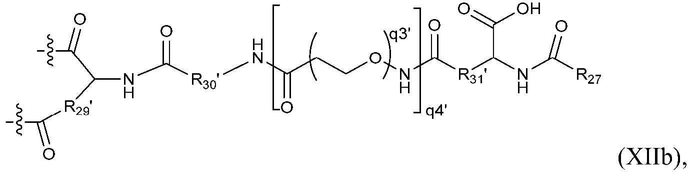
where R₂₉' is each independently -C₁₋₃ alkylene-;
R₃₀' is -C₁₆ alkylene-;
R₃₁' is -C₁₋₆ alkylene-;
q3' is 1, 2, 3, or 4, and preferably 2; and
q4' is 1, 2, or 3, and preferably 2;
optionally, R₂₇ is -C₁₀₋₂₀ alkylene-COOH or -C₁₀₋₂₀ alkyl, and preferably -C₁₄₋₁₈ alkylene-COOH;
optionally, wherein the modification group represented by Formula (XIIb) has a structure as shown below: 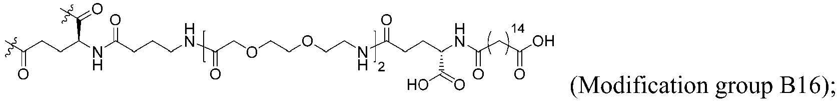 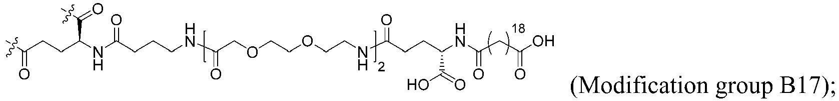 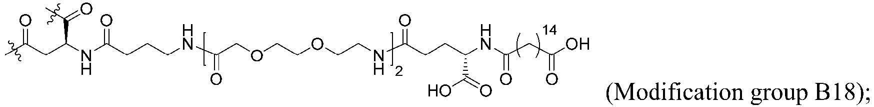 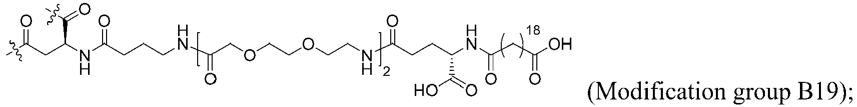 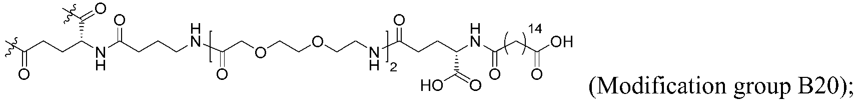 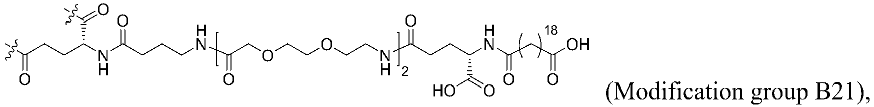
optionally, wherein the modification group represented by Formula (XII) has a structure represented by Formula (XIIc): 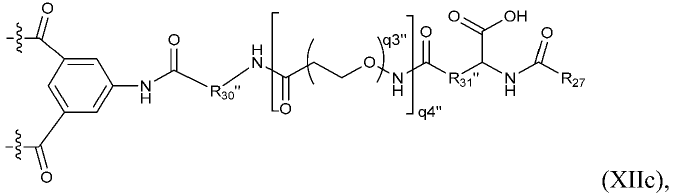
where R₃₀" is -C₁₋₆ alkylene-;
R₃₁" is -C₁₋₆ alkylene-;
q3 is 1, 2, 3, or 4; and
q4 is 1, 2, or 3;
optionally, R₂₇ is -C₁₀₋₂₀ alkylene-COOH or -C₁₀₋₂₀ alkyl, preferably -C1₄₋₁₈ alkylene-COOH or -C₁₀₋₁₉ alkyl;
optionally, the modification group represented by Formula (XIIc) has a structure as shown below: 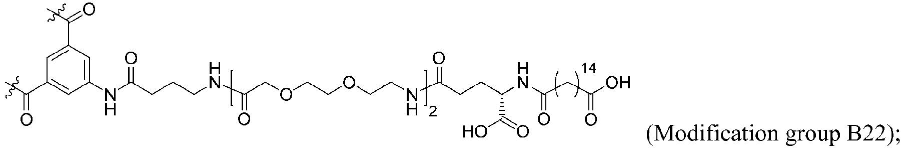 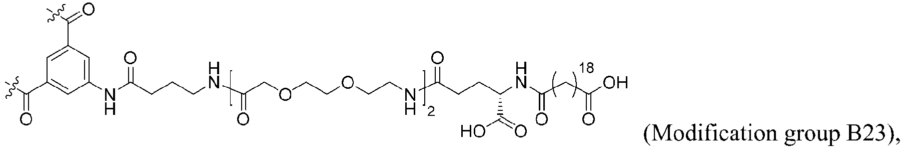
optionally, wherein the polypeptide or the derivative thereof represented by Formula (I) has two amino acids Z₂ with the side chain containing -NH₂, and the modification group has a structure represented by Formula (XII).

13. A polypeptide derivative or a pharmaceutically acceptable salt thereof, wherein the polypeptide derivative has one of the structures shown in Table A:
**Table A**
| Name of Polypeptide | Amino acid sequence of polypeptide | Sites where modification groups are bound | Modification group |
|---|---|---|---|
| TG1 | Y-Aib-QGTFTSDYSKYCDKRAAQCFVQWLLAGGPSSGAPPPS | 14, 21 | A1 |
| TG2 | Y-Aib-QGTFTSDYSKYCDEKRAKCFVQWLLDHHPSSGQPPPS | 14, 21 | A1 |
| TG3 | Y-Aib-QGTFTSDCSKYCDERAAQDFVQWLLAGGPSSGAPPPS | 10, 14 | A1 |
| TG4 | Y-Aib-QGTFTSDCSKYLDECAAQDFVQWLLAGGPSSGAPPPS | 10, 17 | A1 |
| TG5 | Y-Aib-QGTFTSDCSKYLDERAAQCFVQWLLAGGPSSGAPPPS | 10, 21 | A1 |
| TG6 | H-Aib-HGTFTSDYSIYCEKKYAQCFVQWLLAGGPSSGAPPPS | 14, 21 | A1 |
| TG7 | H-Aib-HGTFTSDYSIYCEKRYAQCFVQWLLAGGPSSGAPPPS | 14, 21 | A1 |
| TG8 | H-Aib-HGTFTSDYSIYCEKRYA-Aib-CFVQWLLEGGPSSGAPPPS | 14, 21 | A1 |
| TG9 | Y-Aib-QGTFTSDYSKYLDKCAAQDFVCWLLAGGPSSGAPPPS | 17, 24 | A1 |
| TG10 | H-Aib-HGTFTSDYSKYLDKCYAQDFVCWLLEGGPSSGAPPPS | 17, 24 | A1 |
| TG11 | Y-Aib-QGTFTSDYSILLDKCAQHAFICYLLEGGPSSGAPPPS | 17, 24 | A1 |
| TG12 | Y-Aib-QGTFTSDYSILLDKCAQ-Aib-AFICYLLEGGPSSGAPPPS | 17, 24 | A1 |
| TG13 | H-Aib-HGTFTSDYSRALEKCAARLFICWLLEGGPSSGAPPPS | 17, 24 | A1 |
| TG14 | H-Aib-HGTFTSDYSKYLE-K(Ac)-KYA-Aib-CFVQWLLCGGPSSGA PPPS | 21, 28 | A1 |
| TG15 | Y-Aib-QGTFTSDYSI-αMeL-LDK-K(Ac)-AQ-Aib-CFIEYLLCGGPSS GAPPPS | 21, 28 | A1 |
| TG16 | H-Aib-HGTFTSDLSKL-K(Ac)-EEQRQ-Aib-CFIEWLKCGGPPS-Aib- KPPPK | 21, 28 | A1 |
| TG17 | H-Aib-QGTFTSDLSKQ-K(Ac)-DEQRAKCFIEWLICGGPSSGAPPPS | 21, 28 | A1 |
| TG18 | H-Aib-QGTFTSDYSI-αMeL-LDK-K(Ac)-AQ-Aib-CFIEYLLCGGPSS GAPPPS | 21, 28 | A1 |
| TG19 | (3-iodo-Y)-Aib-QGTFTSDYSI-αMeL-LDK-K(Ac)-AQ-Aib-CFIEYLL CGGPSSGAPPPS | 21, 28 | A1 |
| TG20 | (4-Pal)-Aib-QGTFTSDYSI-αMeL-LDK-K(Ac)-AQ-Aib-CFIEYLLCG GPSSGAPPPS | 21, 28 | A1 |
| TG21 | (4-amino-F)-Aib-QGTFTSDYSI-αMeL-LDK-K(Ac)-AQ-Aib-CFIEYL LCGGPSSGAPPPS | 21, 28 | A1 |
| TG22 | Y-s-QGTFTSDYSI-αMeL-LDK-K(Ac)-AQ-Aib-CFIEYLLCGGPSSG APPPS | 21, 28 | A1 |
| TG23 | Y-Aib-QGT-αMeF-TSDYSI-αMeL-LDK-K(Ac)-AQ-Aib-CFIEYLLCG GPSSGAPPPS | 21, 28 | A1 |
| TG24 | Y-Aib-QGTFT-HoS-DYSI-αMeL-LDK-K(Ac)-AQ-Aib-CFIEYLLCGG PSSGAPPPS | 21, 28 | A1 |
| TG25 | Y-Aib-QGTFTSDYSI-αMeL-HoL-DK-K(Ac)-AQ-Aib-CFIEYLLCGG PSSGAPPPS | 21, 28 | A1 |
| TG26 | Y-Aib-QGTFTSDYSI-αMeL-Npg-DK-K(Ac)-AQ-Aib-CFIEYLLCGG PSSGAPPPS | 21, 28 | A1 |
| TG27 | Y-Aib-QGTFTSDYSI-αMeL-Tle-DK-K(Ac)-AQ-Aib-CFIEYLLCGGP SSGAPPPS | 21, 28 | A1 |
| TG28 | Y-Aib-QGTFTSDYSI-αMeL-LDK-K(Ac)-AQ-Aib-CFIEWLLCGGPSS GAPPPS | 21, 28 | A1 |
| TG29 | Y-Aib-QGTFTSDYSILLDK-K(Ac)-AQ-Aib-CFIEWLLCGGPSSGAP PPS | 21, 28 | A1 |
| TG30 | Y-Aib-QGTFTSDYSI-αMeL-LDKQAQ-Aib-CFIEYLLCGGPSSGAPP PS | 21, 28 | A1 |
| TG31 | Y-Aib-QGTFTSDYSI-αMeL-LDK-K(Ac)-AQ-a-CFIEYLLCGGPSSG APPPS | 21, 28 | A1 |
| TG32 | Y-Aib-QGTFTSDYSI-αMeL-LDK-K(Ac)-AQHCFIEYLLCGGPSSGA PPPS | 21, 28 | A1 |
| TG33 | Y-Aib-QGTFTSDYSI-αMeL-LDK-K(Ac)-AQQCFIEYLLCGGPSSGA PPPS | 21, 28 | A1 |
| TG34 | Y-Aib-QGTFTSDYSI-αMeL-LDK-K(Ac)-A-Aib-QCFIEYLLCGGPSS GAPPPS | 21, 28 | A1 |
| TG35 | Y-Aib-QGTFTSDYSI-αMeL-LDKRAQ-Aib-CFIEYLLCGGPSSGAPP PS | 21, 28 | A1 |
| TG36 | Y-Aib-QGTFTSDYSI-αMeL-LEK-K(Ac)-AQ-Aib-CFIEYLLCGGPSS GAPPPS | 21, 28 | A1 |
| TG37 | Y-Aib-QGTFTSDYSI-αMeL-L-Aad-K-K(Ac)-AQ-Aib-CFIEYLLCGG PSSGAPPPS | 21, 28 | A1 |
| TG38 | Y-Aib-QGTFTSD-4-Pal-SI-αMeL-LDK-K(Ac)-AQ-Aib-CFIEYLLCG GPSSGAPPPS | 21, 28 | A1 |
| TG39 | Y-Aib-QGTFT-αMeS-DYSI-αMeL-LDK-K(Ac)-AQ-Aib-CFIEYLLCG GPSSGAPPPS | 21, 28 | A1 |
| TG40 | Y-Aib-QGT-F(2-F)-TSDYSI-αMeL-LDK-K(Ac)-AQ-Aib-CFIEYLLCG GPSSGAPPPS | 21, 28 | A1 |
| TG41 | Y-Aib-Dab(Ac)-GTFTSDYSI-αMeL-LDK-K(Ac)-AQ-Aib-CFIEYLLC GGPSSGAPPPS | 21, 28 | A1 |
| TG42 | Y-Aib-HGTFTSDYSI-αMeL-LDK-K(Ac)-AQ-Aib-CFIEYLLCGGPSS GAPPPS | 21, 28 | A1 |
| TG43 | Y-Ac4c-QGTFTSDYSI-αMeL-LDK-K(Ac)-AQ-Aib-CFIEYLLCGGPS SGAPPPS | 21, 28 | A1 |
| TG44 | Y-Aib-QGTFTSDYSILLDKCAQH-Aib-FIEYLLCGGPSSGAPPPS | 17, 28 | A1 |
| TG45 | Y-Aib-QGTFTSDYSILLDK-HoC-AQH-Aib-FIEYLL-HoC-GGPSSGA PPPS | 17, 28 | A1 |
| TG46 | Y-Aib-QGT-F(2-F)-TSDYSILLDKCAQQAFICYLLAGGPSSGAPPPS | 17, 24 | A1 |
| TG47 | Y-Aib-QGT-F(2-F)-TSDYSILLDKQAQQCFIEYLLCGGPSSGAPPPS | 21, 28 | A1 |
| TG48 | Y-Aib-QGT-F(2-F)-TSDYSI-Aib-LDK-K(Ac)-AQQCFIEYLLCGGPSS GAPPPS | 21, 28 | A1 |
| TG49 | Y-Aib-QGT-F(2-F)-TSDYSILCDKQAQQCFIEYLLAGGPSSGAPPPS | 14, 21 | A1 |
| TG50 | Y-Aib-QGT-F(2-F)-TSDYSILLD-αMeK-K(Ac)-AQQCFIEYLLCGGP SSGAPPPS | 21, 28 | A1 |
| TG51 | Y-Aib-QGT-F(2-F)-TSDYSILLD-αMeK-K(Ac)-AQ-Aib-CFIEYLICG GPSSGAPPPS | 21, 28 | A1 |
| TG52 | Y-Aib-QGT-F(2-F)-TSDYSI-Iva-LDK-K(Ac)-AQ-Aib-CFIEYLLCGG PSSGAPPPS | 21, 28 | A1 |
| TG53 | Y-Aib-QGT-F(2-F)-TSDYSI-Iva-LDK-K(Ac)-AQ-Aib-CFIEYLLCGG PSSGAPPPS | 21, 28 | A5 |
| TG54 | Y-Aib-QGT-F(2-F)-TSDYSI-Iva-LDK-K(Ac)-AQ-Aib-CFIEYLLCGG PSSGAPPPS | 21, 28 | A8 |
| TG55 | Y-Aib-QGT-F(2-F)-TSDYSI-Iva-LDK-K(Ac)-AQ-Aib-CFIEYLLCGG PSSGAPPPS | 21, 28 | A11 |
| TG56 | Y-Aib-QGT-F(2-F)-TSDY-αMeS-ILLD-αMeK-K(Ac)-AQQCFIEYLLC GGPSSGAPPPS | 21, 28 | A1 |
| TG57 | Y-Aib-QGT-F(2-F)-TSDYSI-αMeL-LD-αMeK-K(Ac)-AQQCFIEYLL CGGPSSGAPPPS | 21, 28 | A1 |
| TG58 | Y-Aib-QGT-F(2-F)-TSDYSI-αMeL-LD-αMeK-K(Ac)-AQQC-HoF-IE YLLCGGPSSGAPPPS | 21, 28 | A1 |
| TG59 | Y-Aib-QGT-F(2-F)-TSDYSI-αMeL-LDKCAQCAFIEYLLAGGPSSGA PPPS | 17, 20 | A1 |
| TG60 | Y-Aib-QGT-F(2-F)-TSDYSI-Iva-LDKCAQCAFIEYLLAGGPSSGAPP PS | 17, 20 | A1 |
| TG61 | Y-Aib-QGT-F(2-F)-TSDY-αMeS-I-αMeL-LD-αMeK-K(Ac)-AQ-Aib-C FIEYLLCGGPSSGAPPPS | 21, 28 | A1 |
| TG62 | Y-Aib-QGT-F(2-F)-TSDY-αMeS-I-αMeL-LD-αMeK-CAQCAFIEYLL AGGPSSGAPPPS | 17, 20 | A1 |
| TG63 | H-Aib-HGT-F(2-F)-TSDYSIYLE-K(Ac)-KYA-Aib-CFVQWLLCGGP SSGAPPPS | 21, 28 | A1 |
| TG64 | H-Aib-HGT-F(2-F)-TSDYSIQ-K(Ac)-EEIAQ-Aib-CFIEWLLCGGPSS GAPPPS | 21, 28 | A1 |
| TG65 | H-Aib-HGT-F(2-F)-TSDYSIY-K(Ac)-EERAQ-Aib-CFIEWLLCGGPS SGAPPPS | 21, 28 | A1 |
| TG66 | H-Aib-HGT-F(2-F)-TSDYSI-Iva-LDK-K(Ac)-AQ-Aib-CFIEYLLCGG PSSGAPPPS | 21, 28 | A1 |
| TG67 | Y-Aib-QGT-F(2-F)-TSDYSI-αMeL-LDKCAHCAFIEYLLAGGPSSGA PPPS | 17, 20 | A1 |
| TG68 | Y-Aib-QGT-F(2-F)-TSDYSI-αMeL-LDKCA-K(Ac)-CAFIEYLLAGGP SSGAPPPS | 17, 20 | A1 |
| TG69 | Y-Aib-QGT-F(2-F)-TSDYSI-αMeL-LDKCAQCAFIEYLLAGGPSSGA PPPS | 17, 20 | A1 |
| TG70 | H-Aib-HGT-F(2-F)-TSDYSI-αMeL-LDKCAQCAFIEYLLAGGPSSGA PPPS | 17, 20 | A1 |
| TG71 | Y-s-QGT-F(2-F)-TSDYSI-αMeL-LDKCAQCAFIEYLLAGGPSSGAPP PS | 17, 20 | A1 |
| TG72 | Y-Aib-QGTFTSDYSILLDKQAQQCFIEYLLCGGPSSGAPPPS | 21, 28 | A1 |
| TG73 | Y-Ac4c-QGT-F(2-F)-TSDYSILLDKCAQCAFIEYLLEGGPSSGAPPP S | 17, 20 | A1 |
| TG74 | Y-Ac4c-QGT-F(2-F)-TSDYSILLDK-K(Ac)-AQ-Aib-CFIEYLLCGGPS SGAPPPS | 21, 28 | A1 |
| TG75 | H-Ac4c-HGT-F(2-F)-TSDYSIQLEEIAQ-Aib-CFIEYLLCGGPSSGAP PPS | 21, 28 | A1 |
| TG76 | Y-Aib-QGTFTSDYSILLDK-K(Ac)-AQ-Aib-c-FIEYLL-c-GGPSSGAP PPS | 21, 28 | A1 |
| TG77 | Y-Aib-QGTFTSDYSILLDK-K(Ac)-AQ-Aib-Hoc-FIEYLL-Hoc-GGPS SGAPPPS | 21, 28 | A1 |
| TG78 | Y-Aib-QGTFTSDYSILLDK-K(Ac)-AQ-Aib-αMeC-FIEYLL-αMeC-G GPSSGAPPPS | 21, 28 | A1 |
| TG79 | Y-Aib-QGT-F(2-F)-TSDYSIQLDK-K(Ac)-AQ-Aib-CFIEYLLCGGPSS GAPPPS | 21, 28 | A1 |
| TG80 | Y-Aib-QGT-F(2-F)-TSDYSI-αMeL-LDK-K(Ac)-AQ-Aib-CFIEYLLCG GPSSGAPPPS | 21, 28 | A11 |
| TG81 | Y-Aib-QGT-F(2-F)-TSDYSI-αMeL-LDK-K(Ac)-AQ-Aib-CFIEYLLCG GPSSGAPPPS | 21, 28 | A18 |
| TG82 | Y-Aib-QGT-F(2-F)-TSDYSI-αMeL-LDK-K(Ac)-AQ-Aib-CFIEYLLCG GPSSGAPPPS | 21, 28 | A20 |
| TG83 | Y-Aib-QGT-F(2-F)-TSDYSI-αMeL-LDK-K(Ac)-AQ-Aib-CFIEYLLCG GPSSGAPPPS | 21, 28 | A22 |
| TG84 | Y-Aib-QGT-F(2-F)-TSDYSI-αMeL-LD-αMeK-K(Ac)-AQ-Aib-KFIEY LLKGGPSSGAPPPS | 21, 28 | B1 |
| TG85 | Y-Aib-QGT-F(2-F)-TSDYSI-αMeL-LDK-K(Ac)-AQ-Aib-KFIEYLLK GGPSSGAPPPS | 21, 28 | B1 |
| TG86 | Y-Aib-QGTFTSDYSILLDK-K(Ac)-AQ-Aib-Orn-FIEYLL-Orn-GGPS SGAPPPS | 21, 28 | B1 |
| TG87 | Y-Aib-QGT-F(2-F)-TSDYSI-αMeL-LDKKAQKAFIEYLLAGGPSSGA PPPS | 17, 20 | B1 |
| TG88 | Y-Aib-QGT-F(2-F)-TSDYSIQLDKKAQKAFIEYLLAGGPSSGAPPPS | 17, 20 | B1 |
| TG89 | Y-Aib-QGTFTSDYSILLDKKAQKAFIEYLLAGGPSSGAPPPS | 17, 20 | B1 |
| TG90 | Y-Aib-QGT-F(2-F)-TSDYSI-αMeL-LDKKAQKAFIEYLLAGGPSSGA PPPS | 17, 20 | B1 |
| TG91 | Y-Aib-QGT-F(2-F)-TSDYSI-αMeL-LDKKAQKAFIEYLLAGGPSSGA PPPS | 17, 20 | B7 |
| TG92 | Y-Aib-QGT-F(2-F)-TSDYSI-αMeL-LDKKAQKAFIEYLLAGGPSSGA PPPS | 17, 20 | B16 |
| TG93 | Y-Aib-QGT-F(2-F)-TSDYSI-αMeL-LDKKAQKAFIEYLLAGGPSSGA PPPS | 17, 20 | B20 |
| TG94 | Y-Aib-QGT-F(2-F)-TSDYSI-αMeL-LDKKAQKAFIEYLLAGGPSSGA PPPS | 17, 20 | B22 |

14. A pharmaceutical composition, comprising:
the polypeptide or the derivative or pharmaceutically acceptable salt thereof according to any one of claims 1 to 12, or the polypeptide derivative or the pharmaceutically acceptable salt thereof according to claim 13; and
optionally, a pharmaceutically acceptable excipient or carrier.

15. The polypeptide or the derivative or pharmaceutically acceptable salt thereof according to any one of claims 1 to 12, the polypeptide derivative or the pharmaceutically acceptable salt thereof according to claim 13, or the pharmaceutical composition according to claim 14 for use in the treatment or prevention of a disease; and/or
use of the polypeptide or the derivative or pharmaceutically acceptable salt thereof according to any one of claims 1 to 12, the polypeptide derivative or the pharmaceutically acceptable salt thereof according to claim 13, or the pharmaceutical composition according to claim 14 in the manufacture of a medicament for treating or preventing a disease and/or in the treatment or prevention of a disease,
wherein the disease comprises at least one of a metabolic disorder related disease, a bone-related disease, a cardiovascular disease, or a neurodegenerative disease;
optionally, the metabolic disorder related disease comprises at least one of obesity, diabetes, dyslipidemia-related disease, fatty liver disease, metabolic syndrome, metabolic liver disease, non-alcoholic steatohepatitis, or non-alcoholic fatty liver disease;
optionally, the neurodegenerative disease comprises at least one of Alzheimer's disease or Parkinson's disease.

16. A method for treating or preventing a disease, the method comprising:
administering to a subject a pharmaceutically acceptable dose of the polypeptide or the derivative or pharmaceutically acceptable salt thereof according to any one of claims 1 to 12, the polypeptide derivative or the pharmaceutically acceptable salt thereof according to claim 13, or the pharmaceutical composition according to claim 14,
wherein the disease comprises at least one of a metabolic disorder related disease, a bone-related disease, a cardiovascular disease, or a neurodegenerative disease;
optionally, the metabolic disorder related disease comprises at least one of obesity, diabetes, dyslipidemia-related disease, fatty liver disease, metabolic syndrome, metabolic liver disease, non-alcoholic steatohepatitis, or non-alcoholic fatty liver disease;
optionally, the neurodegenerative disease comprises at least one of Alzheimer's disease or Parkinson's disease.
